# EUROPEAN PATENT APPLICATION

(11) **EP 1 964 834 A1**
(43) Date of publication of application: **03.09.2008**
(21) Application number: 07090034.5
(22) Date of filing: 01.03.2007
(51) Int. Cl.: C07D 209/16, C07D 405/12, C07D 405/14, C07D 409/14, A61K 31/4045, A61P 15/00

(54) **Sulphonyltryptophanols**

(71) Applicant: Bayer Schering Pharma Aktiengesellschaft, 13353 Berlin (DE)
(72) Inventor: Wortmann, Lars, 10435 Berlin (DE); Menzenbach, Bernd, 07743 Jena (DE); Frenzel, Thomas, 65719 Hofheim (Taunus) (DE); Schrey, Anna, Katharina, 10179 Berlin (DE); Kühne, Ronald, 12683 Berlin (DE); Muhn, Peter, 13465 Berlin (DE); Liesener, Florian, Peter, 83308 Trostberg (DE); Koppitz, Marcus, 10115 Berlin (DE); Kosemund, Dirk, 99091 Erfurt (DE)

(57) **Abstract**

The present invention relates to sulfonyltryptophanols of the general formula I, 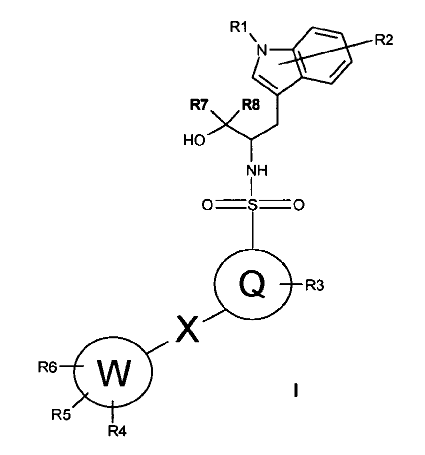 in which Q, X, W, R1, R2, R3, R4, R5, R6, R7 and R8 have the meaning as defined in the description.

The compounds according to the invention are effective FSH receptor antagonists and can be used for example for fertility control in men or in women, or for the prevention and/or treatment of osteoporosis.

## Description

The present invention relates to the use of sulfonyltryptophanols as FSH-receptor antagonists. The present invention also relates to novel sulfonyltryptophanols, process for their preparation, pharmaceutical compositions comprising the compounds according to the invention, their use for fertility control in men or women, and their use for the treatment and/or prevention of osteoporosis.

Follicle-stimulating hormone (FSH) and luteinizing hormone (LH) are together responsible for the control of male and female fertility and of the production of sex steroids.

In the female mammal, FSH controls the early ripening of ovarian primary follicles and the biosynthesis of sex steroids. In the advanced stage of differentiation (preantral follicles), the influence of LH becomes increasingly important for further development of the follicles until ovulation occurs.

In male mammals, FSH is primarily responsible for the differentiation and stimulation of Sertoli cells. Their function consists of assisting spermatogenesis on many levels. LH is primarily responsible for stimulating the Leydig cells and thus androgen production. FSH, LH and TSH (thyrotropic hormone) together form the group of glycoprotein hormones which are formed in the pituitary and are secreted from there. Whereas the alpha subunit is common to the three hormones, their specificity of action is determined by the beta chain which is unique in each case. The molecular weight of FSH including the sugar portion is about 30 kD.

FSH and the other glycoprotein hormones act specifically via their selectively expressed G protein-coupled receptor (GPCR). FSH stimulates, through binding to its receptor, the association thereof with a stimulating G protein (Gₛ) which is thereby stimulated to hydrolyse guanosine triphosphate (GTP) and to activate the membrane-associated adenylate cyclase. Cyclic adenosine monophosphate (cAMP) is accordingly an important and readily quantifiable secondary messenger substance of FSH (G. Vassart, L. Pardo, S. Costagliola, Trends Biochem. Sci. 2004, 29, 119-126).

The importance of FSH for male fertility is the subject of intensive research. It has been possible to show that FSH influences several processes of spermatogenesis such as the proliferation of spermatogonia, the antiapoptotic effect on spermatogonia and spermatocytes and the stimulation of sperm maturation including motility thereof.

The following arguments are also in favour of the FSH receptor as target for male fertility control:
1. The FSH receptor is exclusively expressed on Sertoli cells (high specificity).
2. Contraceptive vaccination against FSH beta chain or the FSH receptor induces infertility in male primates (N. R. Mougdal, M. Jeyakumar, H. N. Krishnamurthy, S. Sridhar, H. Krishnamurthy, F. Martin, Human Reproduction Update 1997, 3, 335-346).
3. Naturally occurring mutations in the FSH receptor or the FSH beta chain may lead to sub- or infertility in men (I. Huhtaniemi, Journal of Reproduction and Fertility 2000, 119, 173-186; L. C. Layman, P. G. McDonough, Molecular and Cellular Endocrinology 2000, 161, 9-17).
4. Neutralizing FSH antiserum has no effect on testis weight and testosterone production (V. Sriraman, A. J. Rao, Molecular and Cellular Endocrionology 2004, 224, 73-82). Adverse effects of FSH blockade on androgen production therefore appear unlikely.

In line with these arguments, FSH antagonists are expected to be suitable for spermatogenesis inhibition (prevention) in men. Moreover, a suitable FSH antagonist may just as well lead to infertility in women, because it suppresses follicle ripening and thus also ovulation. On the other hand, the skilled person expects advantages from non-peptidergic FSH agonists when used to promote fertility in women (stimulation of follicle ripening). There are no reports of experience on the use of FSH or FSH agonists in male infertility, but specific indications are also conceivable in this connection.

New findings demonstrate that there is also a direct effect of FSH on cells of bone metabolism. There are two fundamentally different cell types in bones: osteoclasts and osteoblasts. While osteoclasts play a central role in bone resorption (breakdown of bone), osteoblasts simulate bone density (anabolic effect).

FSH receptors have been detected in osteoclasts but not in osteoblasts. In vitro, FSH stimulates bone resorption by mouse osteoclasts ( Li Sun et al. Cell 2006; 125: 247-60). A clinical correlation between the height of the serum FSH levels and low bone density has been observed in postmenopausal women (Devleta et al, J. Bone Miner. Metab. 2004, 22: 360-4).

These findings among others suggest that FSH stimulates loss of bone mass, and accordingly FSH antagonists will display an antiresorptive effect on bone and are therefore suitable for the therapy and/or prevention of peri- and postmenopausal loss of bone mass and osteoporosis.

FSH receptor modulators are compounds that have a mixed profile of both FSH receptor antagonistic and/or FSH receptor agonistic properties. FSH receptor modulators of various compound classes of low molecular weight, have been reported on recently.

FSH receptor modulators are disclosed in WO 2004/056779, WO 2004/056780; J. Med. Chem. 2005, 48, 1697 [tetrahydroquinolines]; WO 02/70493, Bioorg. Med. Chem. Lett. 2004, 14, 1713 and 1717 [diketopiperazines]; and WO 01/47875 [sulphonamides].

FSH receptor agonists are disclosed in WO 02/09706; J. Comb. Chem. 2004, 6, 196 [Thiazolidinones]; WO 2003/020726 and WO 03/20727, Chem. Biochem. 2002, 10, 1023 {thieno[2,3-d] pyrimidines)}; WO 01/87287 [pyrazoles]; WO 00/08015 [carbazoles]; WO 06/117023, WO 06/117368, WO 06/117370, WO 06/117371, [hexahydroquinolines].

FSH receptor antagonists are disclosed in WO 03/004028 [tetrahydroquinolines], WO 02/09705 [thiazolidinones], WO 00/58277, Bioorg. Med. Chem. 2002, 10, 639 [sulphonic acids]; WO 00/58276, Endocr. 2002, 143, 3822; Synth. Comm. 2002, 32, 2695 [azo compounds]; US 2006/0199806, US 2006/0258644, US 2006/0258645, US 2006/0287522 [pyrrolobenzodiazepines], WO 2007/017289 [acyltryptophanols].

JP11-3432795 describes a broad scope of compounds of sulfonamide-type as a tumor necrosis factor alpha inhibitors. JP11-3432795 discloses sulfonyltryptophanols in claim 10 genericly and discloses one specific compound namely p-tolylethynyl-thiophene-2-sulfonic acid [1-hydroxymethyl-2-(1H-indol-3-yl)-ethyl]-amide [example 37].

In view of the prior art, the objective technical problem to be solved according to the present invention may therefore be seen in providing alternative means of controlling the fertility in men (male fertility control) or women (female contraception), or providing alternative treatment and/or prevention of osteoporosis.

The technical problem has been solved according to the present invention by the use of compounds of the formula I 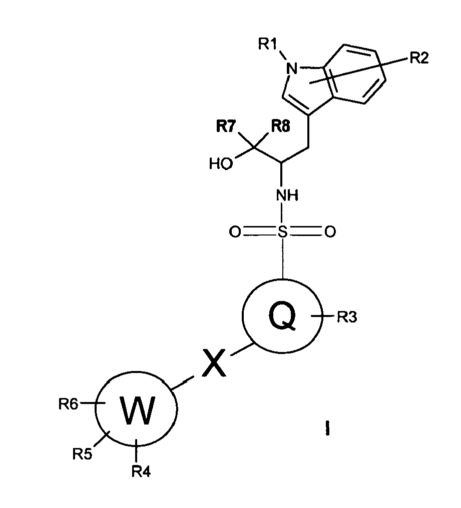 in which
- R1: may be hydrogen, C₁-C₆-alkyl, C₃-C₆-alkenyl, C₃-C₆-alkynyl, C₃-C₇-cycloalkyl, C₁-C₆-alkyloxy-C₁-C₆-alkylene, C₃-C₇-cycloalkyloxy-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups: 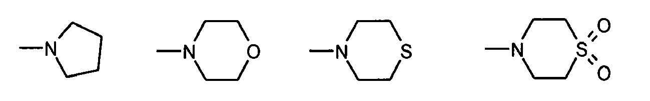 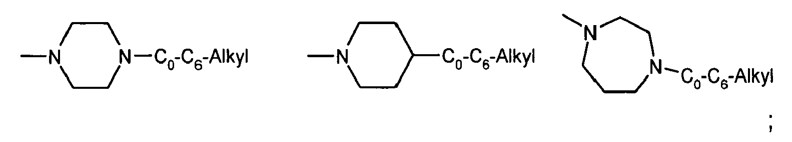
- R2: may be hydrogen, halogen, cyano, -SO₂Me, C₁-C₆-alkyl, C₂-C₆-alkenyl, C₂-C₆-alkynyl, C₁-C₆-alkyloxy or benzyloxy,
where the hydrocarbon chains therein may optionally be substituted one or more times by fluorine;
- R3: may be hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylam ino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups 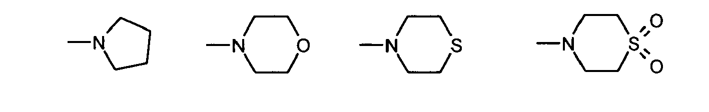 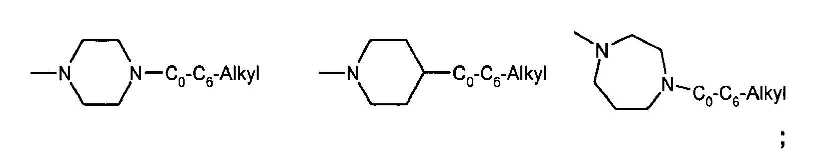
- R4, R5, R6: may be independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl,
where the hydrocarbon chains therein may optionally be substituted one or more times by fluorine, cyano or the radicals: 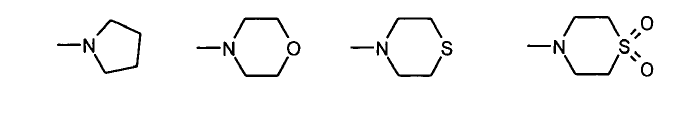 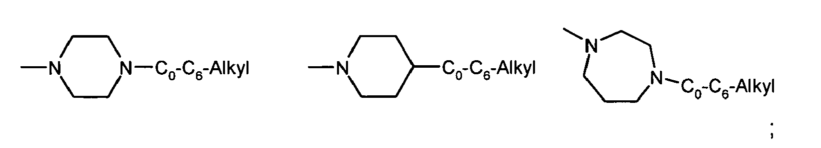 or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇cycloal kyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl,
C₁-C₆-alkylcarbonyl, C₃-C₇-cycloalkylcarbonyl, carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-Cycloalkyl, -N(C₀-C₆-alkyl)-SO₂-C₁-C₆-alkyl, -N(C₁-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the radicals: 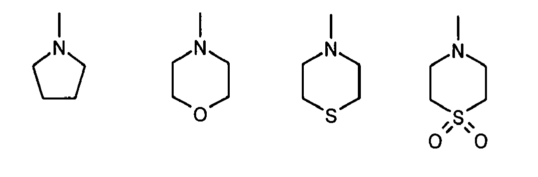 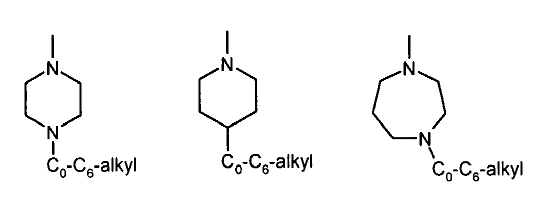 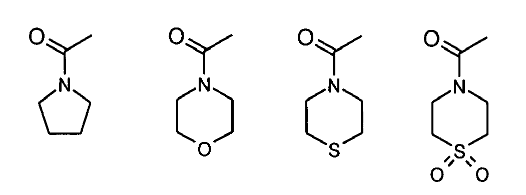 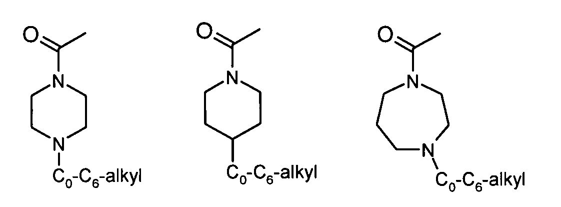 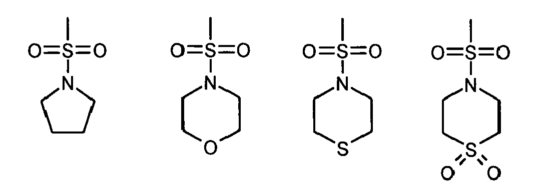 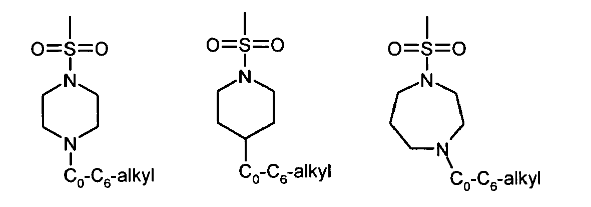 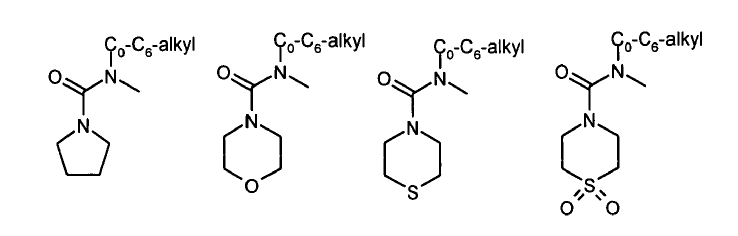 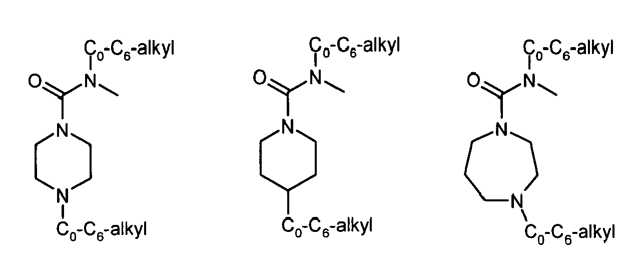 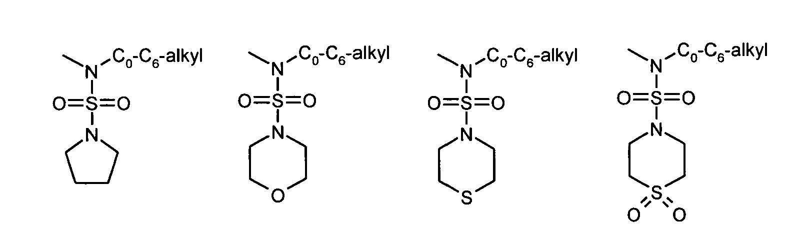 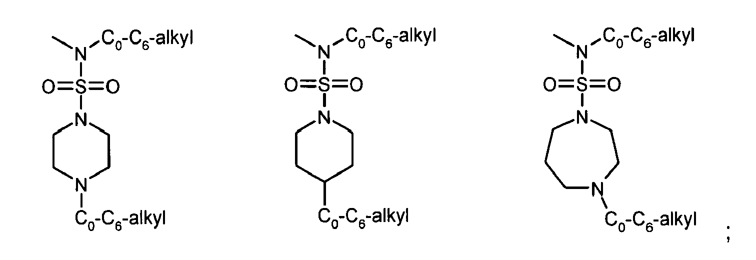
- R5 and R6: may together form heterocycloalkyl, cycloalkyl;
- R7, R8: may be independently of one another hydrogen, methyl, ethyl,
where the methyl and ethyl radicals may be fluorinated one or more times;
- Q: may be an aryl or heteroaryl group
or the group 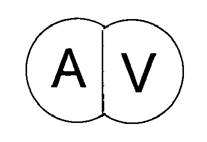 in which
A is a monocyclic aryl or a monocyclic heteroaryl group;
V is a cycloalkylen, cycloalkenylen, heterocycloalkylen or heterocycloalkenylen group;
- X: may be a bond, C₁-C₄-alkylene, C₂-C₄-alkenylene, C₂-C₄-alkynylene;
- W: may be an aryl or heteroaryl group;
where
- R2: may substitute one or more positions of the aryl or heteroaryl ring in the indole residue;
- R3: may substitute one or more positions of the aryl or heteroaryl ring in the radical Q and or the radical V.

The present invention relates to both possible enantiomeric forms at the stereocentre of the tryptophanol residue.

The unbranched C₁-C₆-alkyl groups for the radicals R1 to R6 may be for example a methyl, ethyl, propyl, butyl, pentyl or a hexyl group; and the branched C₃-C₆-alkyl groups for the radicals R1 to R6 may be an isopropyl, isobutyl, sec-butyl, *tert*-butyl, isopentyl, 2-methylbutyl, 1-methylbutyl, 1-ethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or a 1,2-dimethylbutyl group.

The branched or unbranched C₃-C₆-alkenyl groups for the radical R1 may be for example an allyl, (*E*)-2-methylvinyl, (*Z*)-2-methylvinyl, homoallyl, (*E*)-but-2-enyl, (*Z*)-but-2-enyl, (*E*)-but-l-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (Z)-3-ethylbut-2-enyl, (*E*)-2-ethylbut-2-enyl, (Z)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (Z)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl- or a 1-(1,1-dimethylethyl)ethenyl group.

The C₃-C₆-alkynyl groups for the radical R1 may be for example a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

The C₂-C₆-alkenyl groups for the radicals R2 to R6 may, in addition to the C₃-C₆-alkenyl groups mentioned for the radical R1, be for example a vinyl group.

The C₂-C₆-alkynyl groups for the radicals R2 to R6 may, in addition to the C₃-C₆-alkynyl groups mentioned for the radical R1, be for example an ethynyl group. The C₁-C₆-alkyloxy groups for the radicals R2 to R6 may be for example a methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, *ne*opentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy group.

The halogens for the radicals R2 to R6 are fluorine, chlorine, bromine or iodine.

The C₁-C₃-alkylsulphanyl groups for the radicals R4 to R6 may be for example a methylsulphanyl (CH₃S-), ethylsulphanyl (CH₃CH₂S-), propylsulphanyl, isopropylsulphanyl group.

The C₁-C₆-alkylaminocarbonyl groups for the radicals R4 to R6 may be for example a methylaminocarbonyl-, ethylaminocarbonyl-, propylaminocarbonyl-, isopropylaminocarbonyl-, butylaminocarbonyl-, isobutylaminocarbonyl-, sec-butylaminocarbonyl-, *tert-*butylaminocarbonyl-, pentylaminocarbonyl-, isopentylaminocarbonyl-, (2-methylbutyl)-aminocarbonyl-, (1-methylbutyl)aminocarbonyl-, (1-ethylpropyl)aminocarbonyl-, *neo-*pentylaminocarbonyl-, (1,1-dimethylpropyl)aminocarbonyl-, hexylaminocarbonyl-, (4-methylpentyl)aminocarbonyl-, (3-methylpentyl)aminocarbonyl-, (2-methylpentyl)aminocarbonyl-, (1-methylpentyl)aminocarbonyl-, (1-ethylbutyl)aminocarbonyl-, (2-ethylbutyl)-aminocarbonyl-, (3,3-dimethylbutyl)aminocarbonyl-, (2,2-dimethylbutyl)aminocarbonyl-, (1,1-dimethylbutyl)aminocarbonyl-, (2,3-dimethylbutyl)aminocarbonyl-, (1,3-dimethylbutyl)aminocarbonyl- or a (1,2-dimethylbutyl)aminocarbonyl group.

The hydroxy-C₁-C₆-alkylene groups for the radicals R3 to R6 may be a hydroxymethyl (HOCH₂-), 2-hydroxyethyl (HOCH₂CH₂-), 1-hydroxyethyl [CH₃CH(OH)-], 3-hydroxypropyl (HOCH₂CH₂CH₂-), 2-hydroxypropyl [CH₃CH(OH)CH₂-], 1-hydroxypropyl [CH₃CH₂CH(OH)-], 2-hydroxy-1-methylethyl [HOCH₂CH(CH₃)-], 1-hydroxy-1-methylethyl [(CH₃)₂C(OH)-], 4-hydroxybutyl (HOCH₂CH₂CH₂CH₂-), 3-hydroxybutyl [CH₃CH(OH)CH₂CH₂-], 2-hydroxybutyl [CH₃CH₂CH(OH)CH₂-], 1-hydroxybutyl [CH₃CH₂CH₂CH(OH)-], 3-hydroxy-1-methylpropyl [HOCH₂CH₂CH(CH₃)-], 2-hydroxy-1-methylpropyl [CH₃CH(OH)CH(CH₃)-], 1-hydroxy-1-methylpropyl [CH₃CH₂C(CH₃)(OH)-], 1-(hydroxymethyl)propyl [CH₃CH(CH₂OH)-], 3-hydroxy-2-methylpropyl [HOCH₂CH(CH₃)CH₂-], 2-hydroxy-2-methylpropyl [(CH₃)₂C(OH)CH₂-], 1-hydroxy-2-methylpropyl [CH₃CH(CH₃)CH(OH)-] or a 2-hydroxy-1,1-dimethylethyl group [HOCH₂C(CH₃)₂-].

The heterocycloalkyl groups which may form the radicals R4 and R5 together may be for example the following groups:

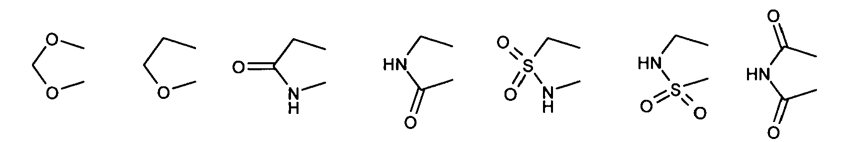

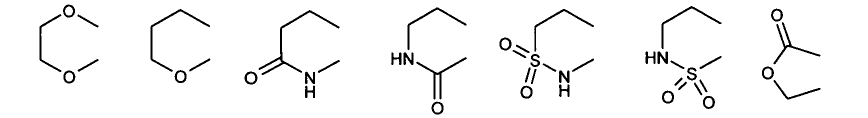

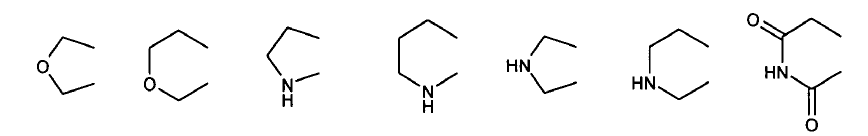

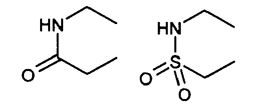

The cycloalkyl groups which may form the radicals R4 and R5 together may be for example the following groups:

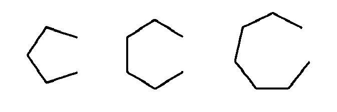

The C₃-C₇-cycloalkyl groups for the radicals R1 to R6 may be for example a cyclopropyl, cyclobutyl, cyclopentyl, cyclohexyl, cycloheptyl group.
The C₃-C₇-heterocycloalkyl groups for the radicals R1 to R6 may be for example a cyclopropyl, cyclobutyl, cycopentyl, cyclohexyl, cycloheptyl group in which one or two carbon atoms of the ring are replaced independently of one another by an oxygen, nitrogen or sulphur atom.

The monocyclic aryl group for A may be for example a phenyl group which is linked via substitutable positions
The aryl group for W or Q may be for example a phenyl, naphthyl group which is linked via substitutable positions.
The monocylic heteroaryl group for A may be for example a pyridinyl, pyrimidinyl, furanyl, thienyl, oxazolyl, isoxazolyl, thiazolyl, pyrrolyl, pyrazolyl or an imidazolyl group which is linked via substitutable positions.

The heteroaryl group for W or Q may be for example a pyridinyl, pyrimidinyl, quinolinyl, isoquinolinyl, quinazolinyl, quinoxalinyl, phthalazinyl, 1.5-naphthyridinyl, 1,6-naphthyridinyl, 1,7-naphthyridinyl, 1,8-naphthyridinyl, benzofuranyl, benzothienyl, 1,3-benzodioxolyl, 2,1,3-benzothiadiazolyl, indolyl, indazolyl, furanyl, thienyl, oxazolyl, isoxazolyl, thiazolyl, pyrrolyl, pyrazolyl or an imidazolyl group which is linked via substitutable positions.

The heterocycloalkylen groups for V may be for example the following groups:

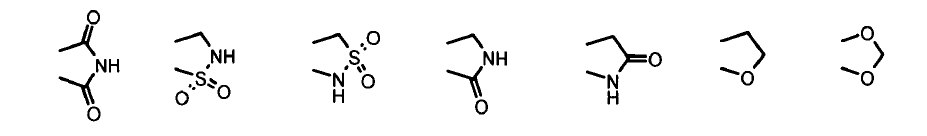

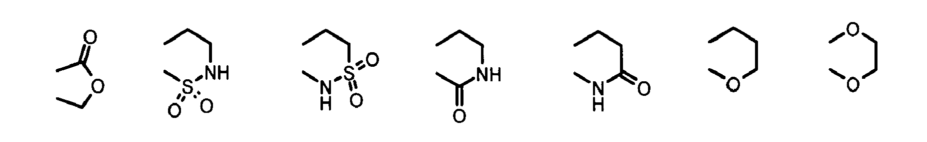

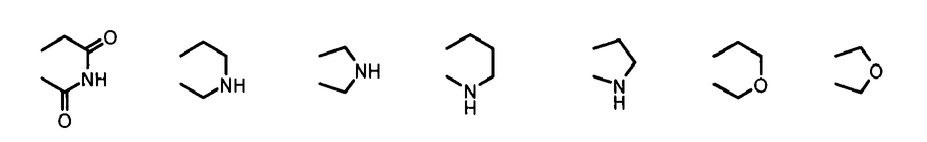

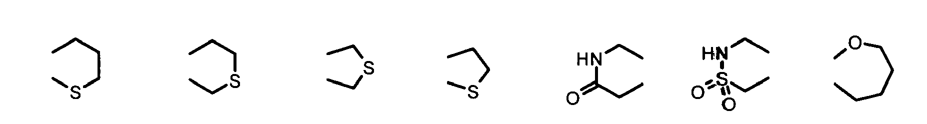

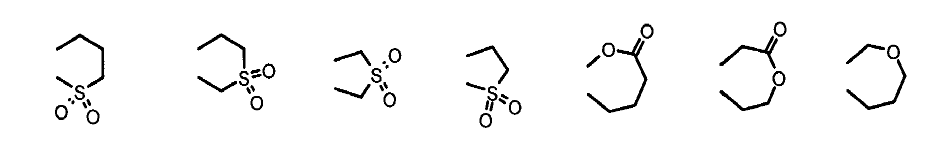

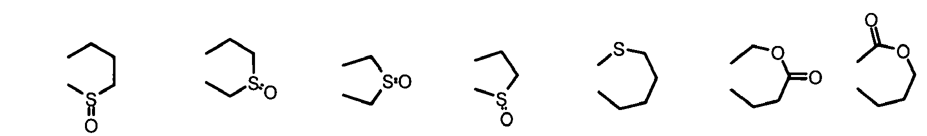

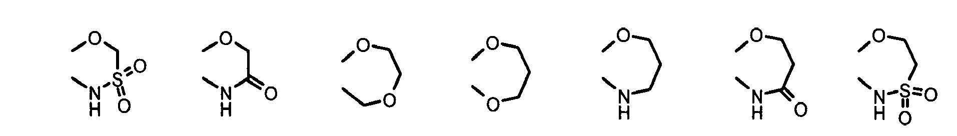

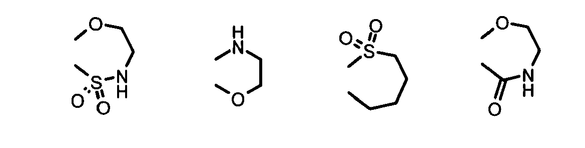

The heterocycloalkenylen groups for V may be for example the following groups:

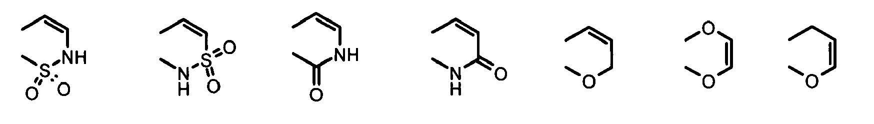

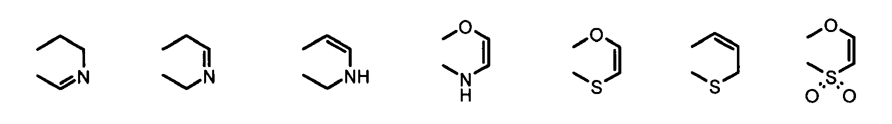

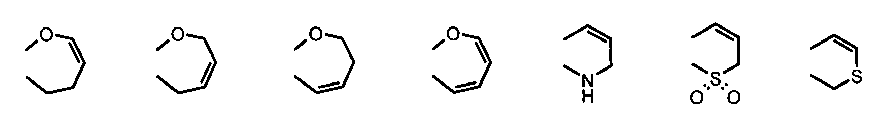

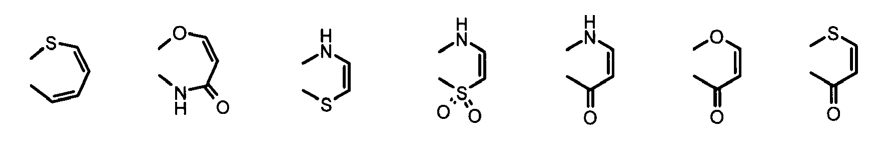

The cycloalkylen groups for V may be for example the following groups:

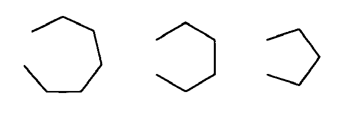

The cycloalkenylen groups for V may be for example the following groups:

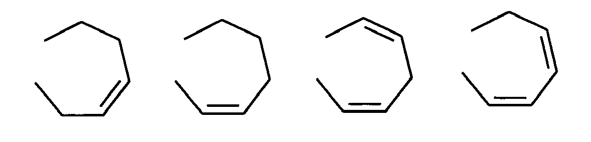

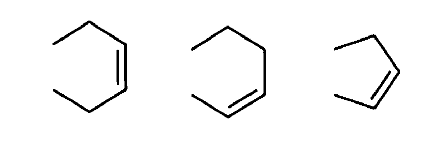

The C₁-C₄-alkylene groups for the radicals X may be for example a methylene (-CH₂-), ethylidene [-CH(CH₃)-], ethylene (-CH₂CH₂-), prop-1,3-ylene (-CH₂CH₂CH₂-), prop-1,2-ylene [-CH₂CH(CH₃)-], but-1,4-ylene (-CH₂CH₂CH₂CH₂-), but-1,3-ylene [-CH₂CH₂CH(CH₃)-], but-1,2-ylene [-CH₂CH(CH₂CH₃)-], but-2,3-ylene [-CHCH(CH₃)-], 2-methylprop-1,2-ylene [-CH₂C(CH₃)₂-] or a 2-methylprop-1,3-ylene group [-CH₂CH(CH₃)CH₂-].

The C₂-C₄-alkenylene groups for the radical X may be for example an ethen-1,2-ylidene (-CH=CH-), prop-2-en-1,3-ylidene (-CH₂-CH=CH-), prop-1-en-1,3-ylidene (-CH=CH-CH₂-), but-1-en-1,4-ylidene (-CH=CH-CH₂-CH₂-), but-2-en-1,4-ylidene (-CH₂-CH=CH-CH₂-) or a but-3-en-1,4-ylidene group (-CH₂-CH₂-CH=CH-).

The C₂-C₄-alkynylene groups for the radical X may be for example an ethyn-1,2-ylidene (-C≡C-), prop-2-yn-1,3-ylidene (-CH₂-C≡C-), prop-1-yn-1,3-ylidene (-C≡C-CH₂-), but-1-yn-1,4-ylidene (-C≡C-CH₂-CH₂-), but-2-yn-1,4-ylidene (-CH₂-C≡C-CH₂-) or a but-3-yn-1,4-ylidene group (-CH₂-CH₂-C≡C-).

The C₁-C₃-alkyleneoxy groups for the radical X may be for example an oxymethylene (-O-CH₂-), methyleneoxy (-CH₂-O-), ethane-1,2-diyloxy (-CH₂-CH₂-O-), oxyethane-1,2-diyl (-O-CH₂-CH₂-), propane-1,3-diyloxy (-CH₂-CH₂-CH₂-O-) or an oxypropane-1,3-diyl (-O-CH₂-CH₂-CH₂-) group.
The C₁-C₃-alkyleneoxy-C₁-C₃-alkyl groups for the radical X may be for example an oxybis(methylene) (-CH₂-O-CH₂-), methyleneoxyethane-2,1-diyl [-CH₂-O-(CH₂)₂-], ethane-1,2-diyloxymethylene [-(CH₂)₂-O-CH₂-], methyleneoxypropane-3,1-diyl [-CH₂-O-(CH₂)₃-], propane-1,3-diyloxymethylene [-(CH₂)₃-O-CH₂-], oxybis(ethane-2,1-diyl) [-(CH₂)₂-O-(CH₂)₂-], propane-1,3-diyloxyethane-2,1-diyl [-(CH₂)₃-O-(CH₂)₂-], ethane-1,2-diyloxypropane-3,1-diyl [-(CH₂)₂-O-(CH₂)₃-] or an oxybis(propane-3,1-diyl) group [-(CH₂)₃-O-(CH₂)₃-].

The C₃-C₇-cycloalkyloxy groups for the radicals R1 to R6 may be for example a cyclopropyloxy, cyclobutyloxy, cyclopentyloxy, cyclohexyloxy, cycloheptyloxy group.
The C₁-C₆-alkylamino groups for the radicals R1 to R6 may be for example methylamino, ethylamino, propylamino, *iso*propylamino, butylamino, *iso*butylamino, *sec*-butylamino, *tert*-butylamino, pentylamino, *iso*pentylamino, (2-methylbutyl)amino, (1-methylbutyl)amino, (1-ethylpropyl)amino, *neo*pentylamino, (1,1-dimethylpropyl)amino, hexylamino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino group.

In the di(C₁-C₆-alkyl)amino groups for the radicals R1 to R6, each of the two radicals on the nitrogen atom of the dialkylamino group may be chosen independently of one another from the following radicals: possible examples are a methyl, ethyl, propyl, isopropyl, butyl, isobutyl, *sec*-butyl, *tert*-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the C₃-C₇-cycloalkyl-C₁-C₆-alkyleneoxy groups for the radicals R1 to R6 it is possible to combine each of the C₃-C₇-cycloalkyl groups of the C₃-C₇-cycloalkyl-C₁-C₆-alkyleneoxy group, for example of a cyclopropyl, cyclobutyl, cyclopentyl, cyclohexyl or cycloheptyl group, independently of one another with each C0-C6-alkyleneoxy group, for example with a methyleneoxy, ethyleneoxy, propyleneoxy, butyleneoxy, pentyleneoxy, hexyleneoxy group.
In the hydroxy-C₃-C₆-alkenylene groups for the radicals R1 to R6 it is possible for the hydroxy group to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-Pent-2-enyl-, (*Z*)-Pent-2-enyl-, (*E*)-Pent-1-enyl-, (*Z*)-Pent-1-enyl-, hex-5-enyl-, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methytprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (Z)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (Z)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group, and to be combined independently of one another.

In the hydroxy-C₃-C₆-alkynyl groups for the radicals R1 to R6 it is possible for the hydroxy group to be located at any desired position of the C₃-C₆-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

In the C₁-C₆-alkyloxy-C₃-C₆-alkenylene groups for the radicals R1 to R6 it is possible for the C₁-C₆-alkyloxy group, for example a methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, neopentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (E)-2-methylbut-1-enyl, (Z)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (Z)-3-methylpent-1-enyl, (*E*)-2-methylpent-1-enyl, (*Z*)-2-methylpent-1-enyl, (*E*)-1-methylpent-1-enyl, (Z)-1-methylpent-1-enyl, 3-ethylbut-3-enyl, 2-ethylbut-3-enyl, 1-ethylbut-3-enyl, (*E*)-3-ethylbut-2-enyl, (Z)-3-ethylbut-2-enyl, (*E*)-2-ethylbut-2-enyl, (Z)-2-ethylbut-2-enyl, (*E*)-1-ethylbut-2-enyl, (Z)-1-ethylbut-2-enyl, (*E*)-3-ethylbut-1-enyl, (Z)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (Z)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (Z)-1-isopropylprop-1-enyl, (*E*)-3,3-dimethylprop-1-enyl, (*Z*)-3,3-dimethylprop-1-enyl or a 1-(1,1-dimethylethyl)ethenyl group and to be combined independently of one another.

In the C₁-C₆-alkyloxy-C₃-C₆-alkynylene groups for the radicals R1 to R6 it is possible for the C₁-C₆-alkyloxy group, for example a methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located at any desired position of the C3-C6-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group, and to be combined independently of one another.

In the C₁-C₆-alkyloxyphenyl-C₁-C₆-alkylene groups for the radical R1 to R6 it is possible for the C₁-C₆-alkyloxy group to be selected independently of one another from methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1 ,2-dimethylbutyl)oxy, and to be combined independently of one another with C₁-C₆-alkylene groups such as, for example, methylene, ethylene, propylene, butylene, pentylene, hexylene.

In the C₃-C₇-cycloalkyl-(C₀-C₆)-alkyleneamino groups of the radicals R3 to R6 it is possible for each of the C₃-C₇-cycloalkyl groups of the C₃-C₇-cycloalkyl-(C₀-C₆)-alkyleneamino group, for example of a cyclopropyl, cyclobutyl, cyclopentyl, cyclohexyl or cycloheptyl group, to be combined independently of one another with each C₀-C₆-alkylene group, for example with a bond, a methylene, ethylene, propylene, butylene, pentylene, hexylene group.

In the C₁-C₆-alkyloxy-C₁-C₆-alkylene groups for the radical R1 to R6, it is possible for the C₁-C₆-alkyloxy group to be selected independently for example from methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy and to be combined independently of one another with C₁-C₆-alkylene groups such as, for example, methylene, ethylene, propylene, butylene, pentylene, hexylene.

In the di(C₁-C₆-alkyl)amino-C₁-C₆-alkylene group for the radical R1 it is possible for each of the two radicals on the nitrogen atom of the amino group to be selected independently for example from methyl, ethyl, propyl, *iso*propyl, butyl, *iso*butyl, *sec*-butyl, *tert*-butyl, pentyl, *iso*pentyl, (2-methylbutyl), (1-methylbutyl), (1-ethylpropyl), *neo*pentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, and to be combined with C₁-C₆-alkylene groups such as, for example, methylene, ethylene, propylene, butylene, pentylene, hexylene.

The C₃-C₇-cydoalkyl-C₁-C₆-alkylene groups for the radicals R1 to R6 may be for example a cyclopropyloxymethylene, cyclopropyloxyethylene, cyclopropyloxypropylene, cyclopropyloxybutylene, cyclopropyloxypentylene, cyclopropyloxyhexylene, cyclobutyloxymethylene, cyclobutyloxyethylene, cyclobutyloxypropylene, cyclobutyloxybutylene, cyclobutyloxypentylene, cyclobutyloxyhexylene, cyclopentyloxymethylene, cyclopentyloxyethylene, cyclopentyloxypropylene, cyclopentyloxybutylene, cyclopentyloxypentylene, cyclopentyloxyhexylene, cyclohexyloxymethylene, cyclohexyloxyethylene, cyclohexyloxypropylene, cyclohexyloxybutylene, cyclohexyloxypentylene, cyclohexyloxyhexylene, cycloheptyloxymethylene, cycloheptyloxyethylene, cycloheptyloxypropylene, cycloheptyloxybutylene, cycloheptyloxypentylene, cycloheptyloxyhexylen group.

In the C₁-C₆-alkylamino-C₁-C₆-alkylene groups for the radicals R1 to R6 it is possible for the C₁-C₆-alkylamino group to be selected independently for example from methylamino, ethylamino, propylamino, isopropylamino, butylamino, isobutylamino, sec-butylamino, *tert*-butylamino, pentylamino, isopentylamino, (2-methylbutyl)amino, (1-methylbutyl)amino, (1-ethylpropyl)amino, *neopenty*lamino, (1,1-dimethylpropyl)amino, hexylamino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino and to be combined with C₁-C₆-alkylene groups such as, for example, methylene, ethylene, propylene, butylene, pentylene, hexylene.

The phenyloxy-C₁-C₆-alkylene groups for the radicals R1 to R6 may be for example a phenyloxymethyl, phenyloxyethyl, phenyloxypropyl, phenyloxybutyl, phenyloxypentyl, phenyloxyhexyl group.

In the C₁-C₆-acyl-(C₀-C₆-alkyl)amido groups for the radicals R4 to R6, it is possible for each of the C₁-C₆-acyl groups, for example a formyl, acetyl, propionyl, 2-methylpropionyl, 2,2-dimethylpropionyl, butyryl, 2-methylbutyryl, 3-methylbutyryl, 2,2-dimethylbutyryl, 2-ethylbutyryl, pentanoyl, 2-methylpentanoyl, 3-methylpentanoyl, 4-methylpentanoyl or a hexanoyl group, to be combined independently of one another with each (C₀-C₆-alkyl)amido group, for example a hydrogen atom, a methylamido, ethylamido, propylamido, isopropylamido, butylamido, isobutylamido, sec-butylamido, tert-butylamido, pentylamido, isopentylamido, (2-methylbutyl)amido, (1-methylbutyl)amido, (1-ethylpropyl)amido, neopentylamido, (1,1-dimethylpropyl)amido, hexylamido, (4-methylpentyl)amido, (3-methylpentyl)amido, (2-methylpentyl)amido, (1-methylpentyl)amido, (1-ethylbutyl)amido, (2-ethylbutyl)amido, (3,3-dimethylbutyl)amido, (2,2-dimethylbutyl)-amido, (1,1-dimethylbutyl)amido, (2,3-dimethylbutyl)amido, (1,3-dimethylbutyl)amido or a (1,2-dimethylbutyl)amido group.

The C₁-C₆-alkylaminocarbonyl groups for the radicals R4 to R6 may be for example a methylaminocarbonyl, ethylaminocarbonyl, propylaminocarbonyl, isopropylaminocarbonyl, butylaminocarbonyl, isobutylaminocarbonyl, sec-butylaminocarbonyl, tert-butylaminocarbonyl, pentylaminocarbonyl, isopentylaminocarbonyl, (2-methylbutyl)-aminocarbonyl, (1-methylbutyl)aminocarbonyl, (1-ethylpropyl)aminocarbonyl, neopentylaminocarbonyl, (1,1-dimethylpropyl)aminocarbonyl, hexylaminocarbonyl, (4-methylpentyl)aminocarbonyl, (3-methylpentyl)aminocarbonyl, (2-methylpentyl)aminocarbonyl, (1-methylpentyl)aminocarbonyl, (1-ethylbutyl)aminocarbonyl, (2-ethylbutyl)aminocarbonyl, (3,3-dimethylbutyl)aminocarbonyl, (2,2-dimethylbutyl)aminocarbonyl, (1,1-dimethylbutyl)aminocarbonyl, (2,3-dimethylbutyl)aminocarbonyl, (1,3-dimethylbutyl)-aminocarbonyl or a (1,2-dimethylbutyl)aminocarbonyl group.

In the di(C₁-C₆-alkyl)aminocarbonyl groups for the radicals R4 to R6, each of the two C₁-C₆-alkyl radicals on the nitrogen atom of the di(C₁-C₆-alkyl)aminocarbonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1 ,3-dimethylbutyl) or a (1,2-dimethylbutyl) group.

The (C₃-C₇-cycloalkyl)aminocarbonyl groups for the radicals R4 to R6 may be for example a cyclopropylaminocarbonyl, cyclobutylaminocarbonyl, cyclopentylaminocarbonyl, cyclohexylaminocarbonyl or cycloheptylaminocarbonyl group.

In the di(C₃-C₇-cycloalkyl)aminocarbonyl groups for the radicals R4 to R6, each of the two C₃-C₇-cycloalkyl radicals on the nitrogen atom of the di(C₃-C₇-cydoalkyl)aminocarbonyl group may be independently of one another for example a cyclopropyl, cyclobutyl, cyclopentyl, cyclohexyl or cycloheptyl group.

In the C₃-C₇-cycloalkyl-C₁-C₆-alkyleneaminocarbonyl groups of the radicals R4 to R6 it is possible for each of the C₃-C₇-cydoalkyl groups of the C₃-C₇-cycloalkyl-C₁-C₆-alkyleneaminocarbonyl groups, for example of a cyclopropyl, cyclobutyl, cyclopentyl, cyclohexyl or cycloheptyl group, to be combined independently of one another with each C₁-C₆-alkyleneaminocarbonyl group, for example with a methyleneaminocarbonyl, ethyleneaminocarbonyl, propyleneaminocarbonyl, butyleneaminocarbonyl, pentyleneaminocarbonyl, hexyleneaminocarbonyl group.

The C₁-C₆-alkylcarbonyl groups for the radicals R4 to R6 may be for example a methylcarbonyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, (2-methylbutyl)carbonyl, (1-methylbutyl)carbonyl, (1-ethylpropyl)carbonyl, neopentylcarbonyl, (1,1-dimethylpropyl)carbonyl, hexylcarbonyl, (4-methylpentyl)carbonyl, (3-methylpentyl)carbonyl, (2-methylpentyl)carbonyl, (1-methylpentyl)carbonyl, (1-ethylbutyl)-carbonyl, (2-ethylbutyl)carbonyl, (3,3-dimethylbutyl)carbonyl, (2,2-dimethylbutyl)carbonyl, (1,1-dimethylbutyl)carbonyl, (2,3-dimethylbutyl)carbonyl, (1,3-dimethylbutyl)-carbonyl or a (1,2-dimethylbutyl)carbonyl group.

The C₃-C₇-cycloalkylcarbonyl groups for the radicals R4 to R6 may be for example a cyclopropylcarbonyl, cyclobutylcarbonyl, cyclopentylcarbonyl, cyclohexylcarbonyl or cycloheptylcarbonyl group.

The C₁-C₆-alkyloxycarbonyl groups for the radicals R4 to R6 may be for example a methyloxycarbonyl, ethyloxycarbonyl, propyloxycarbonyl, isopropyloxycarbonyl, butyloxycarbonyl, isobutyloxycarbonyl, sec-butyloxycarbonyl, tert-butyloxycarbonyl, pentyloxycarbonyl, isopentyloxycarbonyl, (2-methylbutyl)oxycarbonyl, (1-methylbutyl)oxycarbonyl, (1-ethylpropyl)oxycarbonyl, neopentyloxycarbonyl, (1,1-dimethylpropyl)oxycarbonyl, hexyloxycarbonyl, (4-methylpentyl)oxycarbonyl, (3-methylpentyl)oxycarbonyl, (2-methylpentyl)oxycarbonyl, (1-methylpentyl)oxycarbonyl, (1-ethylbutyl)oxycarbonyl, (2-ethylbutyl)oxycarbonyl, (3,3-dimethylbutyl)oxycarbonyl, (2,2-dimethylbutyl)oxycarbonyl, (1,1-dimethylbutyl)oxycarbonyl, (2,3-dimethylbutyl)oxycarbonyl, (1,3-dimethylbutyl)oxycarbonyl or a (1,2-dimethylbutyl)oxycarbonyl group.

The C₁-C₆-alkylsulphonyl groups for the radicals R4 to R6 may be for example a methylsulphonyl, ethylsulphonyl, propylsulphonyl, isopropylsulphonyl, butylsulphonyl, isobutylsulphonyl, sec-butylsulphonyl, tert-butylsulphonyl, pentylsulphonyl, isopentylsulphonyl, (2-methylbutyl)sulphonyl, (1-methylbutyl)sulphonyl, (1-ethylpropyl)sulphonyl, neopentylsulphonyl, (1,1-dimethylpropyl)sulphonyl, hexylsulphonyl, (4-methylpentyl)-sulphonyl, (3-methylpentyl)sulphonyl, (2-methylpentyl)sulphonyl, (1-methylpentyl)-sulphonyl, (1-ethylbutyl)sulphonyl, (2-ethylbutyl)sulphonyl, (3,3-dimethylbutyl)sulphonyl, (2,2-dimethylbutyl)sulphonyl, (1,1-dimethylbutyl)sulphonyl, (2,3-dimethylbutyl)sulphonyl, (1,3-dimethylbutyl)sulphonyl or a (1,2-dimethylbutyl)sulphonyl group.

The C₃-C₇-cycloalkylsulphonyl groups for the radicals R4 to R6 may be for example a cyclopropylsulphonyl, cyclobutylsulphonyl, cyclopentylsulphonyl, cyclohexylsulphonyl or cycloheptylsulphonyl group.

In the C₃-C₇-cycloalkyl-C₁-C₆-alkylenesulphonyl groups of the radicals R4 to R6 it is possible for each of the C₃-C₇-cycloalkyl groups of the C₃-C₇-cycloalkyl-C₁-C₆-alkylenesulphonyl groups, for example of a cyclopropyl, cyclobutyl, cyclopentyl, cyclohexyl or cycloheptyl group, to be combined independently of one another with each C₁-C₆-alkylenesulphonyl group, for example with a methylenesulphonyl, ethylenesulphonyl, propylenesulphonyl, butylenesulphonyl, pentylenesulphonyl, hexylenesulphonyl group. The C₁-C₆-alkylaminosulphonyl groups for the radicals R4 to R6 may be for example a methylaminosulphonyl, ethylaminosulphonyl, propylaminosulphonyl, isopropylaminosulphonyl, butylaminosulphonyl, isobutylaminosulphonyl, sec-butylaminosulphonyl, tert-butylaminosulphonyl, pentylaminosulphonyl, isopentylaminosulphonyl, (2-methylbutyl)-aminosulphonyl, (1-methylbutyl)aminosulphonyl, (1-ethylpropyl)aminosulphonyl, neopentylaminosulphonyl, (1,1-dimethylpropyl)aminosulphonyl, hexylaminosulphonyl, (4-methylpentyl)aminosulphonyl, (3-methylpentyl)aminosulphonyl, (2-methylpentyl)-aminosulphonyl, (1-methylpentyl)aminosulphonyl, (1-ethylbutyl)aminosulphonyl, (2-ethylbutyl)aminosulphonyl, (3,3-dimethylbutyl)aminosulphonyl, (2,2-dimethylbutyl)-aminosulphonyl, (1,1-dimethylbutyl)aminosulphonyl, (2,3-dimethylbutyl)aminosulphonyl, (1,3-dimethylbutyl)aminosulphonyl or a (1,2-dimethylbutyl)aminosulphonyl group. In the di(C₁-C₆-alkyl)aminosulphonyl groups for the radicals R4 to R6, each of the two C₁-C₆-alkyl radicals on the nitrogen atom of the di(C₁-C₆-alkyl)aminosulphonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminosulphonyl groups for the radicals R4 to R6 may be for example a cyclopropylaminosulphonyl, cyclobutylaminosulphonyl, cyclopentylaminosulphonyl, cyclohexylaminosulphonyl or cycloheptylaminosulphonyl group.

In the di(C₃-C₇-cycloalkyl)aminosulphonyl groups for the radicals R4 to R6, each of the two C₃-C₇-cycloalkyl radicals on the nitrogen atom of the di(C₃-C₇-cycloalkyl)aminosulphonyl group may be independently of one another for example a cyclopropyl, cyclobutyl, cyclopentyl, cyclohexyl or cycloheptyl group.

In the C₃-C₇-cycloalkyl-C₁-C₆-alkyleneaminosulphonyl groups of the radicals R4 to R6, each of the C₃-C₇-cycloalkyl groups of the C₃-C₇-cycloalkyl-C₁-C₆-alkyleneaminosulphonyl groups, for example of a cyclopropyl, cyclobutyl, cyclopentyl, cyclohexyl or cycloheptyl group, can be combined independently of one another with each C₁-C₆-alkyleneaminosulphonyl group, for example with a methyleneaminosulphonyl, ethyleneaminosulphonyl, propyleneaminosulphonyl, butyleneaminosulphonyl, pentyleneaminosulphonyl, hexyleneaminosulphonyl group.

The C₁-C₆-alkylsulphonylamido groups for the radicals R4 to R6 may be for example a methylsulphonylamido, ethylsulphonylamido, propylsulphonylamido, isopropylsulphonylamido, butylsulphonylamido, isobutylsulphonylamido, sec-butylsulphonylamido, tert-butylsulphonylamido, pentylsulphonylamido, isopentylsulphonylamido, (2-methylbutyl)-sulphonylamido, (1-methylbutyl)sulphonylamido, (1-ethylpropyl)sulphonylamido, neo-pentylsulphonylamido, (1,1-dimethylpropyl)sulphonylamido, hexylsulphonylamido, (4-methylpentyl)sulphonylamido, (3-methylpentyl)sulphonylamido, (2-methylpentyl)-sulphonylamido, (1-methylpentyl)sulphonylamido, (1-ethylbutyl)sulphonylamido, (2-ethylbutyl)sulphonylamido, (3,3-dimethylbutyl)sulphonylamido, (2,2-dimethylbutyl)-sulphonylamido, (1,1-dimethylbutyl)sulphonylamido, (2,3-dimethylbutyl)sulphonylamido, (1,3-dimethylbutyl)sulphonylamido or a (1,2-dimethylbutyl)sulphonylamido group.

In the -N(C₀-C₆-alkyl)-C(O)-C₁-C₆-alkyl groups of the radicals R4 to R6, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₁-C₆-alkyl group on the carbonyl group of the amide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N-(C₀-C₆-alkyl)-C(O)-C₃-C₇-cycloalkyl groups of the radicals R4 to R6, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the carbonyl group of the amide, for example with a cyclopropyl, cyclobutyl, cyclopentyl, cyclohexyl or cycloheptyl group.

In the -N(C₀-C₆-alkyl)-C(O)-N-di(C₀-C₆-alkyl) groups of the radicals R4 to R6, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-O-(C₀-C₆-alkyl) groups of the radicals R4 to R6, both (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-NH-(C₃-C₇-cycloalkyl) groups of the radicals R4 to R6, each of the (C₀-C₆-alkyl) groups on the nitrogen atom of the -N(C₀-C₆-alkyl)-C(O)-NH-(C₃-C₇-cycloalkyl) groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₃-C₇-cycloalkyl group on the terminal nitrogen atom of the urea, for example with a cyclopropyl, cyclobutyl, cyclopentyl, cyclohexyl or cycloheptyl group.

In the -N(C₀-C₆-alkyl)-SO₂-(C₁-C₆-alkyl) groups of the radicals R4 to R6, each of the (C₀-C₆-alkyl) groups on the nitrogen atom of the -N(C₀-C₆-alkyl)-SO₂-(C₁-C₆-alkyl) group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₁-C₆-alkyl group on the sulphonyl group of the sulphonamide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-C₃-C₇-cycloalkyl groups of the radicals R4 to R6, each of the (C₀-C₆-alkyl) groups on the nitrogen atom of the -N(C₀-C₆-alkyl)-SO₂-C₃-C₇-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cydoalkyl group on the sulphonyl group, for example with a cyclopropyl, cyclobutyl, cyclopentyl, cyclohexyl or cycloheptyl group.

In the -N(C₀-C₆-alkyl)-SO₂-N-di(C₀-C₆-alkyl) groups of the radicals R4 to R6, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-NH-(C₃-C₇)-cycloalkyl groups of the radicals R4 to R6, the C₀-C₆-alkyl group of the -N(C₀-C₆-alkyl)-SO₂-NH-(C₃-C₇)-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group, for example with a cyclopropyl, cyclobutyl, cyclopentyl, cyclohexyl or cycloheptyl group.

In the -C(O)-N(H)-C₂-C₆-alkylene-(C₁-C₆-alkyl)amine groups of the radicals R4 to R6, each of the C₂-C₆-alkylene groups on the nitrogen atom of the -C(O)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-[di(C₁-C₆-alkyl)]amine groups of the radicals R4 to R6, each of the C₂-C₆-alkylene groups on the nitrogen atom of the -C(O)-N(H)-C₂-C₆-alkylene-[di(C₁-C₆-alkyl)]amine group, for example an ethylene, propylene, butylene, pentylene or hexylene group, may be combined independently of one another with each of the two identically or different C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-(C₃-C₇-cycloalkyl)amine groups of the radicals R4 to R6, each of the (C₂-C₆-alkylene) groups of the -C(O)-N(H)-C₂-C₆-alkylene-(C₃-C₇-cycloalkyl)amine group, for example an ethylene, propylene, butylene, pentylene or hexylene group, may be combined independently of one another with each C₃-C₇-cycloalkyl group on the amine, for example with a cyclopropyl, cyclobutyl, cyclopentyl, cyclohexyl or cycloheptyl group.

In the -C(O)-N(H)-C₂-C₆-alkylene-(C₃-C₇-Cycloalkyl-C₁-C₆-alkylene)amine groups of the radicals R4 to R6, each of the (C₂-C₆-alkylene) groups of the -C(O)-N(H)-C2-C6-alkylene-(C3-C6-cycloalkyl-C1-C6-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylene or cycloheptylhexylene group.

In the -S(O₂)-N(H)-C₂-C₆-alkylene-(C₁-C₆-alkyl)amine groups of the radicals R4 to R6, the (C₂-C₆-alkylene) groups of the -S(O₂)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-[di(C₁-C₆-alkyl)]amine groups of the radicals R4 to R6, the C₂-C₆-alkylene group of the -S(O₂)-N(H)-C₂-C₆-alkylene-(di(C₁-C₆-alkyl)]amine group, for example an ethylene, propylene, butylene, pentylene or hexylene group, may be combined independently of one another with each of the two C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-(C₃-C₇-cycloalkyl)amine groups of the radicals R4 to R6, the C₂-C₆-alkylene group of the -S(O₂)-N(H)-C₂-C₆-alkylene-(C₃-C₇-cycloalkyl)-amine group, for example an ethylene, propylene, butylene, pentylene or hexylene group, may be combined independently of one another with each C₃-C₇-cycloalkyl group on the amino group, for example with a cyclopropyl, cyclobutyl, cyclopentyl, cyclohexyl or cycloheptyl group.

In the -S(O₂)-N(H)-C₂-C₆-alkylene-(C₃-C₇-cycloalkyl-C₁-C₆-alkylene)amine groups of the radicals R4 to R6, each C₂-C₆-alkylene group of the -S(O₂)-N(H)-C₂-C₆-alkylene-(C₃-C₇-cydoalkyl-C₁-C₆-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylen or cycloheptylhexylene group.

In the -O-C₂-C₆-alkylene-(C₁-C₆-alkyl)amine groups of the radicals R4 to R6, the C₂-C₆-alkylene group of the -O-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -O-C₂-C₆-alkylene-[di(C₁-C₆-alkyl)]amine groups of the radicals R4 to R6, the C₂-C₆-alkylene group of the -O-C₂-C₆-alkylene-[di(C₁-C₆-alkyl)]amine group, for example an ethylene, propylene, butylene, pentylene or hexylene group, may be combined independently of one another with two freely selectable C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

Preferred according to the present invention is the use of compounds of formula I in which
- R3: may be hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups 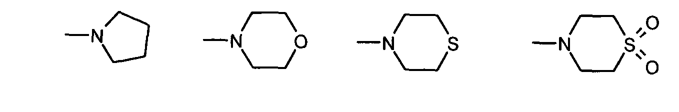 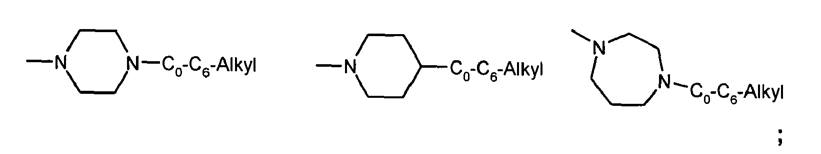
- R4: may be hydrogen, hydroxy, halogen, nitro, amino, cyano, phenyl, C₁-C₆-alkyl, C₂-C₆-alkenyl or C₂-C₆-alkynyl, C₃-C₇-cycloalkyl,
where the hydrocarbon chains therein may optionally be substituted one or more times by fluorine, cyano or the radicals: 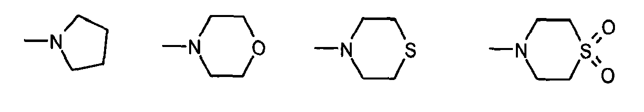 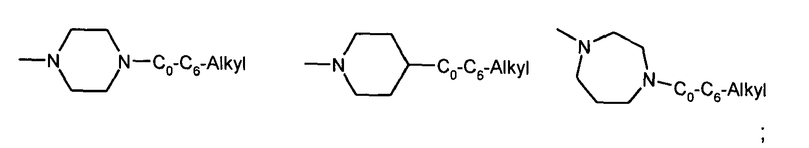 or
independently of one another hydroxy-C₁-C₆-alkylene, C₁-C₆-alkyloxy, C₁-C₆-alkyloxyphenyl-C₁-C₆-alkylene, phenyloxy-C₁-C₆-alkylene,
C₁-C₆-alkylamino, di(C₁-C₆-alkyl)amino
C₁-C₆-acyl-(C₀-C₆-alkyl)amido,
C₁-C₆-alkylcarbonyl,
carboxy, C₁-C₆-alkyloxycarbonyl,
C₁-C₃-alkylsulphanyl, C₁-C₆-alkysulphonyl,-N(C₀-C₆-alkyl)-C(O)-N-di(C₀-C₆-alkyl),
and
R1, R2, R5, R6, R7, R8, Q, X and W have the same meaning as defined in formula I.

The present invention also relates to compounds as such of formula I which are novel over JP11-3432795.

Preferred according to the present invention are the compounds of formula I and use thereof, namely

| | |
|---|---|
| **1** | 4,3',4',5'-Tetramethoxy-biphenyl-3-sulfonic acid [(R)-2-hydroxy-1-(1H-indol-3-yl-methyl)-ethyl]-amide |
| **10** | 4-Isopropoxy-3',4',5'-trimethoxy-biphenyl-3-sulfonic acid [(R)-2-hydroxy-1-(1H-indol-3-ylmethyl)-ethyl]-amide; |
| **11** | 5-Pyridin-2-yl-thiophene-2-sulfonic acid [(R)-2-hydroxy-1-(1H-indol-3-ylmethyl)-ethyl]-amide |
| **12** | 4'-Trifluoromethyl-biphenyl-3-sulfonic acid [(R)-2-hydroxy-1-(1H-indol-3-ylmethyl)-ethyl]-amide |
| **13** | 4'-Methyl-biphenyl-3-sulfonic acid [(R)-2-hydroxy-1-(1H-indol-3-ylmethyl)-ethyl]-amide |
| **14** | 5-(2-Methyl-5-trifluoromethyl-2H-pyrazol-3-yl)-thiophene-2-sulfonic acid [(R)-2-hydroxy-1-(1H-indol-3-ylmethyl)-ethyl]-amide. |

These compounds are not disclosed in JP11-3432795 and are thus novel.

Likewise, preferred are compounds of formula I and use thereof,
in which
- R3: may be hydroxy-C₃-C₆-alkenylene, hydroxy-C₃-C₆-alkynylene, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkylamino-C₁-C₆-alkylene, di(C₁-C₆-alkyl)amino-C₁-C₆-alkylene,
where the hydrocarbon chains therein may optionally be substituted one or more times by fluorine, cyano, hydroxy, amino or the groups 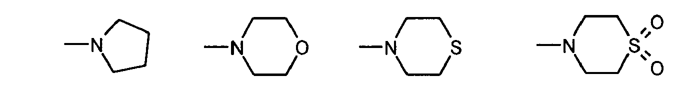 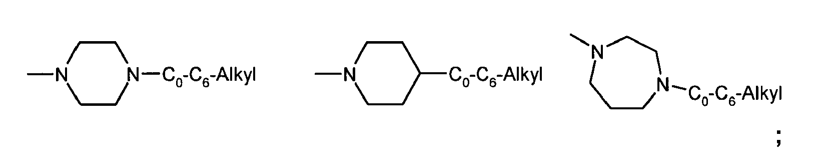
- R4: may be hydrogen, hydroxy, halogen, nitro, amino, cyano, phenyl, C₁-C₆-alkyl, C₂-C₆-alkenyl or C₂-C₆-alkynyl, C₃-C₇-cycloalkyl,
where the hydrocarbon chains therein may optionally be substituted one or more times by fluorine, cyano or the radicals: 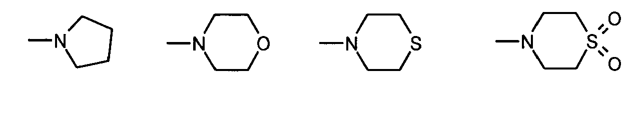 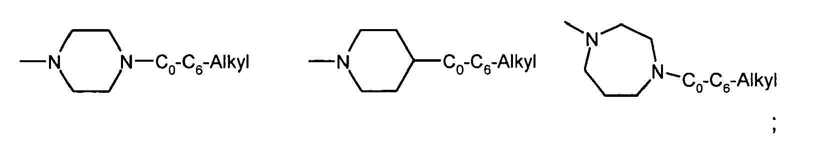 or
independently of one another hydroxy-C₁-C₆-alkylene, C₁-C₆-alkyloxyC₁-C₆-alkyloxyphenyl-C₁-C₆-alkylene, phenyloxy-C₁-C₆-alkylene,
C₁-C₆-alkylamino, di(C₁-C₆-alkyl)amino
C₁-C₆-acyl-(C₀-C₆-alkyl)amido,
C₁-C₆-alkylcarbonyl,
carboxy, C₁-C₆-alkyloxycarbonyl,
C₁-C₃-alkylsulphanyl, C₁-C₆-alkysulphonyl,-N(C₀-C₆-alkyl)-C(O)-N-di(C₀-C₆-alkyl),
and
R1, R2, R5, R6, R7, R8, Q, X and W have the same meaning as defined in formula I.

This subrange of formula I defined above does not overlap with the range of formulas diclosed in JP11-3432795 and is thus novel.

Likewise preferred according to the present invention are those compounds of formula I and use thereof
in which
- R4: may be C₃-C₇-cycloalkyl-C₁-C₆-alkylene, C₃-C₇-heterocycloalkyl,
where the hydrocarbon chains therein may optionally be substituted one or more times by fluorine, cyano or the radicals: 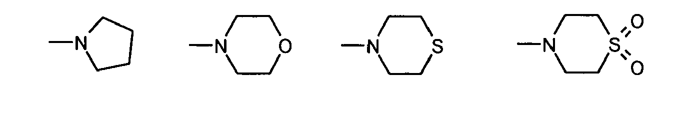 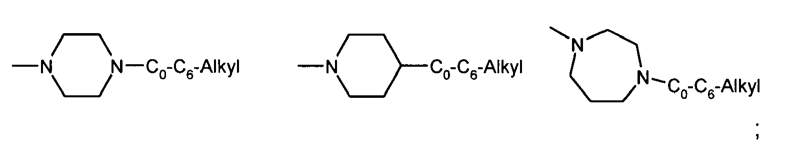 or
independently of one another hydroxy-C₃-C₆-alkenylene, hydroxy-C₃-C₆-alkynylene, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl,
C₃-C₇-cycloalkylcarbonyl,
carboxamido [-C(O)NH₂],
C₃-C₇-cycloalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-O-(C₀-C₆)alkyl, -N(C₀-C₆-alkyl)-C(O)-N H-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the radicals: 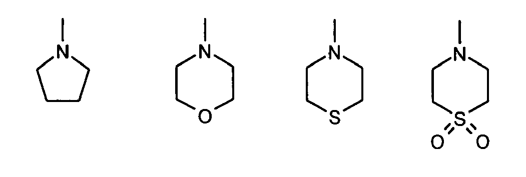 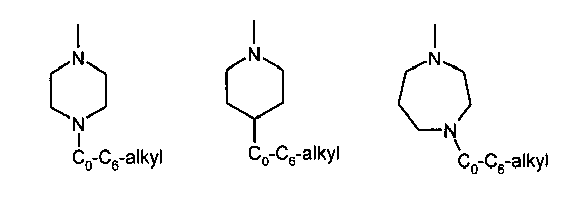 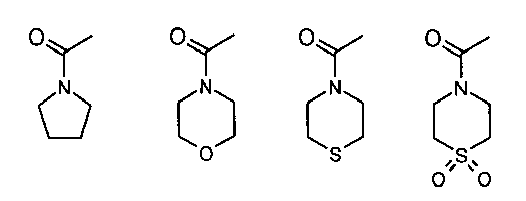 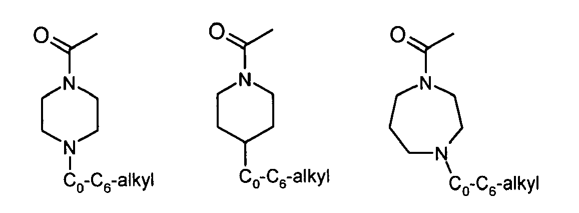 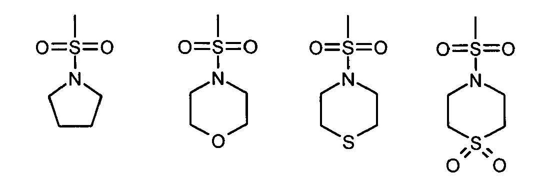 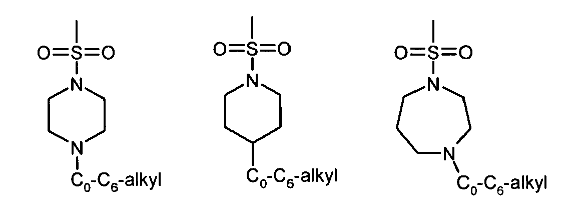 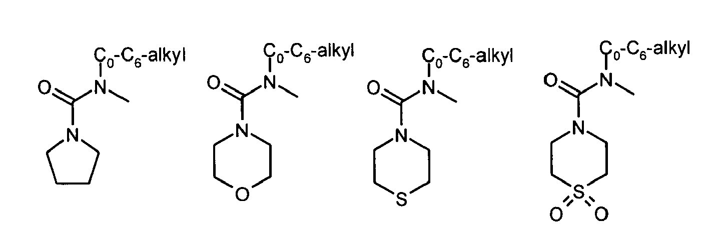 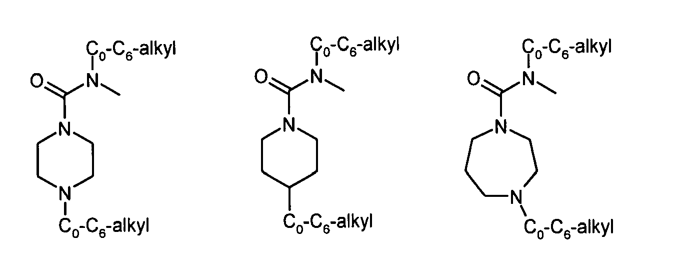 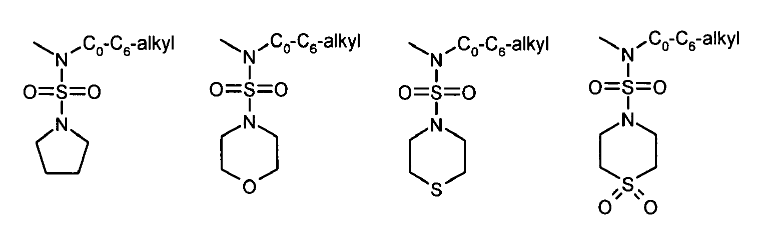 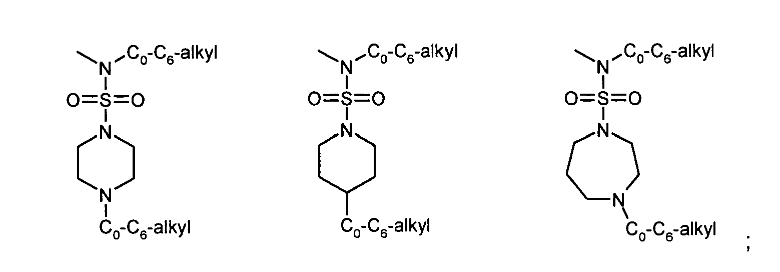
- R4 and R5: may together form heterocycloalkyl, cycloalkyl;
and the groups R1, R2, R3, R5, R6, R7, R8, Q, X and W have the same meaning as defined in formula I.

This subrange of formula I defined above does not overlap with the range of formulas diclosed in JP11-3432795 and is thus novel.

Particularly preferred are compounds of formula II and use thereof 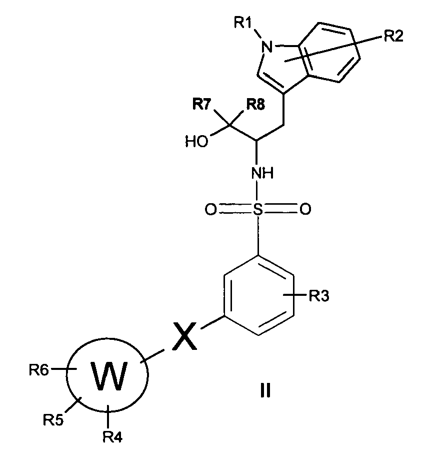 in which
- R4: may be C₃-C₇-cycloalkyl-C₁-C₆-alkylene, C₃-C₇-heterocydoalkyl,
where the hydrocarbon chains therein may optionally be substituted one or more times by fluorine, cyano or the radicals: 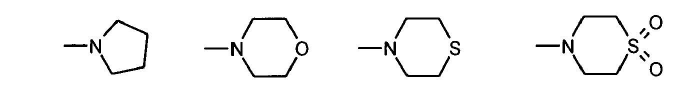 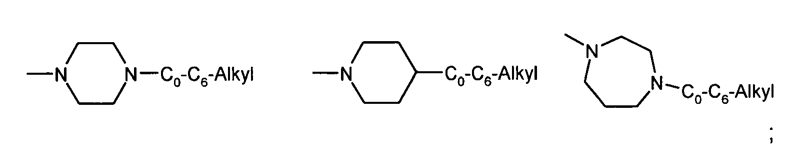 or
independently of one another hydroxy-C₃-C₆-alkenylene, hydroxy-C₃-C₆-alkynylene, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl,
C₃-C₇-cycloalkylcarbonyl,
carboxamido [-C(O)NH₂],
C₃-C₇-cycloalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-O-(C₀-C₆)alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the radicals: 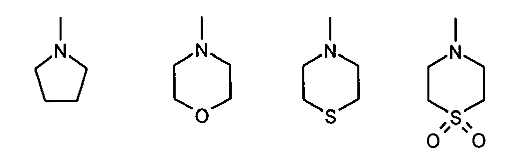 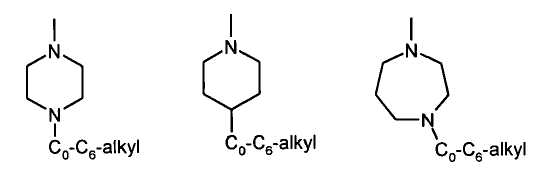 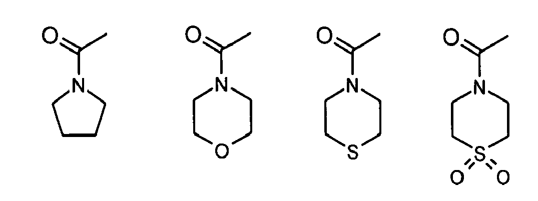 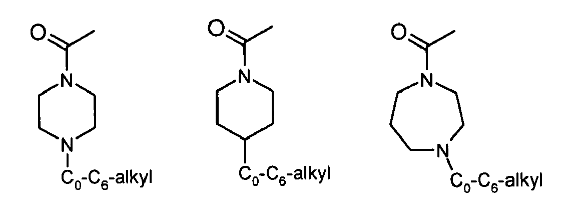 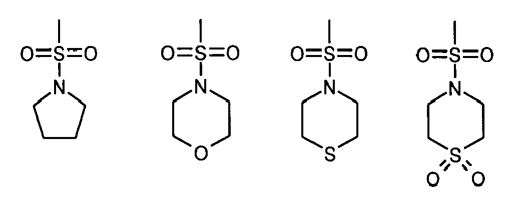 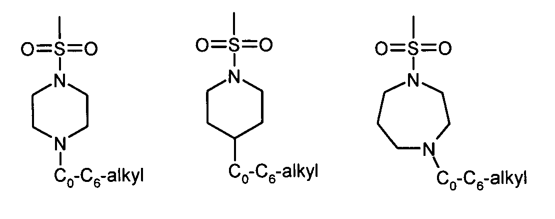 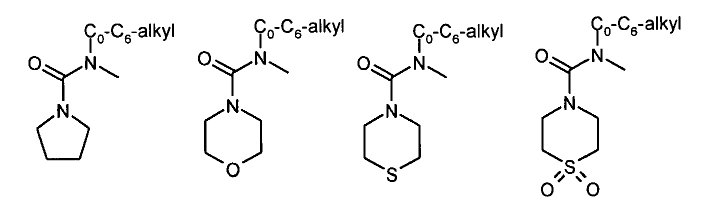 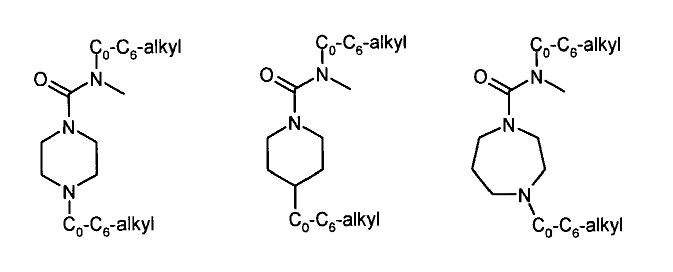 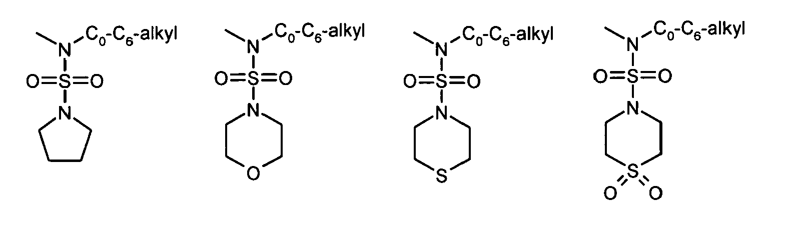 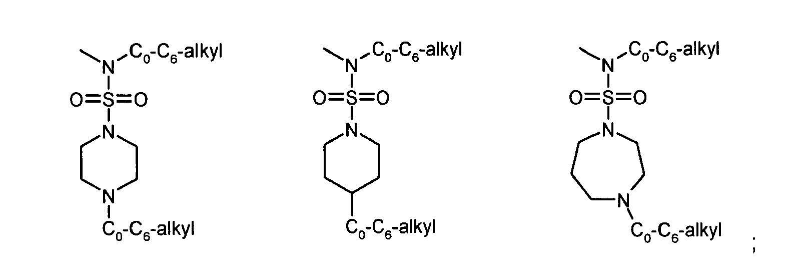
- R4 and R5: may together form heterocycloalkyl, cycloalkyl;
and
R1, R2, R3, R5, R6, R7, R8, X and W have the same meaning as defined in formula 1.

Formula II does not overlap with the range of the formula diclosed in JP11-3432795 and is thus novel.

More particularly preferred according to the present invention are compounds of formula II and use thereof, namely

| | |
|---|---|
| **2** | 3-Chloro-2'-[(R)-2-hydroxy-1-(1H-indol-3-ylmethyl)-ethylsulfamoyl]-biphenyl-4-carboxylic acid methylamide; |
| **3** | 3-Chloro-3'-[(R)-2-hydroxy-1-(1H-indol-3-ylmethyl)-ethylsulfamoyl]-4'-methoxy-biphenyl-4-carboxylic acid methylamide; |
| **4** | 3-Chloro-4'-[(R)-2-hydroxy-1-(1H-indol-3-ylmethyl)-ethylsulfamoyl]-biphenyl-4-carboxylic acid methylamide; |
| **5** | 3'-Chloro-4'-(morpholine-4-carbonyl)-5-trifluoromethyl-biphenyl-3-sulfonic acid [(R)-2-hydroxy-1-(1H-indol-3-ylmethyl)-ethyl]-amide; |
| **6** | 3-Chloro-3'-[(R)-2-hydroxy-1-(1H-indol-3-ylmethyl)-ethylsulfamoyl]-5'-trifluoromethyl-biphenyl-4-carboxylic acid methylamide; |
| **7** | 3-Chloro-3'-[(R)-2-hydroxy-1-(1H-indol-3-ylmethyl)-ethylsulfamoyl]-5'-trifluoromethyl-biphenyl-4-carboxylic acid amide; |
| **8** | 4'-Bromo-3-chloro-3'-[(R)-2-hydroxy-1-(H-indol-3-ylmethyl)-ethylsulfamoyl]-biphenyl-4-carboxylic acid amide; |
| **9** | 3-Chloro-3'-[(R)-2-hydroxy-1-(1H-indol-3-ylmethyl)-ethylsulfamoyl]-4'-iso-propoxy-biphenyl-4-carboxylic acid methylamide; |
| **15** | 3-Chloro-3'-[(R)-2-hydroxy-1-(1H-indol-3-ylmethyl)-2-methyl-propylsulfamoyl]-4'-methoxy-biphenyl-4-carboxylic acid amide; |
| **16** | 3-Chloro-3'-[(R)-2-hydroxy-1-(1H-indol-3-ylmethyl)-2-methyl-propylsulfamoyl]-4'-methoxy-biphenyl-4-carboxylic acid methylamide; |
| **17** | 3'-Chloro-4-methoxy-4'-(morpholine-4-carbonyl)-biphenyl-3-sulfonic acid [(R)-2-hydroxy-1-(1H-indol-3-ylmethyl)-2-methyl-propyl]-amide; |
| **18** | 4-{3-[(R)-2-Hydroxy-1-(1H-indol-3-ylmethyl)-ethylsulfamoyl]-4-methoxy-phenylethynyl}-N-methyl-benzamide |
| **19** | 3-{3-[(R)-2-Hydroxy-1-(1H-indol-3-ylmethyl)-ethylsulfamoyl]-4-methoxy-phenylethynyl}-N-methyl-benzamide; |
| **20** | 3-{3-[(R)-2-Hydroxy-1-(1H-indol-3-ylmethyl)-ethylsulfamoyl]-4-propoxy-phenylethynyl}-N-methyl-benzamide ; |
| **21** | 4-{3-[(R)-2-Hydroxy-1-(1H-indol-3-ylmethyl)-ethylsulfamoyl]-4-propoxy-phenylethynyl}-N-methyl-benzamide; |
| **22** | 4-{3-[(R)-2-Hydroxy-1-(1H-indol-3-ylmethyl)-2-methyl-propylsulfamoyl]-4-methoxy-phenylethynyl}-N-methyl-benzamide |
| **23** | 3-{3-[(R)-2-Hydroxy-1-(1H-indol-3-ylmethyl)-2-methyl-propylsulfamoyl]-4-methoxy-phenylethynyl}-N-methyl-benzamide; |
| **24** | 2-Chloro-4-{7-[(R)-2-hydroxy-1-(1H-indol-3-ylmethyl)-ethylsulfamoyl]-2,3-dihydro-benzofuran-5-yl}-N-methyl-benzamide |
| **35** | 2-{7-[(R)-2-Hydroxy-1-(1H-indol-3-ylmethyl)-ethylsulfamoyl]-2,3-dihydro-benzofuran-5-yl}-benzamide; |
| **38** | 4-{7-[(R)-2-Hydroxy-1-(1H-indol-3-ylmethyl)-ethylsulfamoyl]-2,3-dihydro-benzofuran-5-yl}-N-methyl-benzamide; |
| **42** | 3-Fluoro-5-{7-[(R)-2-hydroxy-1-(1H-indol-3-ylmethyl)-ethylsulfamoyl]-2,3-dihydro-benzofuran-5-yl}-N-methyl-benzamide; |
| **47** | 3-{7-[(R)-2-Hydroxy-1-(1H-indol-3-ylmethyl)-ethylsulfamoyl]-2,3-dihydro-benzofuran-5-yl}-benzamide; |
| **50** | N-(3-{7-[(R)-2-Hydroxy-1-(1H-indol-3-ylmethyl)-ethylsulfamoyl]-2,3-dihydro-benzofuran-5-yl}-benzyl)-acetamide; |
| **52** | 5-(4-Cyanomethyl-phenyl)-2,3-dihydro-benzofuran-7-sulfonic acid [(R)-2-hydroxy-1-(1H-indol-3-ylmethyl)-ethyl]-amide; |
| **54** | 5-Benzo[1,3]dioxol-5-yl-2,3-dihydro-benzofuran-7-sulfonic acid [(R)-2-hydroxy-1-(1H-indol-3-ylmethyl)-ethyl]-amide; |
| **58** | 5-(3-Methanesulfonylamino-phenyl)-2,3-dihydro-benzofuran-7-sulfonic acid [(R)-2-hydroxy-1-(1H-indol-3-ylmethyl)-ethyl]-amide. |

Likewise preferred according to the present invention are compounds of formula III and use thereof 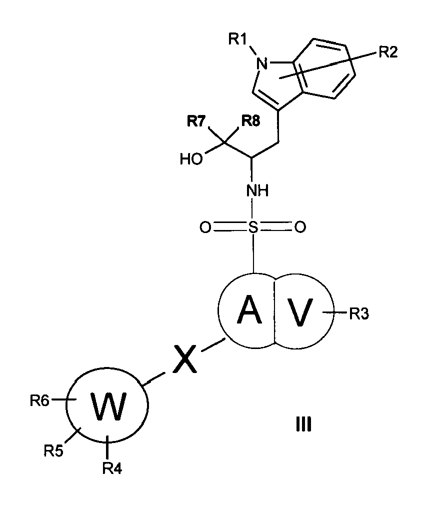 in which
- R4: may be hydrogen, hydroxy, halogen, nitro, amino, cyano, phenyl, C₁-C₆-alkyl, C₂-C₆-alkenyl or C₂-C₆-alkynyl, C₃-C₇-cycloalkyl,
where the hydrocarbon chains therein may optionally be substituted one or more times by fluorine, cyano or the radicals: 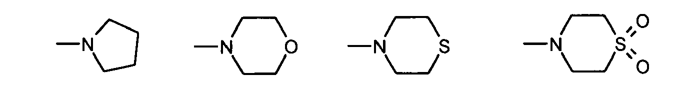 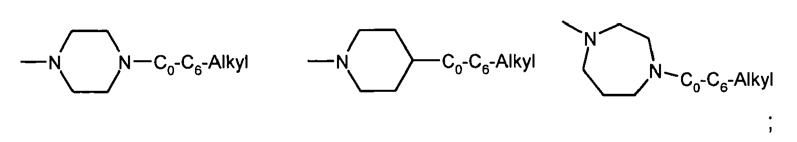 or
independently of one another hydroxy-C₁-C₆-alkylene, C₁-C₆-alkyloxy, C₁-C₆-alkyloxyphenyl-C₁-C₆-alkylene, phenyloxy-C₁-C₆-alkylene, C₁-C₆-alkylamino, di(C₁-C₆-alkyl)amino
C₁-C₆-acyl-(C₀-C₆-alkyl)amido,
C₁-C₆-alkylcarbonyl,
carboxy, C₁-C₆-alkyloxycarbonyl,
C₁-C₃-alkylsulphanyl, C₁-C₆-alkysulphonyl,-N(C₀-C₆-alkyl)-C(O)-N-di(C₀-C₆-alkyl);
and
R1, R2, R3, R5, R6, R7, R8, X, V and W have the same meaning as defined in formula I.

Formula III does not overlap with the range of the formula diclosed in JP11-3432795 and is thus novel.

Particularly preferred are compounds of formula IV and use thereof 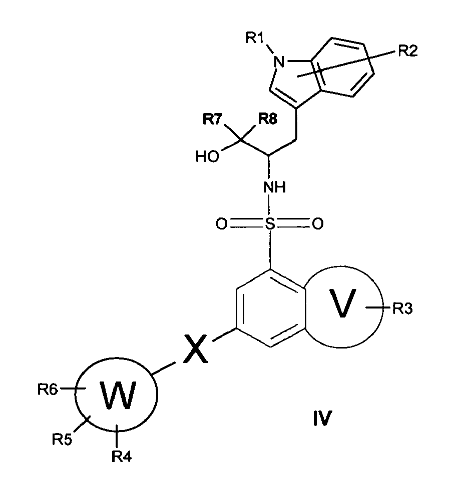 in which
- R4: may be hydrogen, hydroxy, halogen, nitro, amino, cyano, phenyl, C₁-C₆-alkyl, C₂-C₆-alkenyl or C₂-C₆-alkynyl, C₃-C₇-cycloalkyl,
where the hydrocarbon chains therein may optionally be substituted one or more times by fluorine, cyano or the radicals: 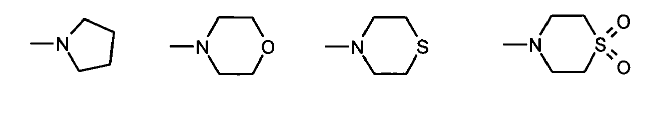 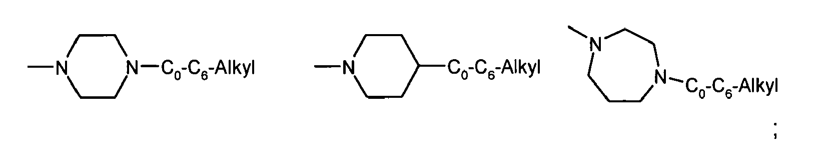 or
independently of one another hydroxy-C₁-C₆-alkylene, C₁-C₆-alkyloxy, C₁-C₆-alkyloxyphenyl-C₁-C₆-alkylene, phenyloxy-C₁-C₆-alkylene, C₁-C₆-alkylamino, di(C₁-C₆-alkyl)amino
C₁-C₆-acyl-(C₀-C₆-alkyl)amido,
C₁-C₆-alkylcarbonyl,
carboxy, C₁-C₆-alkyloxycarbonyl,
C₁-C₃-alkylsulphanyl, C₁-C₆-alkysulphonyl,-N(C₀-C₆-alkyl)-C(O)-N-di(C₀-C₆-alkyl);
and
R1, R2, R3, R5, R6, R7, R8, A, V, X and W have the same meaning as defined in formula 1.

More particularly preferred are compounds of formula IV and use thereof, namely

| | |
|---|---|
| **25** | 5-(3,4,5-Trimethoxy-phenyl)-2,3-dihydro-benzofuran-7-sulfonic acid [(R)-2-hydroxy-1-(1H-indol-3-ylmethyl)-ethyl]-amide; |
| **26** | 5-(4-Acetyl-phenyl)-2,3-dihydro-benzofuran-7-sulfonic acid [(R)-2-hydroxy-1-(1H-indol-3-ylmethyl)-ethyl]-amide |
| **27** | 5-(4-Methylsulfanyl-phenyl)-2,3-dihydro-benzofuran-7-sulfonic acid [(R)-2-hydroxy-1-(1H-indol-3-ylmethyl)-ethyl]-amide; |
| **28** | 5-(3-Amino-phenyl)-2,3-dihydro-benzofuran-7-sulfonic acid [(R)-2-hydroxy-1-(1H-indol-3-ylmethyl)-ethyl]-amide; |
| **29** | 5-(3-Trifluoromethyl-phenyl)-2,3-dihydro-benzofuran-7-sulfonic acid [(R)-2-hydroxy-1-(1H-indol-3-ylmethyl)-ethyl]-amide; |
| **30** | 5-(4-Hydroxy-phenyl)-2,3-dihydro-benzofuran-7-sulfonic acid [(R)-2-hydroxy-1-(1H-indol-3-ylmethyl)-ethyl]-amide; |
| **31** | 5-(4-Fluoro-phenyl)-2,3-dihydro-benzofuran-7-sulfonic acid [(R)-2-hydroxy-1-(1H-indol-3-ylmethyl)-ethyl]-amide; |
| **32** | 5-(4-Cyano-phenyl)-2,3-dihydro-benzofuran-7-sulfonic acid [(R)-2-hydroxy-1-(1H-indol-3-ylmethyl)-ethyl]-amide; |
| **33** | 5-Naphthalen-1-yl-2,3-dihydro-benzofuran-7-sulfonic acid [(R)-2-hydroxy-1-(1H-indol-3-ylmethyl)-ethyl]-amide; |
| **34** | 5-(4-Chloro-phenyl)-2,3-dihydro-benzofuran-7-sulfonic acid [(R)-2-hydroxy-1-(1H-indol-3-ylmethyl)-ethyl]-amide; |
| **36** | 5-(6-Methoxy-pyridin-3-yl)-2,3-dihydro-benzofuran-7-sulfonic acid [(R)-2-hydroxy-1-(1H-indol-3-ylmethyl)-ethyl]-amide; |
| **37** | 5-(2-Fluoro-phenyl)-2,3-dihydro-benzofuran-7-sulfonic acid [(R)-2-hydroxy-1-(1H-indol-3-ylmethyl)-ethyl]-amide; |
| **39** | 5-Quinolin-6-yl-2,3-dihydro-benzofuran-7-sulfonic acid [(R)-2-hydroxy-1-(1H-indol-3-ylmethyl)-ethyl]-amide; |
| **40** | 5-((E)-Styryl)-2,3-dihydro-benzofuran-7-sulfonic acid [(R)-2-hydroxy-1-(1H-indol-3-ylmethyl)-ethyl]-amide; |
| **41** | 5-(3-Hydroxymethyl-phenyl)-2,3-dihydro-benzofuran-7-sulfonic acid [(R)-2-hydroxy-1-(1H-indol-3-ylmethyl)-ethyl]-amide; |
| **43** | 5-(3-Fluoro-5-methoxy-phenyl)-2,3-dihydro-benzofuran-7-sulfonic acid [(R)-2-hydroxy-1-(1H-indol-3-ylmethyl)-ethyl]-amide; |
| **44** | N-(4-{7-[(R)-2-Hydroxy-1-(1H-indol-3-ylmethyl)-ethylsulfamoyl]-2,3-dihydro-benzofuran-5-yl}-phenyl)-acetamide; |
| **45** | 5-(3,5-Dimethyl-phenyl)-2,3-dihydro-benzofuran-7-sulfonic acid [(R)-2-hydroxy-1-(1H-indol-3-ylmethyl)-ethyl]-amide; |
| **46** | 5-Quinolin-3-yl-2,3-dihydro-benzofuran-7-sulfonic acid [(R)-2-hydroxy-1-(1H-indol-3-ylmethyl)-ethyl]-amide; |
| **48** | 5-(2-Fluoro-pyridin-3-yl)-2,3-dihydro-benzofuran-7-sulfonic acid [(R)-2-hydroxy-1-(1H-indol-3-ylmethyl)-ethyl]-amide; |
| **49** | 5-(5-Cyano-thiophen-2-yl)-2,3-dihydro-benzofuran-7-sulfonic acid [(R)-2-hydroxy-1-(1H-indol-3-ylmethyl)-ethyl]-amide; |
| **51** | 5-(2-Methoxy-pyrimidin-5-yl)-2,3-dihydro-benzofuran-7-sulfonic acid [(R)-2-hydroxy-1-(1H-indol-3-ylmethyl)-ethyl]-amide; |
| **53** | 5-(3-Cyano-phenyl)-2,3-dihydro-benzofuran-7-sulfonic acid [(R)-2-hydroxy-1-(1H-indol-3-ylmethyl)-ethyl]-amide; |
| **55** | N-(3-{7-[(R)-2-Hydroxy-1-(1H-indol-3-ylmethyl)-ethylsulfamoyl]-2,3-dihydro-benzofuran-5-yl}-phenyl)-acetamide; |
| **56** | 5-Biphenyl-2-yl-2,3-dihydro-benzofuran-7-sulfonic acid [(R)-2-hydroxy-1-(1H-indol-3-ylmethyl)-ethyl]-amide; |
| **57** | 5-o-Tolyl-2,3-dihydro-benzofuran-7-sulfonic acid [(R)-2-hydroxy-1-(1H-indol-3-ylmethyl)-ethyl]-amide; |
| **59** | 5-(4-Trifluoromethyl-phenyl)-2,3-dihydro-benzofuran-7-sulfonic acid [(R)-2-hydroxy-1-(1H-indol-3-ylmethyl)-ethyl]-amide; |
| **60** | 5-Benzo[b]thiophen-3-yl-2,3-dihydro-benzofuran-7-sulfonic acid [(R)-2-hydroxy-1-(1H-indol-3-ylmethyl)-ethyl]-amide; |
| **61** | 5-Biphenyl-3-yl-2,3-dihydro-benzofuran-7-sulfonic acid [(R)-2-hydroxy-1-(1H-indol-3-ylmethyl)-ethyl]-amide; |
| **62** | 5-(3-Acetyl-phenyl)-2,3-dihydro-benzofuran-7-sulfonic acid [(R)-2-hydroxy-1-(1H-indol-3-ylmethyl)-ethyl]-amide; |
| **63** | 5-(3-Fluoro-phenyl)-2,3-dihydro-benzofuran-7-sulfonic acid [(R)-2-hydroxy-1-(1H-indol-3-ylmethyl)-ethyl]-amide; |
| **64** | 2',3'-Dihydro-[2,5']bibenzofuranyl-7'-sulfonic acid [(R)-2-hydroxy-1-(1H-indol-3-ylmethyl)-ethyl]-amide; |
| **65** | 5-Benzo[b]thiophen-2-yl-2,3-dihydro-benzofuran-7-sulfonic acid [(R)-2-hydroxy-1-(1H-indol-3-ylmethyl)-ethyl]-amide; |
| **66** | 5-(3-Chloro-phenyl)-2,3-dihydro-benzofuran-7-sulfonic acid [(R)-2-hydroxy-1-(1H-indol-3-ylmethyl)-ethyl]-amide; |
| **67** | 5-p-Tolyl-2,3-dihydro-benzofuran-7-sulfonic acid [(R)-2-hydroxy-1-(1H-indol-3-ylmethyl)-ethyl]-amide; |
| **68** | 5-Naphthalen-2-yl-2,3-dihydro-benzofuran-7-sulfonic acid [(R)-2-hydroxy-1-(1H-indol-3-ylmethyl)-ethyl]-amide; |
| **69** | 5-(3-Methoxy-phenyl)-2,3-dihydro-benzofuran-7-sulfonic acid [(R)-2-hydroxy-1-(1H-indol-3-ylmethyl)-ethyl]-amide; |
| **70** | 5-(4-Methoxy-phenyl)-2,3-dihydro-benzofuran-7-sulfonic acid [(R)-2-hydroxy-1-(1H-indol-3-ylmethyl)-ethyl]-amide; |
| **71** | 5-Thiophen-3-yl-2,3-dihydro-benzofuran-7-sulfonic acid [(R)-2-hydroxy-1-(1H-indol-3-ylmethyl)-ethyl]-amide. |

The present invention also relates to a process for preparing the compounds according to the invention. Compounds of the general formula I can be prepared as shown in Scheme 1 by an sulfonamide-formation reaction between the tryptophanol derivative V and the sulfonyl chloride VI. The sulfonyl chloride VI reacts with the tryptophanol derivative V in a suitable solvent and where appropriate in the presence of a base (an organic or inorganic base) which are known to the skilled person. It is possible to generate the sulfonyl chloride VI in situ from the corresponding sulfonic acid by treatment with reagents known to the skilled person, for example thionyl chloride, POCl₃, sulfonyl chloride, PCl₃ or PCl₅.

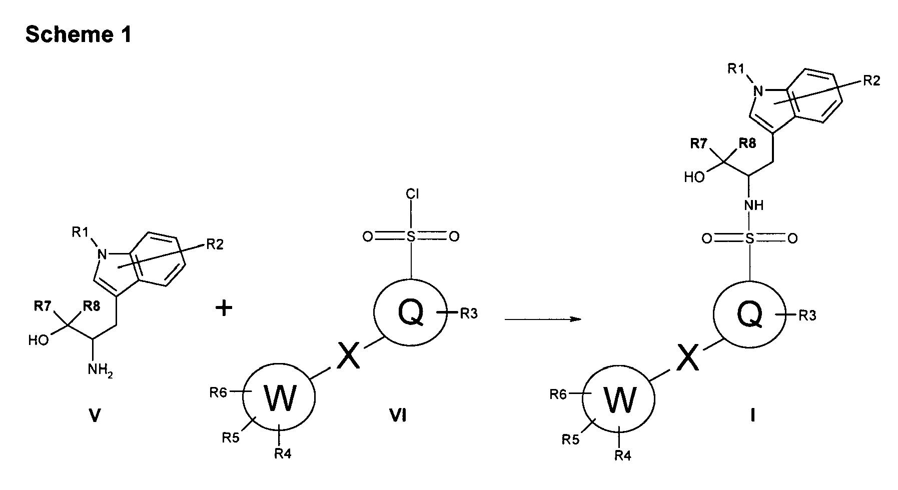

Compounds of the general formula II can be prepared as shown in Scheme 2 by an sulfonamide-formation reaction between the tryptophanol derivative V and the sulfonyl chloride VII. The sulfonyl chloride reacts with the tryptophanol derivative in a suitable solvent and where appropriate in the presence of a base which are known to the skilled person. It is possible to generate the sulfonyl chloride in situ from the corresponding sulfonic acid by treatment with reagents known to the skilled person, for example thionyl chloride, POCl₃, sulfonyl chloride, PCl₃ or PCl₅.

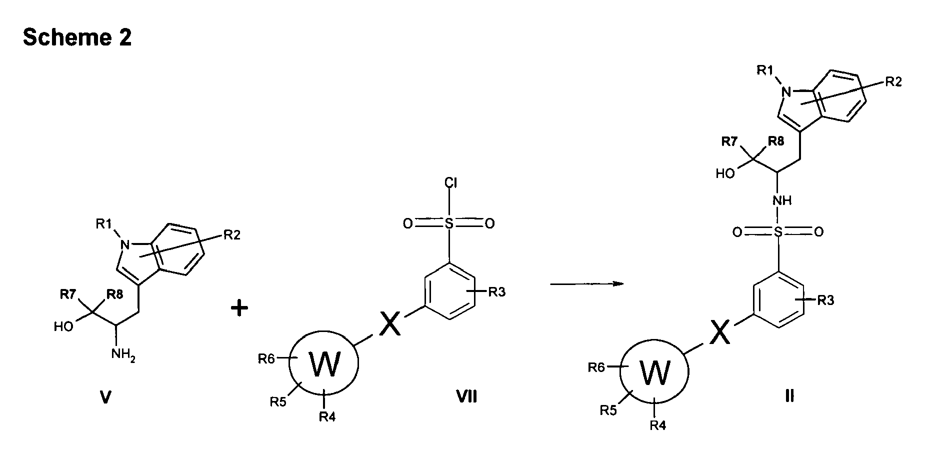

Compounds of the general formula III can be prepared as shown in Scheme 3 by an sulfonamide-formation reaction between the tryptophanol derivative V and the sulfonyl chloride VIII. The sulfonyl chloride VIII reacts with the tryptophanol derivative V in a suitable solvent and where appropriate with a base which are known to the skilled person. It is possible to generate the sulfonyl chloride in situ from the corresponding sulfonic acid by treatment with reagents known to the skilled person, for example thionyl chloride, POCI₃, sulfonyl chloride, PCI₃ or PCl₅.

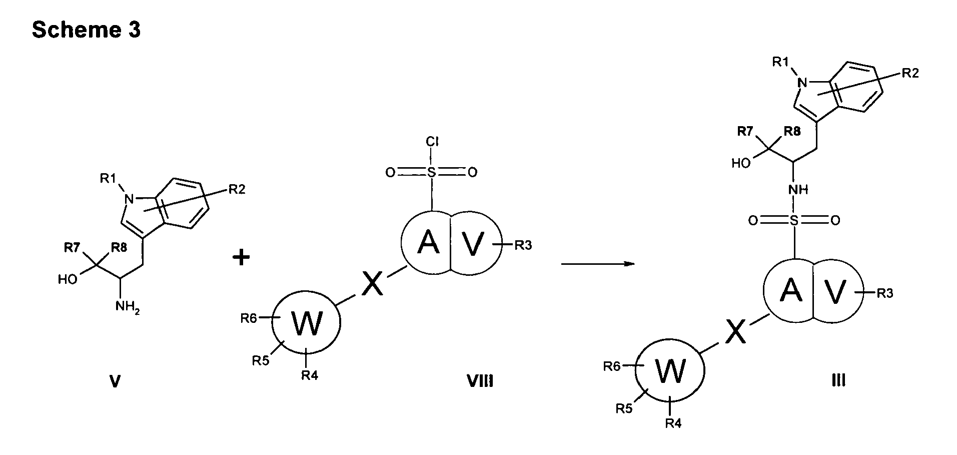

Compounds of the general formula IV can be prepared as shown in Scheme 4 by an sulfonamide-formation reaction between the tryptophanol derivative V and the sulfonyl chloride IX. The sulfonyl chloride IX reacts with the tryptophanol derivative V in a suitable solvent and where appropriate with a base which are known to the skilled person. It is possible to generate the sulfonyl chloride in situ from the corresponding sulfonic acid by treatment with reagents known to the skilled person, for example thionyl chloride, POCl₃, sulfonyl chloride, PCl₃ or PCl₅.

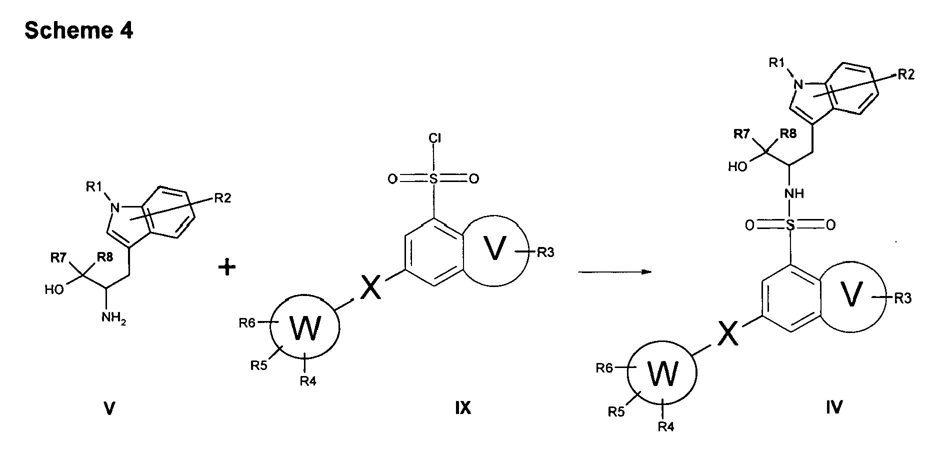

Alternatively, compounds of the general formula I can also be prepared as shown in Scheme 5 by a metal catalyzed cross coupling reaction between the aryl halide XI (wherein Hal stands for chlorine, bromine or iodine) and the correspondingly functionalized building block X known to the skilled person, for example: a Suzuki reaction (R = -B(OH)₂), a Sonogashira coupling (R = H, X = -C≡C-), a Negishi coupling (R = Zn-Hal) or a Stille coupling (R = Sn(alkyl)₃). Preferred metal catalysts used are typically those containing palladium or nickel, depending on the nature of the cross-coupling reaction. For a more detailed description of the metal catalyzed cross-coupling reactions applied and applicable for the synthesis of compounds of the formula I, see e.g. S. Takahashi, Y. Kuroyama, K. Sonogashira and N. Hagihara, Synthesis, 1980, 627; Metal catalyzed Cross-coupling Reactions, ed. F. Diederich and P. J. Stang, Wiley-VCH, Weinheim, 1998; K. Sonogashira, J. Organomet. Chem., 2002, 653 (1-2), 46.

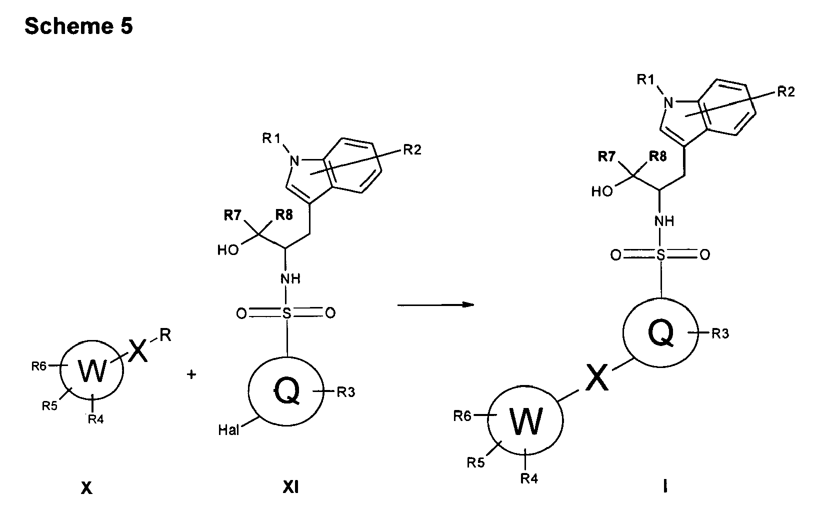

Likewise, compounds of the general formula II can be prepared as shown in Scheme 6 by a metal catalyzed cross coupling reaction between the aryl halide XII and the correspondingly functionalized building block X known to the skilled person.

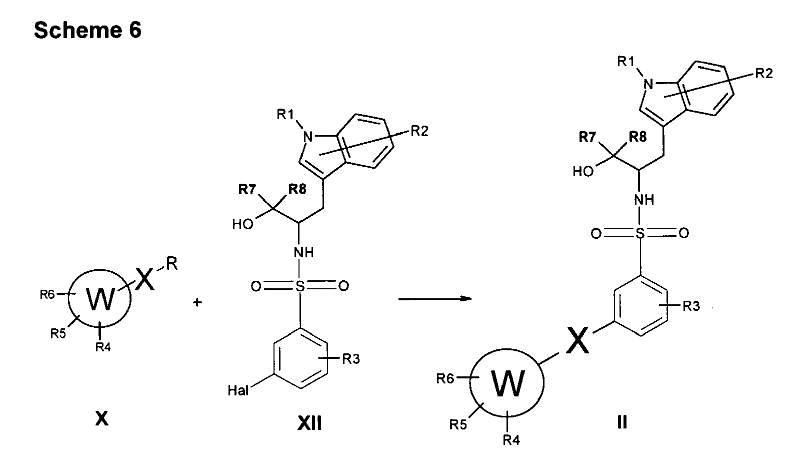

Likewise, compounds of the general formula III can be prepared as shown in Scheme 7 by a metal catalyzed cross coupling reaction between the aryl halide XIII and the correspondingly functionalized building block X known to the skilled person.

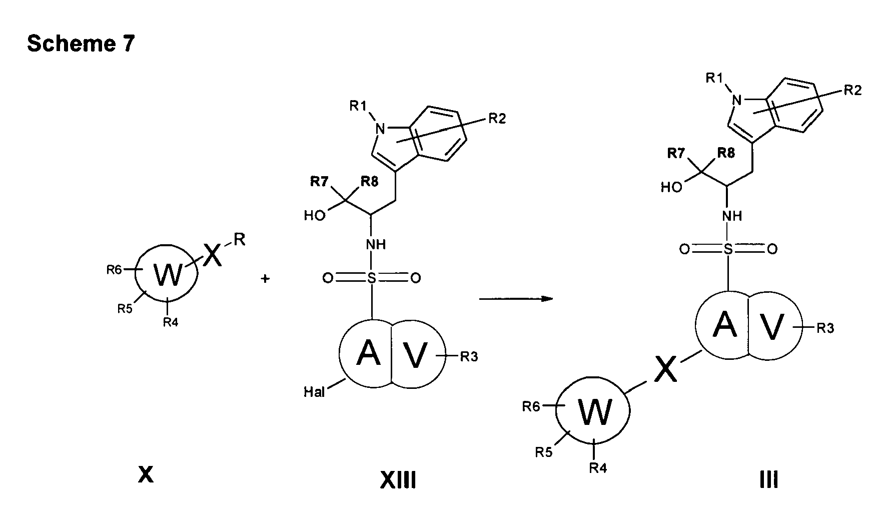

Likewise, compounds of the general formula IV can be prepared as shown in Scheme 8 by a metal catalyzed cross coupling reaction between the aryl halide XIV and the correspondingly functionalized building block X known to the skilled person.

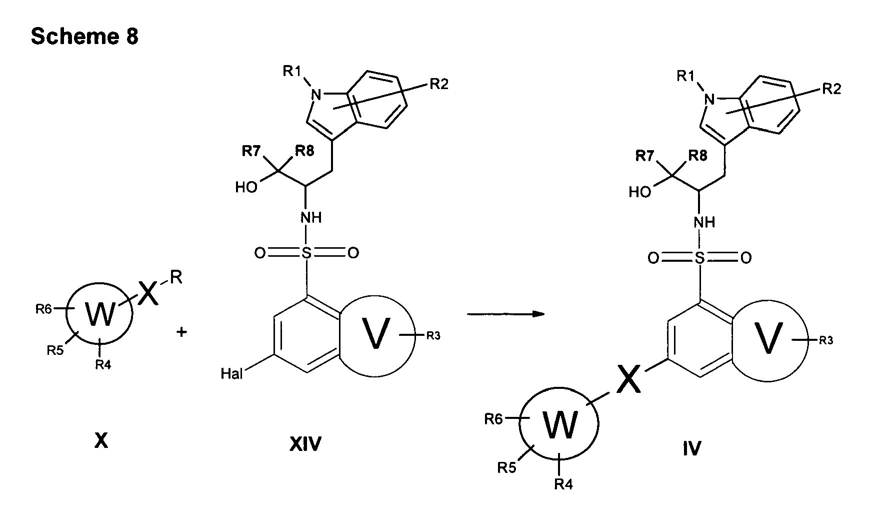

The present invention further relates to the aryl halides of the formulae XI, XII, XIII and XIV as intermediates of the process according to the invention for preparing the compounds according to the invention, namely:
5-Bromo-N-[(R)-2-hydroxy-1-(1H-indol-3-ylmethyl)-ethyl]-2-methoxy-benzenesulfonamide
2-Bromo-N-[(R)-2-hydroxy-1-(1H-indol-3-ylmethyl)-ethyl]-benzenesulfonamide
4-Bromo-N-[(R)-2-hydroxy-1-(1H-indol-3-ylmethyl)-ethyl]-benzenesulfonamide
3-Bromo-N-[(R)-2-hydroxy-1-(1H-indol-3-ylmethyl)-ethyl]-5-trifluoromethylbenzenesulfonamide
2,5-Dibromo-N-[(R)-2-hydroxy-1-(1H-indol-3-ylmethyl)-ethyl]-benzenesulfonamide
5-Bromo-N-[(R)-2-hydroxy-1-(1H-indol-3-ylmethyl)-ethyl]-2-isopropoxy benzenesulfonamide
5-Bromo-N-[(R)-2-hydroxy-1-(1H-indol-3-ylmethyl)-2-methyl-propyl]-2-methoxy-benzenesulfonamide
5-Bromo-N-[(R)-2-hydroxy-1-(1H-indol-3-ylmethyl)-ethyl]-2-propoxy-benzenesulfonamide
5-Bromo-2,3-dihydro-benzofuran-7-sulfonic acid [(R)-2-hydroxy-1-(1H-indol-3-ylmethyl)-ethyl]-amide

### Pharmacological Experiments

### HTRF assay for measuring cAMP in cells

The method is based on a competitive immunoassay between native cAMP, which has been produced by the cells, and cAMP which is labelled with cAMPD2 conjugate. The tracer binding was visualized by a monoclonal antibody, anti-cAMP labelled with cryptate [HTRF = homogeneous time-resolved fluorescence].
The specific signal is inversely proportional to the cAMP concentration of the samples employed.
The 665nm/ 620nm fluorescence ratio was evaluated.

*The following material was used: 96-well plates for the tissue culture, 96-well plates with black edge and black base (e.g. Fluotrac 600 from Greiner), 96-well plates for the substance dilutions of polypropylene and cAMP Femtomolar (4000wells Kit, CIS Bio International # 62AM5PEJ).*
The following reagents were used: BSA (bovine serum albumin) Fraction V protease-free, IBMX (3-isobutyl-1-methylxanthine), hFSH (human follicle stimulating hormone), Triton X-100 analytical grade, potassium fluoride analytical grade, G 418 (Geneticin) and Accutase.
Buffer 1 (washing and testing buffer) contained PBS, 1 mM CaCl2, 1 mM MgCl₂, 0.2% glucose; 0.1% BSA, 1 mM IBMX.
Buffer 2 (2x lysis buffer) contained 1% Triton X-100 in PBS (without CaCl₂ and MgCl₂).
Buffer 3 (assay buffer) contained 50 mM potassium phosphate buffer (pH 7.0); 800 mM potassium fluoride; 0.2% BSA (always added fresh).

### Procedure:

On day 1, the cells were seeded in 96-well plates (3x10⁴ cells per well hFSHR clone 16 cells (CHO cells stably transfected with the human FSH receptor in 150 µl of medium). The next day, test substance dilutions were made up. For this purpose, all the substances were diluted in ice-cold buffer 1 (with or without hFSH), and the substance dilutions were placed on ice until applied to the cells.

The cell supernatant was then aspirated off, and the cells were washed 2x with 200 µl of buffer 1. The cells were treated with 60 µl of the appropriate substance concentrations at 37°C for 2h. The cells were then lysed with 60 µl of buffer 2 (put onto the supernatant) (on a plate shaker at RT for 30 min).

The test conjugates (cAMP-D2 and anti-cAMP cryptate, CIS Bio) were diluted in buffer 3 in accordance with the manufacturers' information. The actual mixture for measurement was pipetted into a black 96-well plate (in each case 15 µl of the cell lysate diluted with 35 µl of buffer 1; firstly 25 µl of cAMP-D2 conjugate were pipetted and, after 10 min, 25 µl of the anti-cAMP cryptate were added). This is followed by incubation at RT for 90 minutes. The measurement was carried out in a PheraStar (BMG).

### Tissue culture conditions

- 1) hFSHr clone 16: Ham's F12
PSG
10% FCS
700 µg/ml G 418 (Geneticin) from PAA.

Dose-effect curve (hFSH) for the human receptor: 1e-8, 3e-9, 1e-9, 3e-10, 1e-10, 3e-11, 1e-11, 3e-12 mol/l.

The test substances were employed in suitable dilutions in the absence (test for agonism) and in the presence of 1 e-9 mol/I hFSH.

### Evaluation

The values of the well ratio were averaged and then entered directly in SigmaPlot versus the concentrations. The maximum and minimum values were determined for each plate, and half the difference is to be regarded as IC₅₀.

The test results (Table 1) show that the compounds according to the invention have an FSH-antagonistic effect.

**Table 1. FSH antagonistic effect of selected compounds in the HTRF assay**

| **Compound [Ex. #]** | **IC₅₀** |
|---|---|
| **1** | **3.5 µM** |
| **3** | **4 µM** |
| **9** | **180 nM** |
| **18** | **2.5 µM** |
| **22** | **3.5 µM** |
| **39** | **5 µM** |
| **52** | **10 µM** |

Being antagonists of the FSH receptor, compounds of the general formula I and their pharmaceutically acceptable salts can thus be used for the fertility control in male and/or in a female animals, in particular in men and/or women; as well as for the treatment and/or prevention of osteoporosis.

### Pharmaceutical compositions

The invention further relates to compounds of the general formula I or pharmaceutically acceptable salts thereof as therapeutic active ingredients, and to pharmaceutical compositions comprising at least one compound of the general formula I or pharmaceutically acceptable salts thereof, where appropriate together with pharmaceutically suitable excipients and/or carriers.

Pharmaceutically acceptable salts of the compounds of the general formula I can be prepared by methods known to the skilled person, depending on the nature of the compound of formula I, either by using as inorganic acids inter alia hydrochloric acid, hydrobromic acid, sulphuric acid and phosphoric acid, nitric acid, as carboxylic acids inter alia acetic acid, propionic acid, hexanoic acid, octanoic acid, decanoic acid, oleic acid, stearic acid, maleic acid, fumaric acid, succinic acid, benzoic acid, ascorbic acid, oxalic acid, salicylic acid, tartaric acid, citric acid, lactic acid, glycolic acid, malic acid, mandelic acid, cinnamic acid, glutamic acid, aspartic acid, and as sulphonic acids inter alia methanesulphonic acid, ethanesulphonic acid, toluenesulphonic acid, benzenesulphonic acid and naphthalenesulphonic acid;
or by using an appropriate base as inorganic base inter alia alkalimetal hydroxide, carbonate, or hydrogencarbonate, as organic base inter alia tertiary amines and N-heterocycles.

These pharmaceutical compositions and medicaments may be intended for oral, rectal, subcutaneous, transdermal, percutaneous, intravenous or intramuscular administration. They comprise besides conventional carriers and/or diluents at least one compound of the general formula I.

The medicaments of the invention are formulated using the customary solid or liquid carriers or diluents and the excipients customarily used in pharmaceutical technology, in accordance with the desired mode of administration with a suitable dosage in a known manner: i.e. by processing the active ingredient with the carrier substances, fillers, substances which influence disintegration, binders, humectants, lubricants, absorbents, diluents, test modifiers, colorants etc. which are used in pharmaceutical technology, and converting into the desired administration form. Reference should be made in this connection to Remington's Pharmaceutical Science, 15th ed. Mack Publishing Company, East Pennsylvania (1980) and to Gennaro, A. R. et al., Remington: The Science and Practice of Pharmacy (20th Edition., Lippincott Williams & Wilkins 2000, see especially Part 5: Pharmaceutical Manufactoring).

Pharmaceutical compositions according to the present invention are preferably administered orally. Suitable for oral administration are in particular tablets, (film)-coated tablets, sugar-coated tablets capsules, pills, powders, granules, pastilles, suspensions, emulsions, solutions or depot forms.

Appropriate tablets can be obtained for example by mixing the active ingredient with known excipients, for example inert diluents such as dextrose, sugar, sorbitol, mannitol, polyvinylpyrrolidone, disintegrants such as maize starch or alginic acid, binders such as starch or gelatine, lubricants such as magnesium stearate or talc and/ or agents to achieve a depot effect such as carboxylpolymethylene, carboxylmethylcellulose, cellulose acetate phthalate or polyvinyl acetate. The tablets may also consist of a plurality of layers.

Correspondingly, coated tablets can be produced by coating cores which have been produced in analogy to the tablets with agents normally used in tablet coatings, for example polyvinylpyrrolidone or shellac, gum Arabic, talc, titanium oxide or sugar. The tablet coating may also consist of a plurality of layers, it being possible to use the excipients mentioned above for tablets.

Solutions or suspensions with the compounds according to the invention of the general formula I may additionally comprise taste-improving agents such as saccharin, cyclamate or sugar and, for example, flavourings such as vanillin or orange extract. They may additionally comprise suspending aids such as sodium carboxymethylcellulose or preservatives such as p-hydroxybenzoates.

Capsules comprising the compounds of the general formula I can be produced for example by the compound(s) of the general formula I being mixed with an inert carrier such as lactose or sorbitol and encapsulated in gelatine capsules.

Parenteral preparations such as solutions for injection are also suitable. Preparations for injection and infusion are possible for parenteral administration. Appropriately prepared crystal suspensions can be used for intraarticular injection. Aqueous and oily solutions for injection or suspensions and corresponding depot preparations can be used for intramuscular injection. The novel compounds can be used for rectal administration in the form of suppositories, capsules, solutions (e.g. in the form of enemas) and ointments both for systemic and for local therapy.

Suitable suppositories can be produced for example by mixing with carriers intended for this purpose, such as neutral fats or polyethylene glycol or derivatives thereof.

Formulations suitable for topical application include gels, ointments, greasy ointments, creams, pastes, dusting powders, milk and tinctures. Topical use can also take place by means of a transdermal system, for example a patch. The concentration of the compounds of the general formula I in these preparations should typically be in the range of 0.01% - 20% in order to achieve an adequate pharmacological effect.

The invention further relates to pharmaceutical compositions in combination with packaging material suitable for said composition, wherein said packaging material including instructions for the use of the composition.

### Dose

Suitable doses for the compounds according to the present invention may vary from 0.005 mg to 50 mg per day per kg of body weight, depending on the age and constitution of the patient. It is possible to administer the necessary daily dose by single or multiple delivery. The preferred daily dose for larger mammals, for example humans, may vary in the range from 10 µg to 30 mg per kg of body weight.

The exact dose and regimen of administration of the drug substance (active ingredient, or a pharmaceutical composition thereof, may however vary with the particular compound, the route of administration, and the age, sex and condition of the individual to whom the medicament is administered. The dose, the dosage as well as the regimen of the administration may thus differ between a male and a female considerably.

The compounds according to the invention of the general formula I can be prepared as described below.

### Abbreviations used:

| | |
|---|---|
| ACN | Acetonitrile |
| DIBAC | Diisobutylaluminium hydride |
| DMF | *N*,*N*-Dimethylformamide |
| EDC | *N*-Ethyl-*N*'-(3-dimethylaminopropyl)carbodiimide |
| EtOH | Ethanol |
| HATU | O-(7-Azabenzotriazol-1-yl)-N,N,N',N'-tetramethyluronium hexafluorophosphate |
| FMOC | (9*H*-Fluoren-9-ylmethoxy)carbonyl |
| HOBt | 1-Hydroxy-1*H*-benzotriazole |
| MeCN | Acetonitrile |
| MeOH | Methanol |
| MTBE | Methyl tert-butyl ether |
| NMM | 4-methylmorpholine |
| NMP | N-Methylpyrrolidinone |
| Rf | Reflux |
| RT | Room temperature |
| TBAF | Tetrabutylammonium fluoride |
| TFA | Trifluoroacetic acid |
| THF | Tetrahydrofuran |

Compounds of the general formula I can in principle be prepared as shown in Scheme 9 by an sulfonamide forming reaction between a tryptophanol derivative V and a sulfonyl chloride VI. Typically the reaction is performed in the presence of a base.

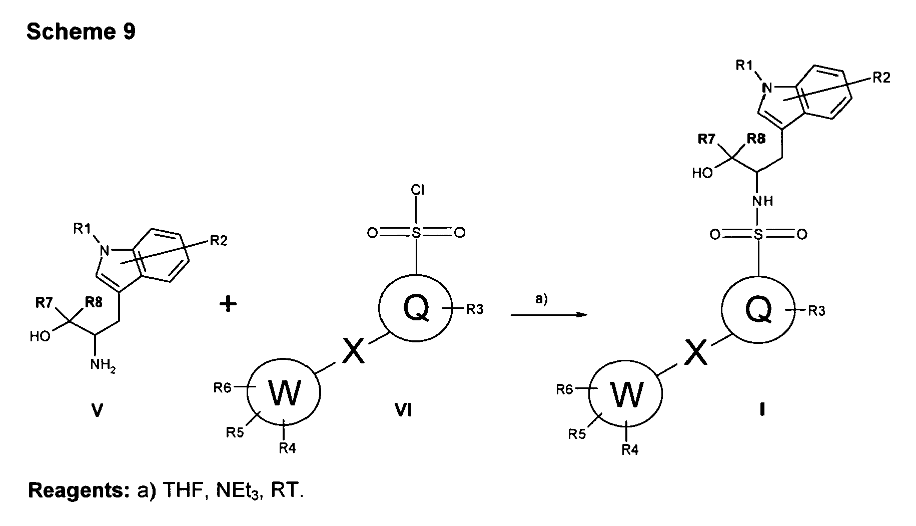

The tryptophanol derivatives of the formula V with R7 = R8 = H can be prepared as shown in Scheme 10 from the corresponding amino acids which can be purchased or are known from the literature.

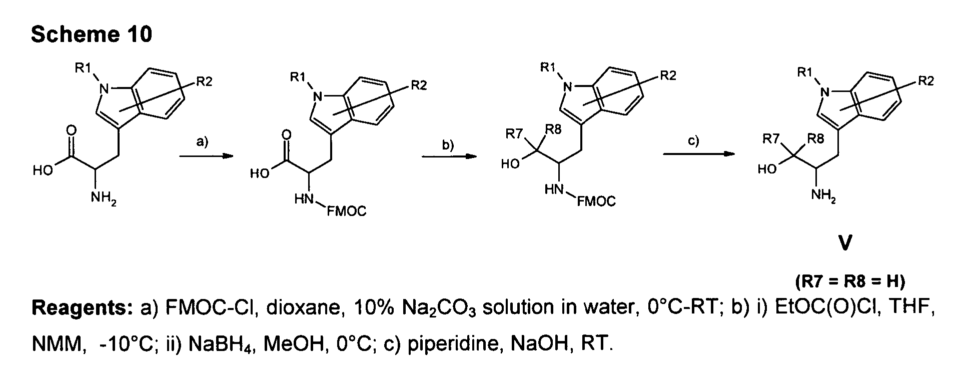

Compounds of the general formula I can in principle also be prepared as shown in Scheme 11 via an Grignard reaction from the corresponding esters XV.

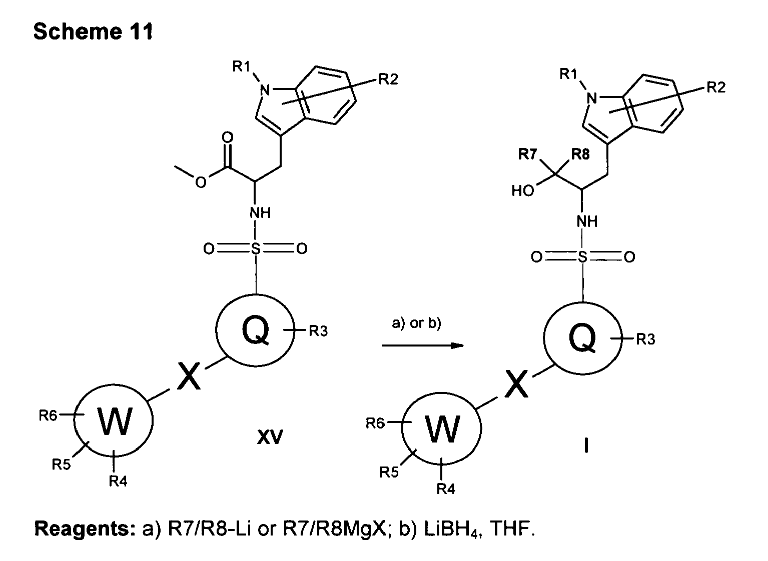

Compounds of the general formula XV can in principle be prepared as shown in Scheme 12 by an sulfonamide forming reaction between a tryptophan derivative XVI and a sulfonyl chloride VI. Typically the reaction is performed in the presence of a base.

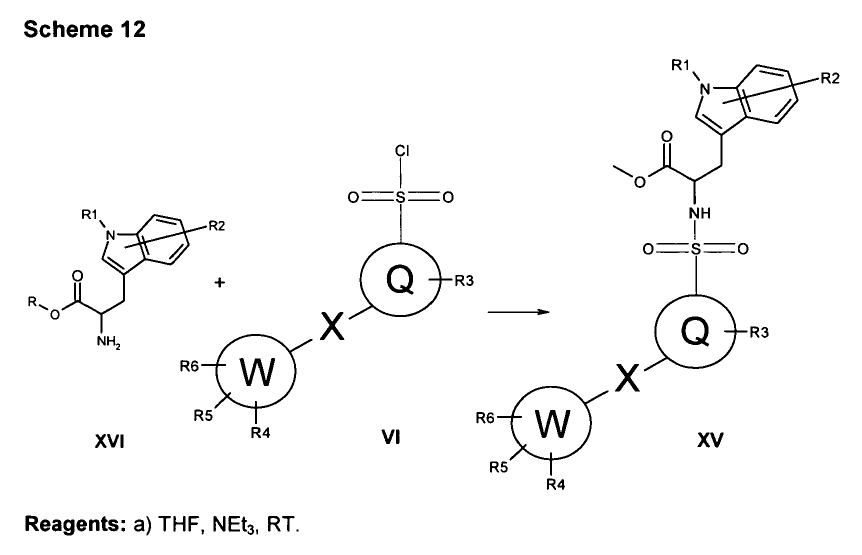

Compounds of the general formula I can in principle also be prepared as shown in Scheme 13 via Suzuki reaction of aryl halide XI and boronic acid XVII.

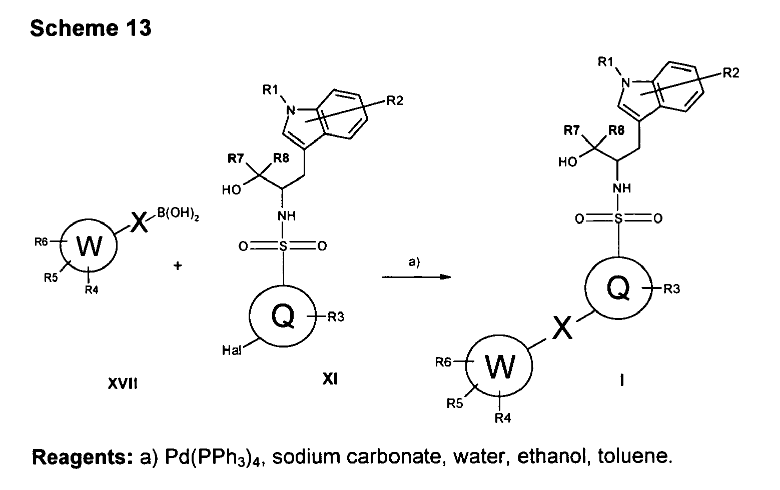

The acetylene derivatives of formula XVIII can in principle also be prepared according to Scheme 14 via a Sonogashira type coupling of terminal acetylene XIX or XXI with the corresponding aryl halides XX or XI.

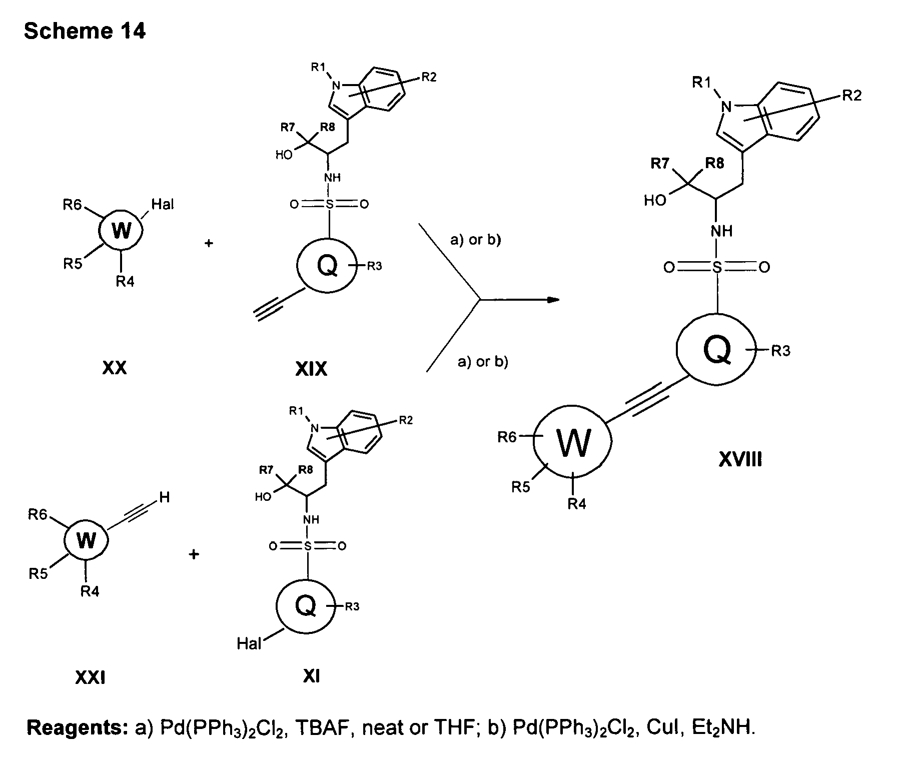

The acetylene derivatives of formula XIX can in principle also be prepared according to Scheme 15 via a Sonogashira type coupling from aryl halide XI. The aryl halide XI itself can be prepared via an sulfonamide forming reaction from the tryptophanol derivative V and sulfonyl chloride XXII.

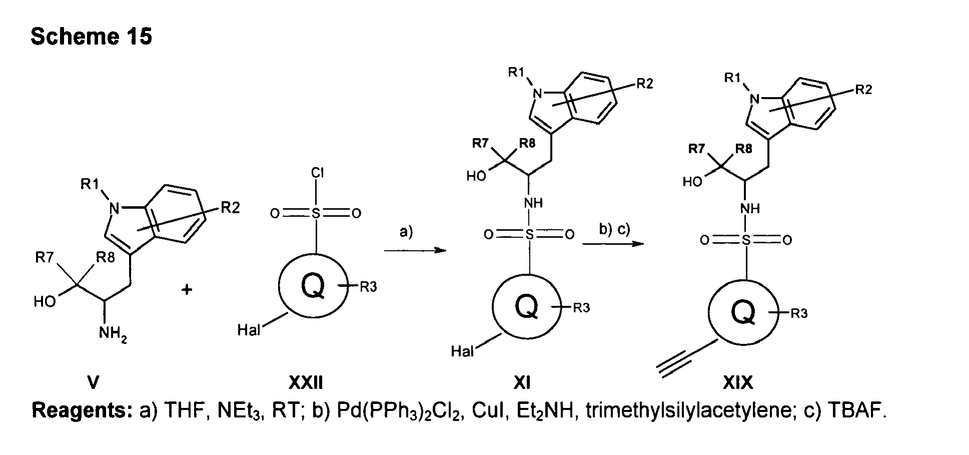

### Synthesis of the compounds according to the invention

### Example 1

### 4,3',4',5'-Tetramethoxy-biphenyl-3-sulfonic acid [(R)-2-hydroxy-1-(1H-indol-3-ylmethyl)-ethyl]-amide

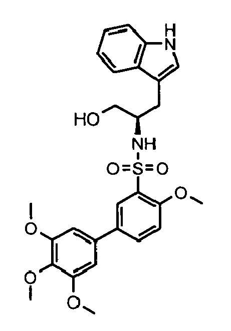

### 1a) 5-Bromo-N-[(R)-2-hydroxy-1-(1H-indol-3-ylmethyl)-ethyl]-2-methoxy-benzenesulfonamide

To a solution of (R)-Tryptophanol (67 mg) and triethylamine (0.12 ml) in THF (2.8 ml) was added slowly a solution of 5-Bromo-2-methoxy-benzenesulfonyl chloride (100 mg) in THF (2 ml) and the mixture was stirred for 1 h at room temperature. Water (2 ml) and brine (8 ml) were added, the aqueous phase extracted with ethyl acetate (2 x 20 ml) and the combines organic phases reduced in vacuo. Flash chromatography of the residue on silica gel afforded 50 mg of the title compound. **¹H-NMR (DMSO-d₆):** 10.69 s (1H); 7.69 d (*J* = 2.5 Hz, 1H); 7.58 dd (*J* = 2.5 Hz / 8.8 Hz, 1H); 7.24 d (*J* = 8.1 Hz, 1H); 7.20 d (*J* = 7.8 Hz, 1H); 7.17 m (1H); 7.02 - 6.98 m (2H); 6.96 d (*J* = 8.8 Hz, 1H); 6.89 m (1H); 4.68 d (*J* = 5.3 Hz, 1H); 3.77 s (3H); 3.35 m (1H); 3.27 m (2H); 2.89 dd (*J* = 6.6 Hz / 14.4 Hz, 1H); 2.64 dd (*J* = 6.3 Hz / 14.4 Hz, 1H).

### 1b) 4,3',4',5'-Tetramethoxy-biphenyl-3-sulfonic acid [(R)-2-hydroxy-1-(1H-indol-3-ylmethyl)-ethyl]-amide

A solution of 5-Bromo-N-[(R)-2-hydroxy-1-(1H-indol-3-ylmethyl)-ethyl]-2-methoxy-benzenesulfonamide (200 mg), 3,4,5-Trimethoxy-benzene boronic acid (97 mg) and Pd(PPh₃)₄ (26 mg) in ethanol (2 mL), toluene (10 mL) and an aqueous sodium carbonate solution (2M, 2 mL) was stirred at reflux for 4 h. The solvent was evaporated and the solid purified by flash chromatography to yield 61 mg of the title compound.
**¹H-NMR (DMSO-d₆):** 10.69 s (1H); 7.89 s (1H); 7.76 dd (*J* = 2.3 Hz / 8.6 Hz, 1H); 7.21 d (*J* = 8.1 Hz); 7.12 m (2H); 7.00 s (1H); 6.99 m (1H); 6.92 m (1H); 6.77 s (2H); 6.72 m (1H); 4.64 m (1H); 3.82 s (6H); 3.66 s (3H); 3.28 m (2H); 2.85 m (1H); 2.65 m (1H).

The following compounds were obtained in analogy to the preparation methods decribed in detail:

| **Ex.** | **Product; *reagents*** | **Method gous to** | **¹H-NMR (400 MHz) δ analo-[ppm]** | **Structure** |
|---|---|---|---|---|
| **2** | 3-Chloro-2'-[(R)-2-hydroxy-1-(1H-indol-3-ylmethyl)-ethylsulfamoyl]-biphenyl-4-carboxylic acid methylamide; *2-Bromo-N-[(R)-2-hydroxy-1-(1H-indol-3-ylmethyl)-ethyl]-benzenesulfonamide* *and* *3-Chloro-4-methylcarbamoyl-phenyl boronic acid* | **1** | **¹H-NMR (DMSO-d₆):** 10.79 s (1H); 8.40 q (*J* = 4.7 Hz, 1H); 7.84 d (*J* = 7.0 Hz, 1H); 7.55 m (1H); 7.45 d (*J* = 6.4 Hz, 1H); 7.42 - 7.26 m (6H); 7.22 dd (*J* = 0.9 Hz / 6.9 Hz, 1H); 7.07, d (*J* = 2.3 Hz, 1H); 7.04 m (1H); 6.93 m (1H); 4.76 t (*J* = 5.1 Hz, 1H); 3.44-3.23 m (3H); 2.93 dd (*J* = 6.6 Hz / 14.5 Hz, 1H); 2.77 d (*J* = 4.5 Hz, 3H); 2.75 m (1H). | 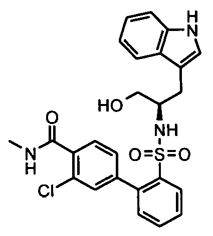 |
| **3** | 3-Chloro-3'-[(R)-2-hydroxy-1-(1H-indol-3-ylmethyl)-ethylsulfamoyl]-4'-methoxybiphenyl-4-carboxylic acid me-thylamide; *5-Bromo-N-[(R)-2-hydroxy-1-(1H-indol-3-ylmethyl)-ethyl]-2-methoxy-benzenesulfonamide* *and* *3-Chloro-4-methylcarbamoylphenyl boronic acid* | **1** | **¹H-NMR (DMSO-d₆):** 10.86 s (1H); 8.38 q (*J* = 4.9 Hz, 1H); 7.91 d (*J* = 2.5 Hz, 1H); 7.81 dd (*J* = 2.3 Hz / 8.5 Hz, 1H); 7.67 d (*J* = 1.5 Hz, 1H); 7.59 dd (*J* = 1.7 Hz / 8.1 Hz, 1H); 7.50 d (*J* = 7.9 Hz, 1H); 7.20 m (2H); 7.12 d (*J* = 8.9 Hz, 1H); 7.03 broad s (1H); 7.01 d (*J* = 1.9 Hz, 1H); 6.93 m (1H); 6.74 m (1H); 4.68 broad s (1H); 3.85 s (3H); 3.39 m (2H); 3.26 m (1H); 2.89 dd (*J* = 6.2 Hz / 14.3 Hz, 1H); 2.78 d (*J* = 4.5 Hz, 1H); 2.68 dd (*J* = 6.2 Hz / 14.3 Hz, 1H). | 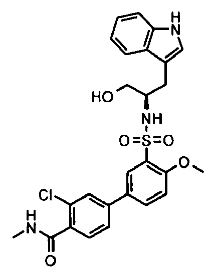 |
| **4** | 3-Chloro-4'-[(R)-2-hydroxy-1-(1H-indol-3-ylmethyl)-ethylsulfamoyl]-biphenyl-4-carboxylic acid methylamide; *4-Bromo-N-[(R)-2-hydroxy-1-(1H-indol-3-ylmethyl)-ethyl]-benzenesulfonamide* *and* *3-Chloro-4-methylcarbamoyl-phenyl boronic acid* | **1** | **¹H-NMR (DMSO-d₆):** 10.67 s (1H); 8.42 q (*J* = 4.7 Hz, 1H); 7.79 d (*J* = 1.7 Hz, 1H); 7.68 m (2H); 7.63 s (4H); 7.54 d (*J* = 7.9 Hz, 1H); 7.26 d (*J* = 7.5 Hz, 1H); 7.15 d (*J* = 7.9 Hz, 1H); 7.05 d (*J* = 2.1 Hz, 1H); 6.93 m (1H); 6.85 m (1H); 4.77 t (*J* = 5.5 Hz, 1H); 3.4 m (1H); 3.28 m (1H); 2.95 dd (*J* = 5.8 Hz / 13.9 Hz, 1H); 2.78 d (*J* = 4.5 Hz, 3H); 2.61 dd(*J*=6.6Hz/14.1 Hz, 1H). | 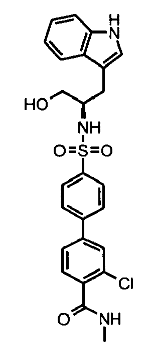 |
| **5** | 3'-Chloro-4'-(morpholine-4-carbonyl)-5-trifluoromethyl-biphenyl-3-sulfonic acid [(R)-2-hydroxy-1-(1H-indol-3-yl-methyl)-ethyl]-amide; *3-Bromo-N-[(R)-2-hydroxy-1-(1H-indol-3-ylmethyl)-ethyl]-5-trifluoromethyl-benzene-sulfonamide and 3-Chloro-4-(morpholine-4-carbonyl)-phenyl boronic acid* | **1** | **¹H-NMR (DMSO-d₆):** 10.59 s (1H); 8.16 d (*J* = 8.5 Hz, 1H); 7.96 d (*J* = 7.0 Hz, 1H); 7.92 s (1H); 7.90 d (*J* = 1.5 Hz, 1H); 7.75 dd (*J* = 1.3 Hz / 7.7 Hz, 1H); 7.53 d (*J* = 8.1 Hz, 1H); 7.27 d (*J* = 7.5 Hz, 1H); 7.15 d (*J* = 8.1 Hz, 1H); 6.97 d (*J* = 1.9 Hz, 1H); 6.91 m (1H); 6.78 m (1H); 4.80 t (*J* = 5.3 Hz, 1H); 3.7 m (4H); 3.57 m (2H); 3.40 m (2H); 3.31 m (1H); 3.19 m (2H); 2.90 dd (*J* = 8.7 Hz/14.5 Hz, 1H); 2.62 dd (*J* = 6.4 Hz / 14.5 Hz, 1H). | 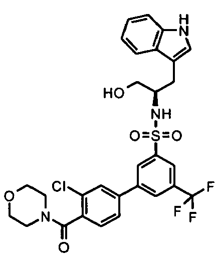 |
| **6** | 3-Chloro-3'-[(R)-2-hydroxy-1-(1H-indol-3-ylmethyl)-ethylsulfamoyl]-5'-trifluoromethyl-biphenyl-4-carboxylic acid me-thylamide; *3-Bromo-N-[(R)-2-hydroxy-1-(1H-indol-3-ylmethyl)-ethyl]-5-trifluoromethyl-benzene-sulfonamide* *and* *3-Chloro-4-methylcarbamoyl-phenyl boronic acid* | **1** | **¹H-NMR (DMSO-d₆):** 10.58 s (1H); 8.43 q (*J* = 4.7 Hz, 1H); 8.18 s (1H); 8.13 s (1H);7.97 d (*J* = 6.6 Hz, 1H); 7.92 s (1H); 7.87 d (*J* = 1.5 Hz, 1H); 7.72 dd (*J* = 1.5 Hz / 8.1 Hz, 1H); 7.56 d (*J* = 8.1 Hz, 1H); 7.26 d (*J* = 7.7 Hz, 1H); 7.16 d (*J* = 7.9 Hz, 1H); 6.99 d (*J* = 1.9 Hz, 1H); 6.92 m (1H); 6.79 m (1H); 4.79 t (*J* = 5.5 Hz, 1H); 3.48 - 3.26 m (3H); 2.91 dd (*J* = 7.5 Hz / 14.3 Hz, 1H); 2.79 d (*J* = 4.7 Hz, 3H); 2.63 dd (*J* = 6.2 Hz/14.3 Hz, 1H). | 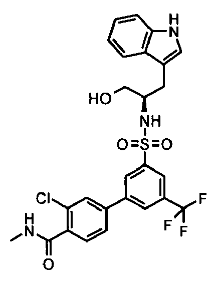 |
| **7** | 3-Chloro-3'-[(R)-2-hydroxy-1-(1H-indol-3-ylmethyl)-ethylsulfamoyl]-5'-trifluoro-methyl-biphenyl-4-carboxylic acid amide; *3-Bromo-N-[(R)-2-hydroxy-1-(1H-indol-3-ylmethyl)-ethyl]-5-trifluoromethyl-benzene-sulfonamide* *and* *3-Chloro-4-carbamoyl-phenyl boronic acid* | **1** | **¹H-NMR (DMSO-d₆):** 10.59 s (1H); 8.18 s (1H); 8.13 s (1H); 7.96 broad s (2H); 7.92 s (1H); 7.92 s (1H); 7.86 d (*J* = 1.7 Hz, 1H); 7.72 dd (*J* = 1.7 Hz / 8.1 Hz, 1H); 7.68 s (1H); 7.58 d (*J* = 8.1 Hz, 1H); 7.26 d (*J* = 7.7 Hz, 1H); 7.16 d (*J* = 8.1 Hz, 1H); 6.99 d (*J* = 2.1 Hz, 1H); 6.93 m (1H); 6.79 m (1H); 4.79 m (1H); 3.46 - 3.26 m (3H); 2.91 dd (*J* = 6.6 Hz / 14.5 Hz, 1H); 2.63 dd (*J* = 6.8 Hz / 14.5 Hz, 1H). | 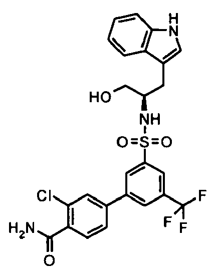 |
| **8** | 4'-Bromo-3-chloro-3'-[(R)-2-hydroxy-1-(1H-indol-3-yl-methyl)-ethylsulfamoyl]-biphenyl-4-carboxylic acid amide; *2,5-Dibromo-N-[(R)-2-hydroxy-1-(1H-indol-3-ylmethyl)-ethyl]-benzenesulfonamide* *and* *3-Chloro-4-carbamoyl-phenyl boronic acid* | **1** | **¹H-NMR (DMSO-d₆):** 10.65 s (1H); 8.10 d (*J* = 2.3 Hz, 1H); 8.13 s (1H); 7.93 s (1H); 7.87 - 7.61 m (5H); 7.59 dd (*J* = 1.7 Hz / 8.1 Hz, 1H); 7.54 d (*J* = 7.9 Hz, 1H); 7.23 d (*J* = 7.9 Hz, 1H); 7.18 d (*J* = 8.1 Hz, 1H); 7.04 d (*J* = 2.1 Hz, 1H); 6.90 m (1H); 6.75 m (1H); 4.81 t (*J* = 5.3 Hz, 1H); 3.53 - 3.34 m (3H); 2.94 dd (*J* = 6.4 Hz / 14.7 Hz, 1H); 2.71 dd (*J* = 6.2 Hz / 14.3 Hz, 1H). | 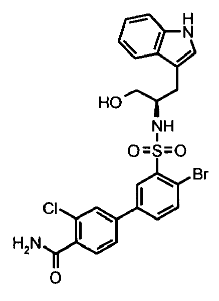 |

### Example 9

### 3-Chloro-3'-[(R)-2-hydroxy-1-(1H-indol-3-ylmethyl)-ethylsulfamoyl]-4'-isopropoxybiphenyl-4-carboxylic acid methylamide

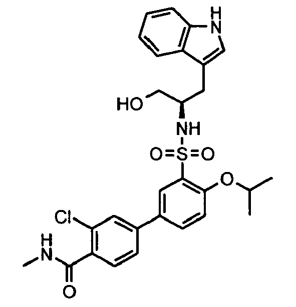

### 9a) 5-Bromo-2-hydroxy-benzenesulfonyl chloride

Chloro sulphonic acid (13.5 ml) was cooled to 0°C and 4-Bromo-phenol (5 g) slowly added in small portions.The clear colourless solution was allowed to warm to room temperature and stirred for 16 h. The mixture was poured over ice and a fine precipitate formed. Filtration afforded 2.02 g the title compound as a white pasty solid that was not further purified. Extraction of the aqueous filtrate with ethyl acetate, drying of the combined organic phases with sodium sulfate and removal of solvent afforded another 2.32 g of the title compound. **¹H-NMR (DMSO-d₆):** 7.46 d (*J* = 2.5 Hz, 1H); 7.32 dd (*J* = 2.5 Hz /8.8 Hz, 1H); 6.73(*J* = 8.6 Hz, 1H); no sharp signal corresponding to the phenolic hydroxy-proton detected.

### 9b) (R)-2-(5-Bromo-2-hydroxy-benzenesulfonylamino)-3-(1H-indol-3-yl)-propionic acid methyl ester

To a solution of (R)-Tryptophane methyl ester hydrochloride (493 mg) and triethylamine (0.77 ml) in DMF (5 ml) was added crude 5-Bromo-2-hydroxy-benzenesulfonyl chloride (500 mg) and the solution stirred for 16 h. Upon addition of water and hydrochloric acid (2 M) a white precipitate formed. The mixture was extracted with ethyl acetate, the combined organic phases washed with saturated aqueous sodium bicarbonate solution, dried over sodium sulfate and reduced in vacuo. Flash chromatography on silica gel afforded 138 mg of the title compound.
**¹H-NMR (CDCl₃):** 8.65 d (*J* = 4.1Hz, 1H); 8.21 s (1H); 7.64 d (*J* = 2.5 Hz, 1H); 7.48 d (*J* = 7.7 Hz, 1H); 7.38 dd (*J* = 2.5 Hz / 8.9 Hz, 1H); 7.33 m (1H); 7.22 m (1H); 7.13 m (1H); 7.07 d (*J* = 2.5 Hz, 1H); 6.81 d (*J* = 8.7 Hz, 1H); 4.34 m (1H); 3.61 s (3H); 3.32 dd (*J* = 4.9 Hz / 14.7 Hz, 1H); 3.26 dd (*J* = 6.1 Hz / 14.7 Hz, 1H).

### 9c) (R)-2-(5-Bromo-2-isopropoxy-benzenesulfonylamino)-3-(1H-indol-3-yl)-propionic acid methyl ester

To a solution of (R)-2-(5-Bromo-2-hydroxy-benzenesulfonylamino)-3-(1H-indol-3-yl)-propionic acid methyl ester (138 mg) in DMF (6 ml) were added potassium carbonate (105 mg) and 2-Iodo-propane (0.061 ml) and the mixture was heated to 60°C for 2 h. Water and sulphuric acid (1 M) were added and the mixture extracted with ethyl acetate. The combined organic phases were dried over sodium sulfate, the solvent removed in vacuo and the residue chromatographed on silica gel to afford 75 mg of the title compound. **¹H-NMR (CDCl₃):** 8.11 s (1H); 7.97 d (*J* = 2.5 Hz, 1H); 7.57-7.52 m (2H); 7.37 d (*J* = 8.1 Hz, 1H); 7.21 m (1H); 7.13 m (1H); 7.12 d (*J* = 2.5 Hz, 1H); 6.79 d (*J* = 8.9 Hz, 1H); 5.67 d (*J* = 8.1 Hz, 1H); 4.57 sept. (*J* = 6.0 Hz, 1H); 4.41 m (1H); 3.47 s (3H); 3.36 m (2H); 1.34 d (*J* = 6.0 Hz, 3H); 1.26 d (*J* = 6.0 Hz, 3H).

### 9d) 5-Bromo-N-[(R)-2-hydroxy-1-(1H-indol-3-ylmethyl)-ethyl]-2-isopropoxy benzenesulfonamide

A solution of (R)-2-(5-Bromo-2-isopropoxy-benzenesulfonylamino)-3-(1H-indol-3-yl)-propionic acid methyl ester (171 mg) was dissolved in THF (4 mL) and a solution of lithium borohydride (2 M in THF, 518 µL) was added at 0°C followed by methanol (77 µL). The reaction was allowed to warm to room temperature and stirred overnight, then quenched with methanol and water. The solvent was distilled off under reduced pressure and the title compound was obtained after flash chromatography in 82 % yield (161 mg). **¹H-NMR (DMSO-d₆):** 10,72 s (1H); 7.76 d (*J* = 2.5 Hz, 1H); 7.60 dd (*J* = 2.8 Hz / 9.1 Hz, 1H); 7.24 d (*J* = 8.1 Hz, 1H); 7.19 d (*J* = 7.8 Hz, 1H); 7.06 d (*J* = 9.1 Hz, 1H); 7.01 d (*J* = 2.0 Hz, 1H); 6.98 m (1H); 6.88 m (1H); 6.59 d (*J* = 7.1 Hz, 1H); 4.72 - 4.64 m (2H); 3.32 m (1H); 3.25 m (2H); 2.85 dd (*J* = 8.1 Hz / 14.2 Hz, 1H); 2.67 dd (*J* = 5.3 Hz / 14.2 Hz, 1H); 1.26 d (*J* = 5.8 Hz, 3H); 1.23 (*J* = 5.8 Hz, 3H).

### 9e) 3-Chloro-3'-[(R)-2-hydroxy-1-(1H-indol-3-ylmethyl)-ethylsulfamoyl]-4'-isopropoxybiphenyl-4-carboxylic acid methylamide

To a solution of 5-Bromo-N-[(R)-2-hydroxy-1-(1H-indol-3-ylmethyl)-ethyl]-2-isopropoxy benzenesulfonamide (64 mg) and 3-Chloro-4-methylcarbamoyl-benzeneboronic acid (32 mg) in toluene (1.8 ml) and EtOH (1.8 ml) were added aqueous Na₂CO₃ (1 M, 340 µL) and Pd(PPh₃)₄ (16 mg) and the mixture was stirred at 100°C for 4 h. After addition of water and brine the mixture was extracted with ethyl acetate, the combined organic phases washed with brine, dried over Na₂SO₄ and reduced in vacuo. Flash chromatography on silica gel afforded 56 mg of the title compound (74 %). **¹H-NMR (DMSO-d₆):** 10,71 s (1H); 8.36 q (*J* = 4.6 Hz, 1H); 7.98 d (*J* = 2.3 Hz, 1H); 7.83 dd (*J* = 2.5 Hz / 8.6 Hz, 1H); 7.68 d (*J* = 1.5 Hz, 1H); 7.59 dd (*J* = 1.8 Hz / 8.1 Hz, 1H); 7.47 d (*J* = 7.8 Hz, 1H); 7.21 m (2H); 7.18 d (*J* = 7.8 Hz, 1H); 7.00 d (*J* = 2.0 Hz, 1H); 6.93 m (1H); 6.75 m (1H); 4.78 m (1H); 4.72 t (*J* = 5.5 Hz, 1H); 3.33 m (1H); 3.25 m (2H); 2.85 dd (*J* = 8.3 Hz / 14.4 Hz, 1H); 2.75 d (*J* = 4.6 Hz, 3H) 2.70 dd (*J* = 5.8 Hz / 14.4 Hz, 1H); 1.31 d (*J* = 6.1 Hz, 3H); 1.28 (*J* = 5.8 Hz, 3H).

The following compounds were obtained in analogy to the preparation methods decribed in detail:

| **Ex.** | **Product; *reagents*** | **Method analogous to** | **¹H-NMR (400 MHz) δ [ppm]** | **Structure** |
|---|---|---|---|---|
| **10** | 4-lsopropoxy-3',4',5'-trimethoxy-biphenyl-3-sulfonic acid [(R)-2-hydroxy-1-(1H-indol-3-ylmethyl)-ethyl]-amide; *5-Bromo-N-[(R)-2-hydroxy-1-(1H-indol-3-ylmethyl)-ethyl]-2-isopropoxy-benzenesulfonamide* *and* *3,4,5-Trimethoxyphenylboronic acid* | **9** | **¹H-NMR (DMSO-d₆):** 10,77 s (1H); 8.01 d (*J* = 2.5 Hz, 1H); 7.83 dd (*J* = 2.5 Hz / 8.8 Hz, 1H); 7.26 d (*J* = 7.1 Hz, 1H); 7.23 d (*J* = 6.1 Hz, 1H); 7.06 d (*J* = 2.3 Hz, 1H); 7.03 d (*J* = 8.6 Hz, 1H); 6.98 m (1H); 6.83 s (2H); 6.79 m (1H); 6.46 d (*J* = 7.1 Hz, 1H); 4.82 m (1H); 4.75 t (*J* = 5.5 Hz, 1H); 3.85 s (6H); 3.69 s (3H); 3.38 m (1H); 3.31 - 3.21 m (2H); 2.88 dd (*J* = 8.8 Hz / 14.4 Hz, 1H); 2.76 dd (*J* = 5.3 Hz / 14.4 Hz, 1H); 1.34 d (*J* = 6.1 Hz, 3H); 1.31 (*J* = 5.8 Hz, 3H). | 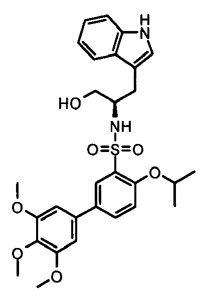 |

### Example 11

### 5-Pyridin-2-yl-thiophene-2-sulfonic acid [(R)-2-hydroxy-1-(1H-indol-3-ylmethyl)-ethyl]-amide

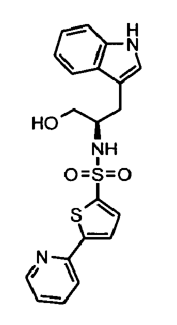

To a solution of (R)-Tryptophanol (75 mg) and triethylamine (0.14 ml) in THF (3.1 ml) was added slowly a solution of 5-Pyridin-2-yl-thiophene-2-sulfonyl chloride (100 mg) in THF (2 ml) and the mixture stirred for 1 h at room temperature. Water (2 ml) and brine (8 ml) were added, the aqueous phase extracted with ethyl acetate (2 x 20 ml) and the combines organic phases reduced in vacuo. Flash chromatography of the residue on silica gel afforded 87 mg of the title compound. **¹H-NMR (DMSO-d₆):** 10.72 s (1H); 8.56 d (*J* = 4.6 Hz, 1H); 7.95 d (*J* = 7.8 Hz, 1H); 7.92 - 7.87 m (2H); 7.59 d (*J* = 4.0 Hz, 1H); 7.39 - 7.35 m (3H); 7.17 d (*J* = 8.1 Hz, 1H); 7.07 d (*J* = 2.0 Hz, 1H); 6.93 m (1H); 6.84 m (1H); 4.75 t (*J* = 5.6 Hz, 1H); 3.47 - 3.24 m (3H); 2.95 dd (*J* = 7.1 Hz / 14.2 Hz, 1H); 2.68 dd (*J* = 6.3 Hz / 14.4 Hz, 1H).

The following compounds were obtained in analogy to the preparation methods decribed in detail:

| **Ex.** | **Product; *reagents*** | **Method analogous to** | **¹H-NMR (400 MHz)** δ **[ppm]** | **Structure** |
|---|---|---|---|---|
| **12** | 4'-Trifluoromethyl-biphenyl-3-sulfonic acid [(R)-2-hydroxy-1-(1H-indol-3-ylmethyl)-ethyl]-amide; *4 '-Trifluoromethyl-biphenyl-3 sulfonyl chloride* *and* *(D)-Tryptophanol* | **11** | **¹H-NMR (DMSO-d₆):** 10.67 s (1H); 8.04 t (*J* = 1.5 Hz, 1H); 7.92 - 7.83 m (5H); 7.72 m (2H); 7.54 t (*J* = 7.7 Hz, 1H); 7.24 m (1H); 7.01 d (*J* = 2.1 Hz, 1H); 6.96 m (1H); 6.81 m (1H); 4.74 t (*J* = 5.1 Hz, 1H); 3.39 - 3.21 m (3H); 2.91 dd (*J* = 9.2 Hz / 14.3 Hz, 1H); 2.62 dd (*J* = 5.8 Hz / 14.3 Hz, 1H). | 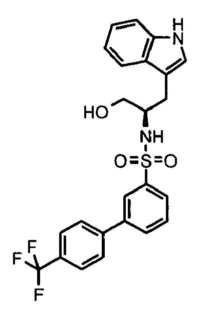 |
| **13** | 4'-Methyl-biphenyl-3-sulfonic acid [(R)-2-hydroxy-1-(1H-indol-3-ylmethyl)-ethyl]-amide; *4'-Methyl-biphenyl-3-sulfonyl chloride* *and* *(D)-Tryptophanol* | **11** | **¹H-NMR (DMSO-d₆):** 10.71 s (1H); 8.04 t (*J* = 1.3 Hz, 1H); 7.8 d (*J* = 7.9 Hz, 1H); 7.67 m (2H); 7.60-7.47 m (3H); 7.31 d (*J* = 8.1 Hz, 1H); 7.25 d (*J* =8.1 Hz, 1H); 7.02 d (*J* = 2.1 Hz, 1H); 6.98 m (1H); 6.83 m (1H); 4.70 t (*J* = 5.3 Hz, 1H); 3.37 - 3.20 m (3H); 2.89 dd (*J* = 7.2 Hz / 14.0 Hz, 1H); 2.64 dd (*J* = 5.3 Hz / 14.0 Hz, 1H); 2.36 s (3H). | 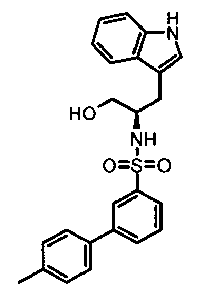 |
| **14** | 5-(2-Methyl-5-trifluoromethyl-2H-pyrazol-3-yl)-thiophene-2-sulfonic acid [(R)-2-hydroxy-1-(1H-indol-3-ylmethyl)-ethyl]-amide; *5-(2-Methyl-5-trifluoromethyl-2H-pyrazol-3-yl)-thiophene-2-sulfonyl chloride* *and* *(D)-Tryptophanol* | **11** | **¹H-NMR (DMSO-d₆):** 10.69 s (1H); 8.04 broad s (1H); 7.39 m (1H); 7.30 d (J = 4.0 Hz, 1H); 7.23 d (*J* = 4.0 Hz, 1H); 7.13 m (1H);7.06d(J=1.7Hz, 1H); 7.03 s (1H); 6.96 - 6.85 m (2H); 4.85 t (*J* = 5.5 Hz, 1H);3.96s(3H); 3.44 m (2H); 3.37-3.28 m (1H, overlapping with water signal); 3.00 dd (J = 5.5 Hz /14.3 Hz, 1H); 2.66 dd (*J* = 7.5 Hz /14.3 Hz, 1H). | 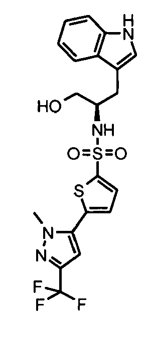 |

### Example 15

### 3-Chloro-3'-[(R)-2-hydroxy-1-(1H-indol-3-ylmethyl)-2-methyl-propylsulfamoyl]-4'-methoxy-biphenyl-4-carboxylic acid amide

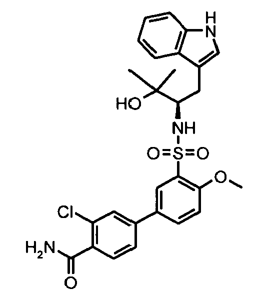

### 15a) (R)-2-(5-Bromo-2-methoxy-benzenesulfonylamino)-3-(1H-indol-3-yl)-propionic acid methyl ester

A solution of 5-Bromo-2-methoxy-benzenesulfonyl chloride (1.0 g) in dichloromethane (10 mL) was added to a solution of (R)-2-Amino-3-(1H-indol-3-yl)-propionic acid methyl ester hydrochloride (1.07 g) in dichloromethane (10 mL) and triethyl amine (1.46 mL) at ambient temperature. The reaction was stirred at ambient temperature over night and quenched by the addition of hydrochloric acid (1N). The phases were separated and the organic phase was washed with saturated aqueous sodium carbonate solution, and dried over sodium sulphate. The solvent was distilled off under reduced pressure. The title compound was obtained in 95% yield (1.55 g) as a colourless foam.
**¹H-NMR (DMSO-d₆):** 10.82 s (1H); 8.19 d (*J* = 8.3 Hz, 1H); 7.65 m (2H); 7.28 d (*J* = 8.1 Hz, 1H); 7.25 d (*J* = 8.3 Hz, 1H); 7.08 d (*J* = 2.0 Hz, 1H); 7.03 m (2H); 6.94 m (1H); 4.08 m (1H); 3.76 s (3H); 3.39 s (3H); 3.09 dd (*J* = 14.6, 6.8 Hz, 1H); 2.94 dd (*J* = 14.4, 7.8 Hz, 1H).

### 15b) 5-Bromo-N-[(R)-2-hydroxy-1-(1H-indol-3-ylmethyl)-2-methyl-propyl]-2-methoxy-benzenesulfonamide

A solution of methyl lithium (6.4 mL, 1.6 M in diethyl ether) was added dropwise to a solution of (R)-2-(5-Bromo-2-methoxy-benzenesulfonylamino)-3-(1H-indol-3-yl)-propionic acid methyl ester (1.89 g) in tetrahydrofurane (20 mL) at 0 °C. The mixture was brought to room temperature an stirred for six hours. Additional methyl lithium (2.5 mL, 1.6 M in diethyl ether) was added to the mixture. The reaction was quenched after 30 minutes by the addition of water. The mixture was extracted with ethyl acetate. The organic phase was dried over magnesium sulphate, filtered and concentrated in vacuo. Purification by flash chromatography afforded the title compound in 29% yield (557 mg). **¹H-NMR (DMSO-d₆):** 10.33 s (1H); 7.32 m (2H); 7.13 d (*J* = 2.6 Hz, 1H); 7.10 d (*J* = 8.1 Hz, 1H); 6.95 m (2H); 6.87 m (1H); 6.81 d (*J* = 1.7 Hz, 1H); 6.70 d (*J* = 9.0 Hz, 1H); 4.41 s (1H); 3.75 s (3H); 3.51 m (1H); 3.06 dd (*J* = 14.1, 2.5 Hz, 1H); 2.60 dd (*J* = 14.7, 10.2 Hz, 1H); 1.17 s (3H); 1.16 s (3H).

### 15c) 3-Chloro-3'-[(R)-2-hydroxy-1-(1H-indol-3-ylmethyl)-2-methyl-propylsulfamoyl]-4'-methoxy-biphenyl-4-carboxylic acid amide

A solution of 5-Bromo-N-[(R)-2-hydroxy-1-(1H-indol-3-ylmethyl)-2-methyl-propyl]-2-methoxy-benzenesulfonamide (101 mg), 3-Chloro-4-carboxamide-phenyl boronic acid (42 mg), Pd(PPh₃)₄ (7.1 mg) and aqueous sodium carbonate solution (2M, 0.22 mL) in ethanol (1.3 mL) and toluene (2.0 mL) was stirred in a sealed microwave reactor at 100°C for 30 minutes. The solvent was evaporated and the solid purified by flash chromatography to yield 55 % of the title compound (64 mg).
**¹H-NMR (DMSO-d₆):** 10.25 d (*J* = 1.9 Hz, 1H); 7.88 s (1H); 7.61 s (1H); 7.56 dd (*J* = 8.5, 2.5 Hz, 1H), 7.48 d (*J* = 7.9 Hz, 1H), 7.35 m (4H); 6.97 m (1H); 6.90 d (*J* = 8.7 Hz, 2H); 6.76 m (3H); 4.42 s (1H); 3.83 s (3H); 3.56 m (1H); 3.08 dd (*J* = 14.9, 2.6 Hz, 1H); 2.61 dd (*J* = 14.9, 10.6 Hz, 1H); 1.20 s (3H); 1.18 s (3H).

The following compounds were obtained in analogy to the preparation methods decribed in detail:

| **Ex.** | **Product; *Reagents*** | **Method analogous to** | **¹H-NMR (400 MHz) δ [ppm]** | **Structure** |
|---|---|---|---|---|
| **16** | 3-Chloro-3'-[(R)-2-hydroxy-1-(1H-indol-3-ylmethyl)-2-methyl-propylsulfamoyl]-4'-methoxybiphenyl-4-carboxylic acid methylamide; *5-Bromo-N-[(R)-2-hydroxy-1-(1H-indol-3-ylmethyl)-2-methylpropyl]-2-methoxybenzenesulfonamide* *and* *3-Chloro-4-(N-methylcarbamoyl)benzene boronic acid* | **15** | **¹H-NMR (DMSO-d₆): 10.24** s (1H); 8.36 q (*J* = 4.6 Hz, 1H); 7.56 dd (*J* = 8.6, 2.5 Hz, 1H), 7.44 d (*J* = 7.8 Hz, 1H); 7.38 m (1H); 7.35 dd (*J* = 8.1, 1.8 Hz, 1H); 7.31 m (2H); 6.96 m (1H); 6.89 d (*J* = 8.6 Hz, 2H); 6.75 m (3H); 4.43 s (1H); 3.83 s (3H); 3.55 m (1H); 3.08 m (1H); 2.78 d (*J* = 4.6 Hz, 3H); 2.61 dd (*J* = 14.7, 12.9 Hz, 1H); 1.20 s (3H); 1.18 s (3H). | 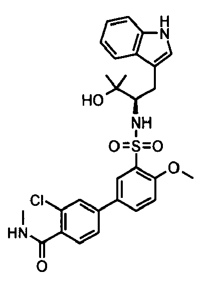 |
| **17** | 3'-Chloro-4-methoxy-4'-(morpholine-4-carbonyl)-biphenyl-3-sulfonic acid [(R)-2-hydroxy-1-(1H-indol-3-ylmethyl)-2-methyl-propyl]-amide; *5-Bromo-N-[(R)-2-hydroxy-1-(1H-indol-3-ylmethyl)-2-methylpropyl]-2-methoxybenzenesulfonamide* *and* *3-Chloro-4-(Morpholine-4-carbonyl)benzene boronic acid* | **15** | **¹H-NMR (DMSO-d₆):** 10.23 s (1H); 7.58 dd (*J* = 8.6, 2.0 Hz, 1H), 7.44 d (*J* = 7.3 Hz, 1H); 7.40 s (2H); 7.31 m (2H); 6.96 m (1H); 6.90 m (2H); 6.74 m (3H); 4.43 s (1H); 3.83 s (3H); 3.67 m (4H); 3.55 m (3H); 3.19 t (*J* = 4.3 Hz, 2H); 3.07 m (1H); 2.60 dd (*J* = 14.7, 10.4 Hz, 1H); 1.20 s (3H); 1.17 s (3H). | 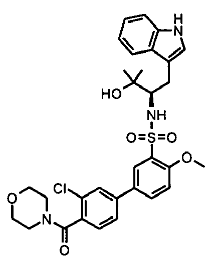 |

### Example 18

### 4-{3-[(R)-2-Hydroxy-1-(1H-indol-3-ylmethyl)-ethylsulfamoyl]-4-methoxyphenylethynyl}-N-methyl-benzamide

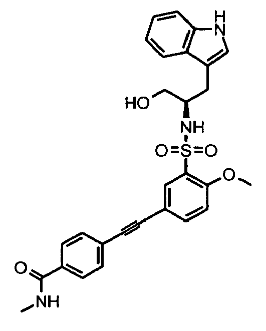

### 18a) (R)-2-(5-Bromo-2-methoxy-benzenesulfonylamino)-3-(1H-indol-3-yl)-propionic acid methyl ester was prepared as described above.

### 18b) 5-Bromo-N-[(R)-2-hydroxy-1-(1H-indol-3-ylmethyl)-ethyl]-2-methoxy-benzenesulfonamide

A solution of (R)-2-(5-Bromo-2-methoxy-benzenesulfonylamino)-3-(1H-indol-3-yl)-propionic acid methyl ester (1.48 g) was dissolved in THF (30 mL) and a solution of lithium borohydride (2M in THF, 4.75 mL) was added at ambient temperature. The reaction was stirred 72 hours, then quenched with hydrochloric acid (4 M). The mixture was extracted with ethyl acetate The organic phase was dried over magnesium sulphate and the solvent was distilled off under reduced pressure. The title compound was obtained after flash chromatography in 30 % yield (420 mg). ¹**H-NMR (DMSO-d₆):** 10.70 s (1H); 7.69 d (*J* = 2.6 Hz, 1H); 7.59 dd (*J* = 8.9, 2.6 Hz, 1H); 7.24 d (*J* = 7.9 Hz, 1H); 7.19 m (2H); 6.98 m (3H); 6.89 m (1H); 4.68 m (1H); 3.77 s (3H); 3.29 m (3H); 2.89 dd (*J* = 14.3, 6.4 Hz, 1H); 2.64 dd (*J* = 14.5, 6.0 Hz, 1H).

### 18c) 4-{3-[(R)-2-Hydroxy-1-(1H-indol-3-ylmethyl)-ethylsulfamoyl]-4-methoxyphenylethynyl}-N-methyl-benzamide

A solution of 5-Bromo-N-[(R)-2-hydroxy-1-(1H-indol-3-ylmethyl)-ethyl]-2-methoxy-benzenesulfonamide (100 mg), Pd(PPh₃)₂Cl₂ (9 mg), 4-Ethynyl-N-methyl-benzamide (38 mg), TBAF x 3 water (180 mg) in THF (3 mL) and ethanol (0.3 mL) was stirred at 110°C in a sealed microwave reactor for 30 min. The solvents were distilled off under reduced pressure. The crude mixture was taken up in ethyl acetate and extracted with water. The organic phase was reduced *in vacuo* and the title compound was obtained in 35% yield (41 mg) after flash chromatography. **¹H-NMR (DMSO-d₆):** 10.70 s (1H); 8.54 q (*J* = 4.6 Hz, 1H); 7.88 d (*J* = 8.1 Hz, 2H); 7.82 d (*J* = 2.3 Hz, 1H); 7.65 m (3H); 7.25 d (*J* = 8.6 Hz, 2H); 7.13 d (*J* = 6.3 Hz, 1H); 7.08 d (*J* = 8.8 Hz, 1H); 6.99 m (2H); 6.89 t (*J* = 7.1 Hz, 1H); 4.68 t (*J* = 5.3 Hz, 1H); 3.82 s (3H); 3.31 m (3H); 2.90 dd (*J* = 14.7, 6.8 Hz, 1H); 2.80 d (*J* = 4.3 Hz, 3H); 2.68 dd (*J* = 14.4, 6.1 Hz, 1H).

The following compound was obtained in analogy to the preparation methods decribed in detail:

| **Ex.** | **Product; *Reagents*** | **Method analogous to** | **¹H-NMR (400 MHz) δ [ppm]** | **Structure** |
|---|---|---|---|---|
| **19** | 3-{3-[(R)-2-Hydroxy-1-(1H-indol-3-ylmethyl)-ethylsulfamoyl]-4-methoxy-phenylethynyl}-N-methylbenzamide; *5-Bromo-N-[(R)-2-hydroxy-1-(1H-indol-3-ylmethyl)-ethyl]-2-methoxy-benzenesulfonamide* *and* *3-Ethynyl-N-methyl-benzamide* | **18** | **¹H-NMR (DMSO-d₆):** 10.71 s (1H); 8.56 m (1H); 8.02 m (1H); 7.86 m (1H); 7.82 d (*J* = 2.3 Hz, 1H); 7.70 m (1H); 7.66 dd (*J* = 8.7, 2.1 Hz, 1H); 7.53 t (*J* = 7.7 Hz, 1H); 7.25 d (*J* = 8.3 Hz, 2H); 7.13 d (*J* = 5.8 Hz, 1H); 7.09 d (*J* = 8.7 Hz, 1H); 6.99 m (2H); 6.89 m (1H); 4.67 t (*J* = 4.9 Hz, 1H); 3.82 s (3H); 3.31 m (3H); 2.91 dd (*J* = 14.1, 5.7 Hz, 1H); 2.80 d (*J* = 4.5 Hz, 3H); 2.67 dd (*J* = 14.0, 5.1 Hz, 1H). | 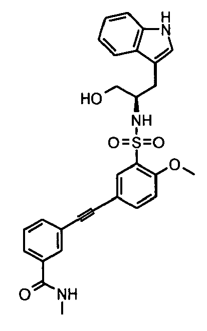 |

### Example 20

### 3-{3-[(R)-2-Hydroxy-1-(1H-indol-3-ylmethyl)-ethylsulfamoyl]-4-propoxy-phenylethynyl}-N-methyl-benzamide

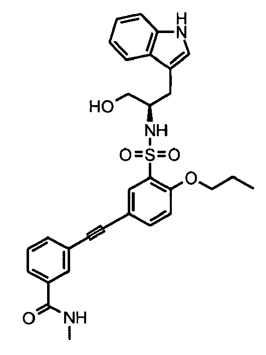

### 20a) 5-Bromo-N-[(R)-2-hydroxy-1-(1H-indol-3-ylmethyl)-ethyl]-2-propoxy-benzenesulfonamide

A solution of 5-Bromo-2-propoxy-benzenesulfonyl chloride (5.0 g) in tetrahydrofurane (50 mL) was added to a solution of (R)-2-Amino-3-(1H-indol-3-yl)-propan-1-ol (3.64 g) in tetrahydrofurane (50 mL) and diisopropyl ethyl amine (8.19 mL) at 0 °C. The reaction mixture was warmed to room temperature, stirred over night and then concentrated under reduced pressure. The title compound was obtained in 82% yield (6.1 g). **¹H-NMR (DMSO-d₆):** 10.70 s (1H); 7.73 d (*J* = 2.5 Hz, 1H); 7.56 dd (*J* = 8.6, 2.5 Hz, 1H); 7.23 m (2H); 7.00 m (2H); 6.90 m (2H); 6.68 d (*J* = 6.3 Hz, 1H); 4.76 t (*J* = 5.3 Hz, 1H); 3.89 m (2H); 3.32 m (3H); 2.90 dd (*J* = 14.4, 7.1 Hz, 1H); 2.69 dd (*J* = 14.4, 6.3 Hz, 1H); 1.72 m (2H); 0.95 t (*J* = 7.3 Hz, 3H).

### 20b) 3-{3-[(R)-2-Hydroxy-1-(1H-indol-3-ylmethyl)-ethylsulfamoyl]-4-propoxyphenylethynyl}-N-methyl-benzamide

A solution of 5-Bromo-N-[(R)-2-hydroxy-1-(1H-indol-3-ylmethyl)-ethyl]-2-propoxy-benzenesulfonamide (100 mg), Pd(PPh₃)₂Cl₂ (7 mg), 3-Ethynyl-N-methyl-benzamide (34 mg), TBAF x 3 water (280 mg) in THF (3 mL) and ethanol (0.3 mL) was stirred at 110°C in a sealed microwave reactor for 30 min. The solvents were distilled off under reduced pressure. The crude mixture was taken up in ethyl acetate and extracted with water. The organic phase was reduced *in vacuo* and the title compound was obtained in 38% yield (44 mg) after flash chromatography.
**¹H-NMR (DMSO-d₆):** 10.71 s (1H); 8.55 q (*J* = 4.3 Hz, 1H); 8.02 m (1H); 7.86 m (2H); 7.70 m (1H); 7.63 dd (*J* = 8.7, 2.3 Hz, 1H); 7.53 t (*J* = 7.7 Hz, 1H); 7.27 d (*J* = 7.5 Hz, 1H); 7.26 d (*J* = 7.9 Hz, 1H); 7.01 m (3H); 6.89 t (*J* = 7.4 Hz, 1H); 6.62 d (*J* = 5.7 Hz, 1H); 4.75 m (1H); 3.94 m (2H); 3.32 m (3H); 2.92 dd (*J* = 14.7, 7.2 Hz, 1H); 2.80 d (*J* = 4.5 Hz, 3H); 2.72 dd (*J* = 14.3, 5.7 Hz, 1H); 1.73 m (2H); 0.96 t (*J* = 7.5 Hz, 3H).

The following compound was obtained in analogy to the preparation methods decribed in detail:

| **Ex.** | **Product; *Reagents*** | **Method gous to** | **¹H-NMR (400 MHz) δ analo-[ppm]** | **Structure** |
|---|---|---|---|---|
| **21** | 4-{3-[(R)-2-Hydroxy-1-(1H-indol-3-ylmethyl)-ethylsulfamoyl]-4-propoxy-phenylethynyl}-N-methylbenzamide; *5-Bromo-N-[(R)-2-hydroxy-1-(1H-indol-3-ylmethyl)-ethyl]-2-propoxy-benzenesulfonamide* *and* *4-Ethynyl-N-methyl-benzamide* | **20** | **¹H-NMR (DMSO-d₆):** 10.76 s (1H); 8.59 m (1H); 7.89 m (2H); 7.85 d (*J* = 2.3 Hz, 1H); 7.65 m (3H); 7.27 m (2H); 7.01 m (3H); 6.88 t (*J* = 7.5 Hz, 1H); 6.64 d (*J* = 6.6 Hz, 1H); 4.79 m (1H); 3.93 m (2H); 3.37 m (2H); 3.27 m (1H); 2.91 dd (*J* = 14.7, 7.3 Hz, 1H); 2.80 d (*J* = 4.6 Hz, 3H); 2.72 dd (*J* = 14.5, 6.1 Hz, 1H); 1.73 m (2H); 0.96 t (*J* = 7.6 Hz, 3H). | 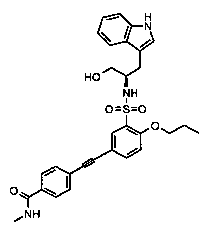 |

### Example 22

### 4-{3-[(R)-2-Hydroxy-1-(1H-indol-3-ylmethyl)-2-methyl-propyisulfamoyl]-4-methoxyphenylethynyl}-N-methyl-benzamide

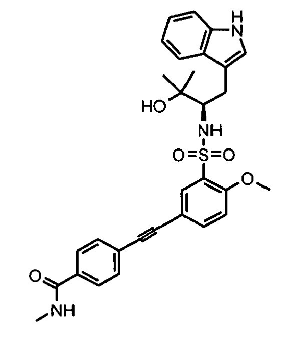

### 22a) 5-Bromo-N-[(R)-2-hydroxy-1-(1H-indol-3-ylmethyl)-2-methyl-propyl]-2-methoxy-benzenesulfonamide was prepared as described above.

### 22b) 4-{3-[(R)-2-Hydroxy-1-(1H-indol-3-ylmethyl)-2-methyl-propylsulfamoyl]-4-methoxyphenylethynyl}-N-methyl-benzamide

A solution of 5-Bromo-N-[(R)-2-hydroxy-1-(1H-indol-3-ylmethyl)-2-methyl-propyl]-2-methoxy-benzenesulfonamide (85 mg), Pd(PPh₃)₂Cl₂ (6 mg), 4-Ethynyl-N-methyl-benzamide (30 mg), TBAF x 3 water (140 mg) in THF (3 mL) and ethanol (0.3 mL) was stirred at 110°C in a sealed microwave reactor for 30 min. The solvents were distilled off under reduced pressure. The crude mixture was taken up in ethyl acetate and extracted with water. The organic phase was reduced *in vacuo* and the title compound was obtained in 27% yield (27 mg) after flash chromatography. **¹H-NMR (DMSO-d₆):** 10.35 s (1H); 8.54 q (*J* = 4.6 Hz, 1H); 7.88 d (*J* = 8.5 Hz, 2H); 7.62 d (*J* = 8.3 Hz, 2H); 7.42 dd (*J* = 8.5, 2.1 Hz, 1H); 7.35 d (*J* = 7.7 Hz, 1H); 7.28 d (J = 2.3 Hz, 1H); 7.12 d (*J* = 7.7 Hz, 1H); 6.90 m (5H); 4.41 s (1H); 3.77 s (3H); 3.52 m (1H); 3.08 m (1H); 2.80 d (*J* = 4.3 Hz, 3H); 2.62 dd (*J* = 14.5, 10.1 Hz, 1H); 1.15 s (6H).

The following compound was obtained in analogy to the preparation methods decribed in detail:

| **Ex.** | **Product; *reagents*** | **Method analogous to** | **¹H-NMR (400 MHz) δ [ppm]** | **Structure** |
|---|---|---|---|---|
| **23** | 3-{3-[(R)-2-Hydroxy-1-(1H-indol-3-ylmethyl)-2-methyl-propylsulfamoyl]-4-methoxy-phenylethynyl}-N-methylbenzamide; *5-Bromo-N-((R)-2-hydroxy-1-(1H-indol-3-ylmethyl)-2-methylpropyl]-2-methoxy-benzenesulfonamide* *and* *3-Ethynyl-N-methyl-benzamide* | 22 | **¹H-NMR (DMSO-d₆):** 10.35 m(1H); 8.55 m (1H); 7.99 m (1H); 7.86 m (1H); 7.67 m (1H); 7.52 t (*J* = 7.7 Hz, 1H); 7.42 dd (*J* = 8.5, 2.1 Hz, 1H); 7.35 d (*J* = 7.4 Hz, 1H); 7.29 d (*J* = 2.1 Hz, 1H); 7.13 d (*J* = 7.5 Hz, 1H); 6.90 m (5H); 4.40 s (1H); 3.78 s (3H); 3.52 m (1H); 3.08 m (1H); 2.81 d (*J* = 4.5 Hz, 3H); 2.62 dd (*J* = 14.7, 10.5 Hz, 1H); 1.15 s (6H). | 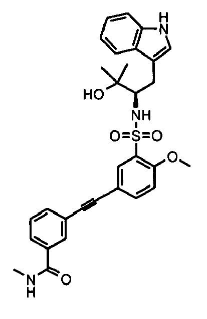 |

### Example 24

### 2-Chloro-4-{7-[(R)-2-hydroxy-1-(1H-indol-3-ylmethyl)-ethylsulfamoyl]-2,3-dihydrobenzofuran-5-yl}-N-methyl-benzamide

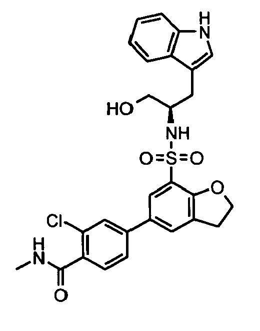

### 24a) 5-Bromo-2,3-dihydro-benzofuran-7-sulfonic acid [(R)-2-hydroxy-1-(1H-indol-3-ylmethyl)-ethyl]-amide

5-Bromo-2,3-dihydro-benzofuran-7-sulfonyl chloride (300 mg) was added to a solution of (D)-Trypophanol (240 mg) in dichloromethane (6 mL) and pyridine (1 mL) at ambient temperature. The reaction was stirred at ambient temperature for one our and quenched by the addition of sulphuric acid (2N). The reaction mixture was diluted with brine and dichloromethane and the phases were separated. The organic phases were dried over sodium sulphate and the solvent was distilled off under reduced pressure. The titite compound was obtained in 29% yield (130 mg) after flash chromatography. **¹H-NMR (DMSO-d₆):** 10.60 s (1H); 7.35 d (*J* = 7.3 Hz, 1H); 7.28 s (2H); 7.23 m (2H); 6.96 m (2H); 6.87 m (1H); 4.72 m (1H); 4.43 m (2H); 3.41 m (1H); 3.34 m (1H); 3.23 m (1H); 3.04 m (2H); 2.88 m (1H); 2.57 m (1H).

### 24b) 2-Chloro-4-{7-[(R)-2-hydroxy-1-(1H-indol-3-ylmethyl)-ethylsulfamoyl]-2,3-dihydrobenzofuran-5-yl}-N-methyl-benzamide

A solution of 5-Bromo-2,3-dihydro-benzofuran-7-sulfonic acid [(R)-2-hydroxy-1-(1H-in dol-3-ylmethyl)-ethyl]-amide (35 mg), 3-Chloro-4-methylcarbamoyl-phenyl boronic acid (18 mg), Pd(PPh₃)₄ (9 mg) in ethanol (1.04 mL), toluene (1.04 mL) and sodium carbonate (21 mg) in water (190 µL) was stirred at 100°C overnight. The solvent was evaporated and the solid purified by flash chromatography to yield 69 % of the title compound (29 mg). **¹H-NMR (DMSO-d6):** 10.56 s (1H); 8.34 d (*J* = 4.6 Hz, 1H); 7.55 s (1H); 7.48 m (4H); 7.26 d (*J* = 6.8 Hz, 1H); 7.19 m (2H); 6.95 s (1H); 6.88 m (1H); 6.71 m (1H); 4.71 m (1H); 4.52 m (2H); 3.42 m (1H); 3.33 m (1H); 3.24 m (1H); 3.12 m (2H); 2.87 m (1H); 2.62 m (1H).

The following compound was obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **25** | 5-(3,4, 5-Trimethoxy-phenyl)-2,3-dihydro-benzofuran-7-sulfonic acid [(R)-2-hydroxy-1-(H-indol-3-ylmethyl)-ethyl]-amide; *5-Bromo-2,3-dihydrobenzofuran-7-sulfonic acid [(R)-2-hydroxy-1-(1H-indol-3-ylmethyl)-ethyl]-amide* *and* *3,4,5-Trimethoxy-phenyl boronic acid* | **24** | **¹H-NMR (DMSO-d₆):** 10,83 s (1H); 7.56 m (2H); 7.22 m (3H); 6.98 d (*J* = 2.0 Hz, 1H); 6.93 m (1H); 6.75 m (1H); 6.72 s (2H); 4.67 m (1H); 4.53 m (2H); 3.81 s (6H); 3.65 s (3H); 3.35 m (2H); 3.23 m (1H); 3.15 m (1H); 2.87 m (1H); 2.63 m (1H). | 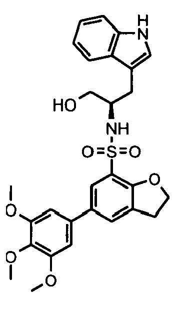 |

### Example 26

### 5-(4-Acetyl-phenyl)-2,3-dihydro-benzofuran-7-sulfonic acid [(R)-2-hydroxy-1-(1H-indol-3-ylmethyl)-ethyl]-amide

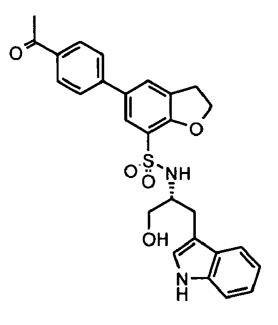

5-Bromo-2,3-dihydro-benzofuran-7-sulfonic acid [(R)-2-hydroxy-1-(1H-indol-3-ylmethyl)-ethyl]-amide (0.15 mmol, 0.2M in THF), 4-Acetyl-phenylboronic acid (1.5 equivalents, 0.4 M in THF), palladium-(II)-actate (0.1 equivalents, 0.0375M in THF), trisorthotolylphosphine (0.2 equivalents, 0.05 M in THF), triethylamine (1.2 equivalents, 0.6 M in THF) and water (200 µL) were stirred under microwave irradiation at 130°C for 45 minutes. The reaction mixture was cooled to ambient temperature, the solvent was removed under reduced pressure, DMSO (2 mL) were added and the crude product was purified via HPLC to yield the title compound.
HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75').
Molecular peak (ESI, M+1): 492; Retention time: 3.49 min.

The following compound was obtained in analogy to the preparation methods described in detail:

| **Ex.** | **Product; *reagents*** | **Method analogous to** | **HPLC-MS** | **Structure** |
|---|---|---|---|---|
| **27** | 5-(4-Methylsulfanyl-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzo-furan-7-sulfonic acid [(R)-2-hydroxy-1-(1H-indol-3-yl-methyl)-ethyl]-amide* *and* *4-Methylsulfanyl-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 496 Retention time: 4.03 min. | 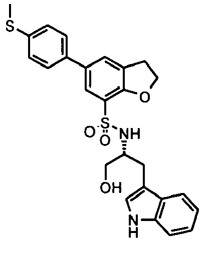 |
| **28** | 5-(3-Amino-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Amino-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 465 Retention time: 2.65 min. | 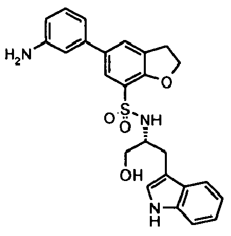 |
| **29** | 5-(3-Trifluoromethyl-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Trifluoromethyl-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 518 Retention time: 4.11 min. | 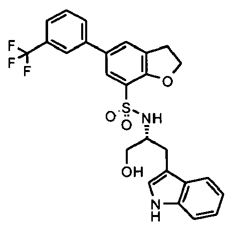 |
| **30** | 5-(4-Hydroxy-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *4-Hydroxy-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 466 Retention time: 3.22 min. | 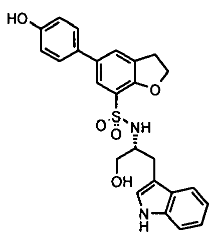 |
| **31** | 5-(4-Fluoro-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *4-Fluoro-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 468 Retention time: 3.86 min. | 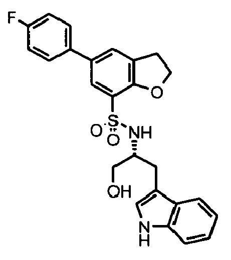 |
| **32** | 5-(4-Cyano-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *4-Cyano-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 475 Retention time: 3.59 min. | 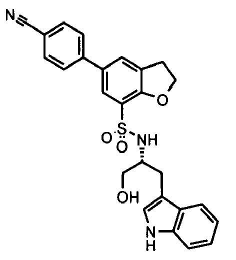 |
| **33** | 5-Naphthalen-1-yl-2,3-dihydro-benzofuran-7-sulfonic acid [(R)-2-hydroxy-1-(1-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Naphthalene-1- boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1%(1.75'). Molecular peak (ESI, M+1): 500 Retention time: 4.22 min. | 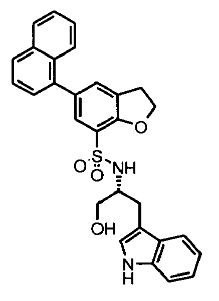 |
| **34** | 5-(4-Chloro-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *4-Chloro-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 484 Retention time: 4.16 min. | 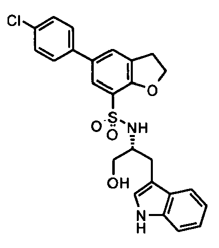 |
| **35** | 2-{7-[(R)-2-Hydroxy-1-(1H-indol-3-ylmethyl)-ethylsulfamoyl]-2,3-dihydro-benzofuran-5-yl}-benzamide; *5-Bromo-2,3-dihydro-benzofuran-7-sulfonic acid [(R)-2-hydroxy-1-(1H-indol-3-ylmethyl)-ethyl]-amide* *and* *2-Carbamoyl-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 493 Retention time: 3.07 min. | 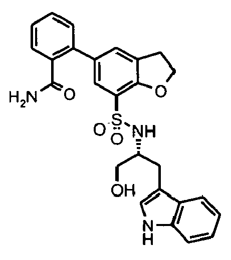 |
| **36** | 5-(6-Methoxy-pyridin-3-yl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *6-Methoxy-pyridine-3-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 481 Retention time: 3.21 min. | 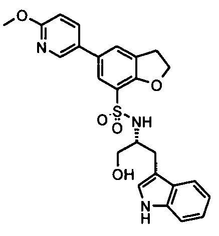 |
| **37** | 5-(2-Fluoro-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *2-Fluoro-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 468 Retention time: 3.87 min. | 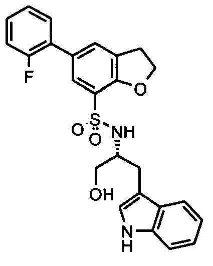 |
| **38** | 4-{7-[(R)-2-Hydroxy-1-(1H-indol-3-ylmethyl)-ethyl-sulfamoyl]-2,3-dihydro-benzofuran-5-yl}-N-methyl-benzamide; *5-Bromo-2, 3-dihydro-benzofuran-7-sulfonic acid [(R)-2-hydroxy-1-(1H-indol-3-ylmethyl)-ethyl]-amide* *and* *4-Methylcarbamoyl-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 507 Retention time: 2.99 min. | 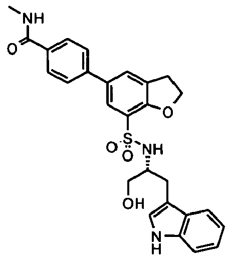 |
| **39** | 5-Quinolin-6-yl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Quinoline-6-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 501 Retention time: 2.65 min. | 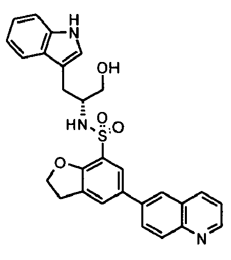 |
| **40** | 5-((E)-Styryl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *(E)-2-Phenyl-etheneboronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 476 Retention time: 4.07 min. | 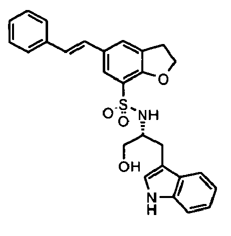 |
| **41** | 5-(3-Hydroxymethyl-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Hydroxymethyl-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 480 Retention time: 3.22 min. | 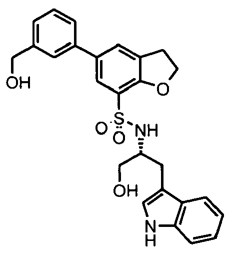 |
| **42** | 3-Fluoro-5-{7-[(R)-2-hydroxy-1-(1H-indol-3-ylmethyl)-ethylsulfamoyl]-2,3-dihydro-benzofuran-5-yl}-N-methyl-benzamide; *5-Bromo-2,3-dihydro-benzofuran-7-sulfonic acid [(R)-2-hydroxy-1-(1H-indol-3-ylmethyl)-ethyl]-amide* *and* *3-Fluoro-5-meth ylcarbamoyl-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 525 Retention time: 3.23 min. | 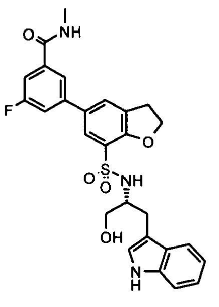 |
| **43** | 5-(3-Fluoro-5-methoxy-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Fluoro-5-methoxy-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 498 Retention time: 3.87 min. | 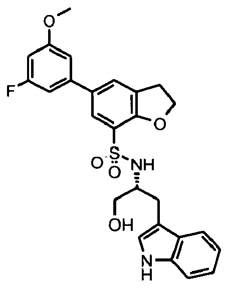 |
| **44** | N-(4-{7-[(R)-2-Hydroxy-1-(1H-indol-3-ylmethyl)-ethyl-sulfamoyl]-2,3-dihydro-benzofuran-5-yl}-phenyl)-acetamide; *5-Bromo-2,3-dihydro-benzofuran-7-sulfonic acid [(R)-2-hydroxy-1-(1H-indol-3-ylmethyl)-ethyl]-amide* *and* *4-Acetamido-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 507 Retention time: 3.1 min. | 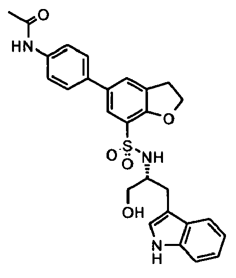 |
| **45** | 5-(3, 5-Di methyl-phenyl)-2, 3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3,5-Dimethyl-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 478 Retention time: 4.28 min. | 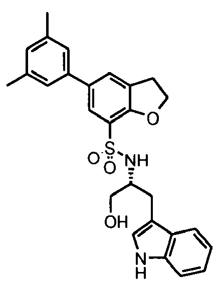 |
| **46** | 5-Quinolin-3-yl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Quinoline-3-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 501 Retention time: 2.72 min. | 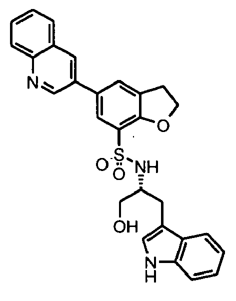 |
| **47** | 3-{7-[(R)-2-Hydroxy-1-(1H-indol-3-ylmethyl)-ethyl-sulfamoyl]-2,3-dihydro-benzofuran-5-yl}-benzamide; *5-Bromo-2,3-dihydro-benzofuran-7-sulfonic acid [(R)-2-hydroxy-1-(1H-indol-3-ylmethyl)-ethyl]-amide* *and* *3-Carbamoyl-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 493 Retention time: 2.97 min. | 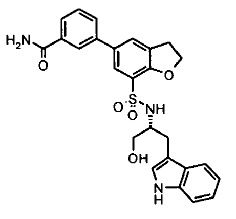 |
| **48** | 5-(2-Fluoro-pyridin-3-yl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *2-Fluoro-pyridine-3-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1%(1.75'). Molecular peak (ESI, M+1): 469 Retention time: 3.34 min. | 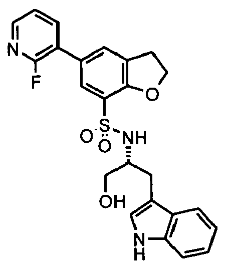 |
| **49** | 5-(5-Cyano-thiophen-2-yl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *2-Cyano-thiophene-5-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 481 Retention time: 3.59 min. | 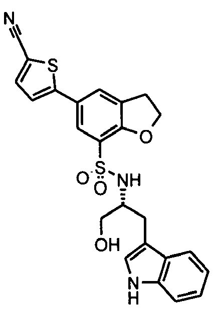 |
| **50** | N-(3-{7-[(R)-2-Hydroxy-1-(1H-indol-3-ylmethyl)-ethylsulfamoyl]-2,3-dihydro-benzofuran-5-yl}-benzyl)-acetamide; *5-Bromo-2,3-dihydro-benzofuran-7-sulfonic acid [(R)-2-hydroxy-1-(1H-indol-3-ylmethyl)-ethyl]-amide* *and* *3-(Acetylamino-methyl)-phenyl boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 521 Retention time: 3.09 min. | 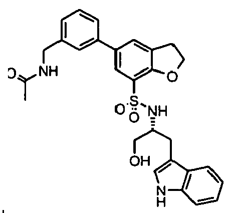 |
| **51** | 5-(2-Methoxy-pyrimidin-5-yl)-2,3-dihydro-benzofuran-7-sulfonic acid [(R)-2-hydroxy-1-(1H-indol-3-yl-methyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *2-Methoxy-pyrimidine-5-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 482 Retention time: 3.01 min. | 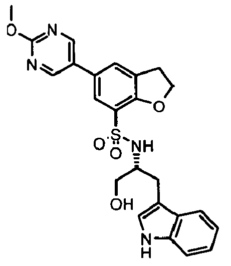 |
| **52** | 5-(4-Cyanomethyl-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *4-Cyanomethyl-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 489 Retention time: 3.62 min. | 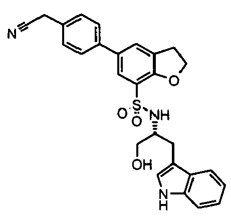 |
| **53** | 5-(3-Cyano-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Cyano-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 475 Retention time: 3.6 min. | 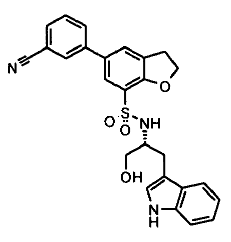 |
| **54** | 5-Benzo[1,3]dioxol-5-yl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide and Benzo[1,3]dioxole-5-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 494 Retention time: 3.71 min. | 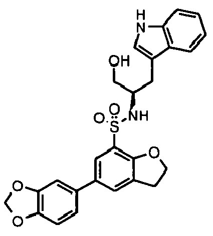 |
| **55** | N-(3-{7-[(R)-2-Hydroxy-1-(1H-indol-3-ylmethyl)-ethyl-sulfamoyl]-2,3-dihydro-benzofuran-5-yl}-phenyl)-acetamide; *5-Bromo-2,3-dihydro-benzofuran-7-sulfonic acid [(R)-2-hydroxy-1-(1H-indol-3-ylmethyl)-ethyl]-amide* *and* *3-Acetamido-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 507 Retention time: 3.19 min. | 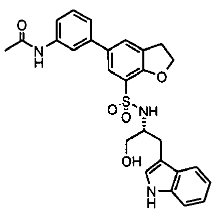 |
| **56** | 5-Biphenyl-2-yl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Biphenyl-2-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 526 Retention time: 4.37 min. | 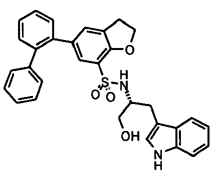 |
| **57** | 5-o-Tolyl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *2-Methyl-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 464 Retention time: 4.02 min. | 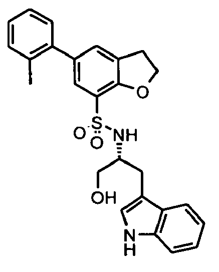 |
| **58** | 5-(3-Methanesulfonylamino-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* 3-Methanesulfonylamino-phenyl-boronic acid | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 543 Retention time: 3.34 min. | 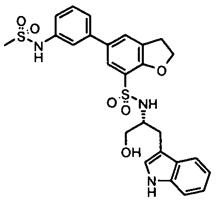 |
| **59** | 5-(4-Trifluoromethyl-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *4-Trifluoromethyl-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 518 Retention time: 4.18 min. | 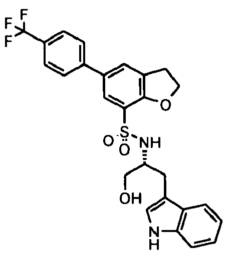 |
| **60** | 5-Benzo[b]thiophen-3-yl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Benzo[b]thiophene-3-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 506 Retention time: 4.29 min. | 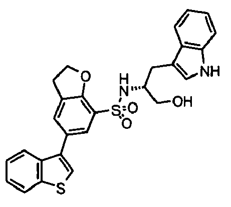 |
| **61** | 5-Biphenyl-3-yl-2, 3-dihyd ro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Biphenyl-3-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 526 Retention time: 4.36 min. | 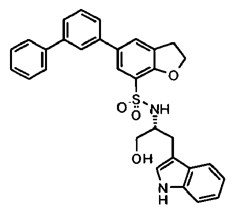 |
| **62** | 5-(3-Acetyl-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Acetyl-phenylboronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 492 Retention time: 3.52 min. | 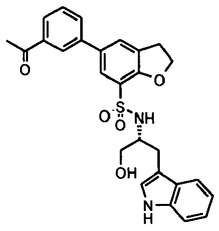 |
| **63** | 5-(3-Fluoro-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Fluoro-phenyl-boronic acid* | **26** | HPLC purification: Column X-Bridge RP C 18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 468 Retention time: 3.88 min. | 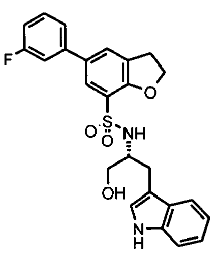 |
| **64** | 2',3'-Dihydro-[2,5']bibenzofuranyl-7'-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide and Benzofuran-2-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 490 Retention time: 4.07 min. | 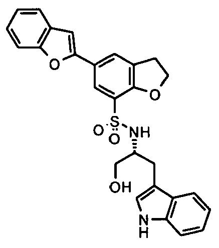 |
| **65** | 5-Benzo[b]thiophen-2-yl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Benzothiophen-2-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 506 Retention time: 4.18 min. | 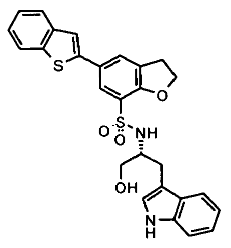 |
| **66** | 5-(3-Chloro-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Chloro-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 484 Retention time: 4.12 min. | 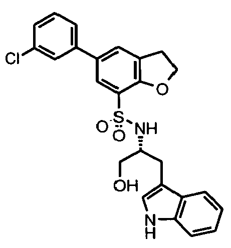 |
| **67** | 5-p-Tolyl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *4-Methyl-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O B 0.1% TFA in ACN; gradient in each case based on B: 1% to 99% (5') to 99% (1') to 1% (0.25') to 1% (1.75'). Molecular peak (ESI, M+1): 464 Retention time: 4.04 min. | 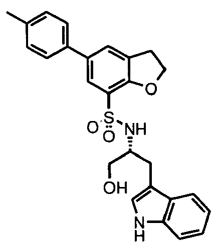 |
| **68** | 5-Naphthalen-2-yl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Naphthalene-2-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 500 Retention time: 4.2 min. | 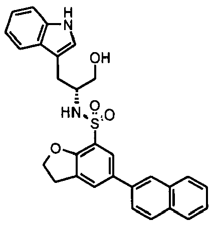 |
| **69** | 5-(3-Methoxy-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *3-Methoxy-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 480 Retention time: 3.88 min. | 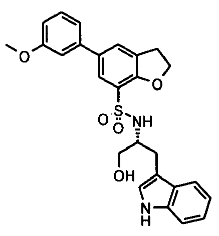 |
| **70** | 5-(4-Methoxy-phenyl)-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *4-Methoxy-phenyl-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 480 Retention time: 3.77 min. | 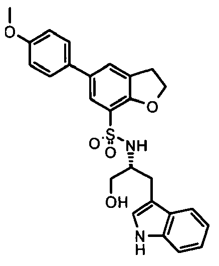 |
| **71** | 5-Thiophen-3-yl-2,3-dihydro-benzofuran-7-sulfonic acid [(R)-2-hydroxy-1-(1H-indol-3-ylmethyl)-ethyl]-amide; *5-Bromo-2,3-dihydro-benzofuran-7-sulfonic acid [(R)-2-hydroxy-1-(1H-indol-3-ylmethyl)-ethyl]-amide* *and* *Thiophene-3-boronic acid* | **26** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 456 Retention time: 3.77 min. | 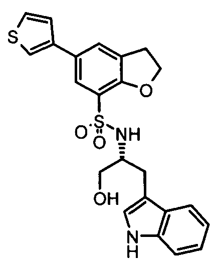 |

## Claims

1. Use of compounds of the formula I 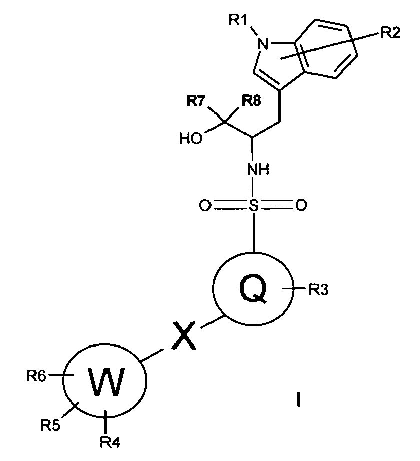 in which
R1 may be hydrogen, C₁-C₆-alkyl, C₃-C₆-alkenyl, C₃-C₆-alkynyl, C₃-C₇-cycloalkyl, C₁-C₆-alkyloxy-C₁-C₆-alkylene, C₃-C₇-cycloalkyloxy-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups: 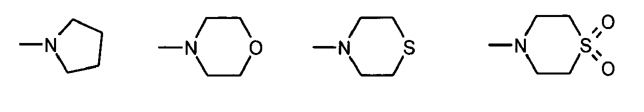 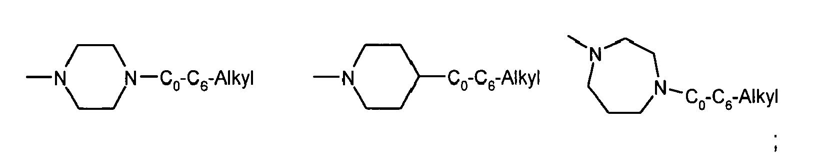
R2 may be hydrogen, halogen, cyano, -SO₂Me, C₁-C₆-alkyl, C₂-C₆-alkenyl, C₂-C₆-alkynyl, C₁-C₆-alkyloxy or benzyloxy,
where the hydrocarbon chains therein may optionally be substituted one or more times by fluorine;
R3 may be hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylam ino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups 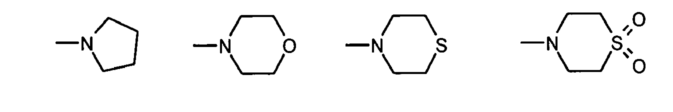 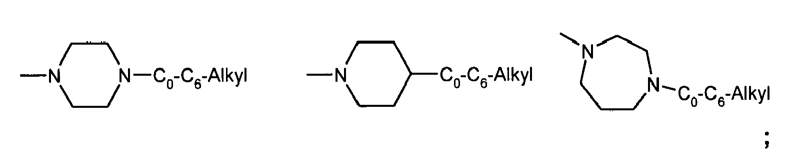
R4, R5, R6 may be independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl,
where the hydrocarbon chains therein may optionally be substituted one or more times by fluorine, cyano or the radicals: 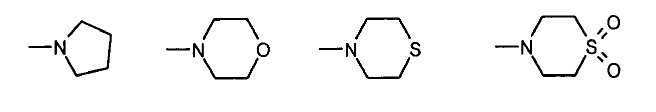 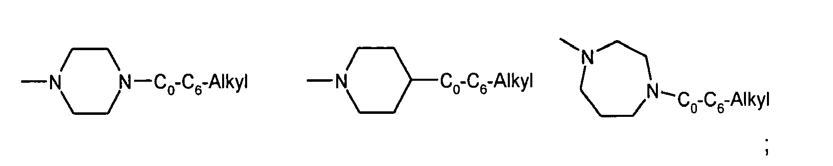 or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl,
C₁-C₆-alkylcarbonyl, C₃-C₇-cycloalkylcarbonyl, carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇cycloalkyl, -N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C,-C₆-alkylene)]amine,
or the radicals: 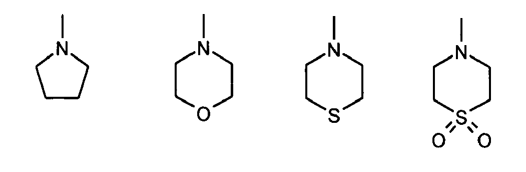 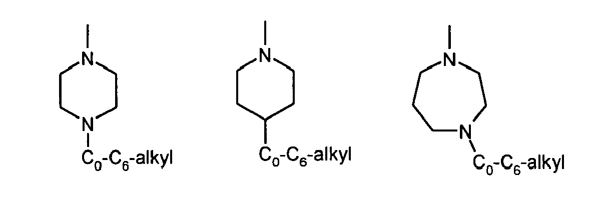 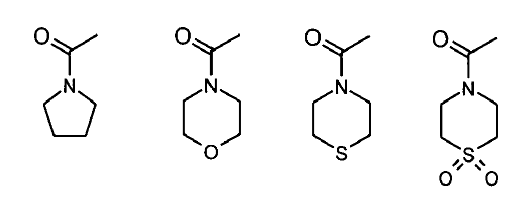 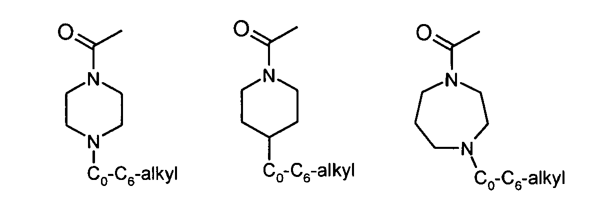 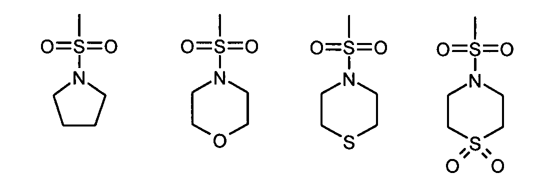 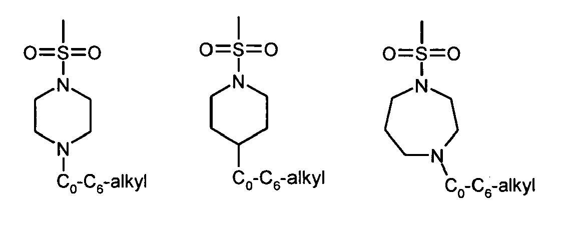 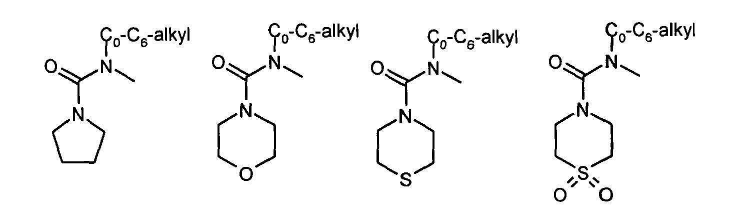 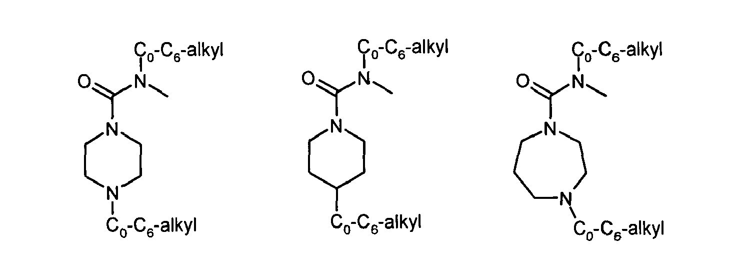 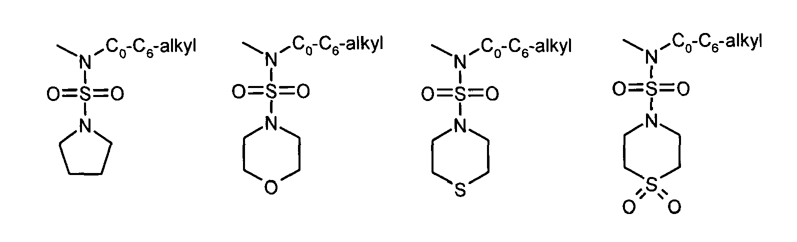 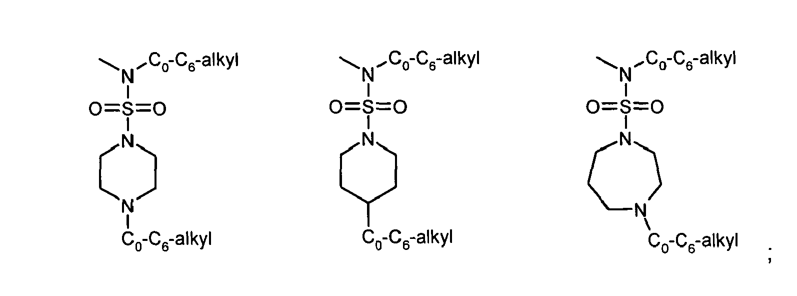
R5 and R6 may together form heterocycloalkyl, cycloalkyl;
R7, R8 may be independently of one another hydrogen, methyl, ethyl,
where the methyl and ethyl radicals may be fluorinated one or more times;
Q may be an aryl or heteroaryl group or
the group 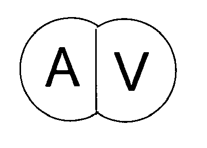 in which
A is a monocyclic aryl or a monocyclic heteroaryl group;
V is a cycloalkylen, cycloalkenylen, heterocycloalkylen or heterocycloalkenylen group;
X may be a bond, C₁-C₄-alkylene, C₂-C₄-alkenylene, C₂-C₄-alkynylene;
W may be an aryl or heteroaryl group;
where
R2 may substitute one or more positions of the aryl or heteroaryl ring in the indole residue;
R3 may substitute one or more positions of the aryl or heteroaryl ring in the radical Q and or the radical V,
for producing medicaments for the treatment and/or prevention of osteoporosis.

2. Use of compounds of formula I as defined in claim 1 for fertility control in men or women.

3. Use according to any of the preceding claims, **characterised in that** in formula I
R3 may be hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano, hydroxy, amino or the groups 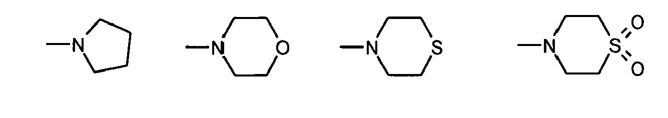 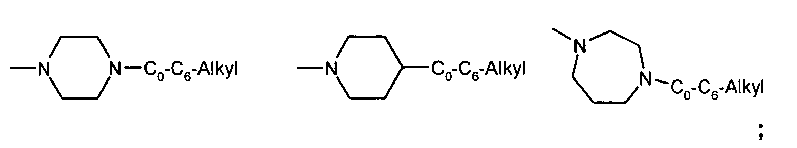
R4 may be hydrogen, hydroxy, halogen, nitro, amino, cyano, phenyl, C₁-C₆-alkyl, C₂-C₆-alkenyl or C₂-C₆-alkynyl, C₃-C₇-cycloalkyl,
where the hydrocarbon chains therein may optionally be substituted one or more times by fluorine, cyano or the radicals: 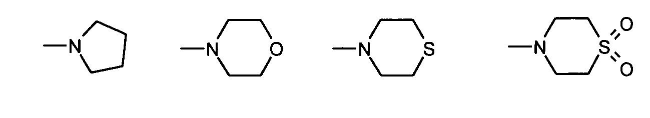 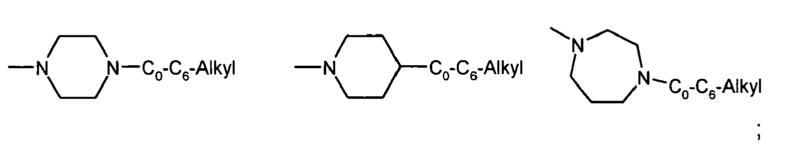 or
independently of one another hydroxy-C₁-C₆-alkylene, C₁-C₆-alkyloxy, C₁-C₆-alkyloxyphenyl-C₁-C₆-alkylene, phenyloxy-C₁-C₆-alkylene, C₁-C₆-alkylamino, di(C₁-C₆-alkyl)amino-;
C₁-C₆-acyl-(C₀-C₆-alkyl)amido,
C₁-C₆-alkylcarbonyl,
carboxy, C₁-C₆-alkyloxycarbonyl,
C₁-C₃-alkylsulphanyl, C₁-C₆-alkysulphonyl,-N(C₀-C₆-alkyl)-C(O)-N-di(C₀-C₆-alkyl),
and
R1, R2, R5, R6, R7, R8, Q, X and W have the same meaning as defined in claim 1.

4. Compounds of formula I, namely
1 4,3',4',5'-Tetramethoxy-biphenyl-3-sulfonic acid [(R)-2-hydroxy-1-(1H-indol-3-yl-methyl)-ethyl]-amide;
10 4-Isopropoxy-3',4',5'-trimethoxy-biphenyl-3-sulfonic acid [(R)-2-hydroxy-1-(1H-indol-3-ylmethyl)-ethyl]-amide;
11 5-Pyridin-2-yl-thiophene-2-sulfonic acid [(R)-2-hydroxy-1-(1H-indol-3-yimethyl)-ethyl]-amide
12 4'-Trifluoromethyl-biphenyl-3-sulfonic acid [(R)-2-hydroxy-1-(1H-indol-3-ylmethyl)-ethyl]-amide
13 4'-Methyl-biphenyl-3-sulfonic acid [(R)-2-hydroxy-1-(1H-indol-3-ylmethyl)-ethyl]-amide
14 5-(2-Methyl-5-trifluoromethyl-2H-pyrazol-3-yl)-thiophene-2-sulfonic acid [(R)-2-hydroxy-1-(1H-indol-3-ylmethyl)-ethyl]-amide.

5. Compounds of formula I of claim 1 **characterised in that**
R3 may be hydroxy-C₃-C₆-alkenylene, hydroxy-C₃-C₆-alkynylene, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkylamino-C₁-C₆-alkylene, di(C₁-C₆-alkyl)amino-C₁-C₆-alkylene,
where the hydrocarbon chains therein may optionally be substituted one or more times by fluorine, cyano, hydroxy, amino or the groups 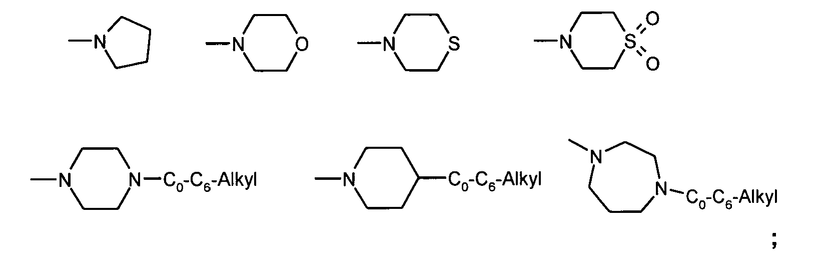
R4 may be hydrogen, hydroxy, halogen, nitro, amino, cyano, phenyl, C₁-C₆-alkyl, C₂-C₆-alkenyl or C₂-C₆-alkynyl, C₃-C₇-cycloalkyl,
where the hydrocarbon chains therein may optionally be substituted one or more times by fluorine, cyano or the radicals: 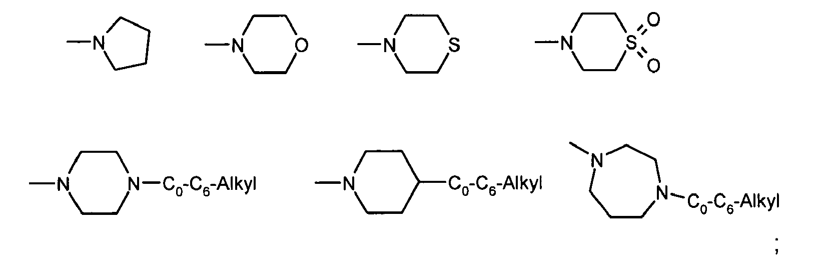 or
independently of one another hydroxy-C₁-C₆-alkylene, C₁-C₆-alkyloxy, C₁-C₆-alkyloxyphenyl-C₁-C₆-alkylene, phenyloxy-C₁-C₆-alkylene, C₁-C₆-alkylamino, di(C₁-C₆-alkyl)amino
C₁-C₆-acyl-(C₀-C₆-alkyl)amido,
C₁-C₆-alkylcarbonyl,
carboxy, C₁-C₆-alkyloxycarbonyl,
C₁-C₃-alkylsulphanyl, C₁-C₆-alkysulphonyl,-N(C₀-C₆-alkyl)-C(O)-N-di(C₀-C₆-alkyl),
and
R1, R2, R5, R6, R7, R8, Q, X and W have the same meaning as defined in claim 1.

6. Compounds of formula I of claim 1 **characterised in that**
R4 may be C₃-C₇-cycloalkyl-C₁-C₆-alkylene, C₃-C₇-heterocycloalkyl,
where the hydrocarbon chains therein may optionally be substituted one or more times by fluorine, cyano or the radicals: 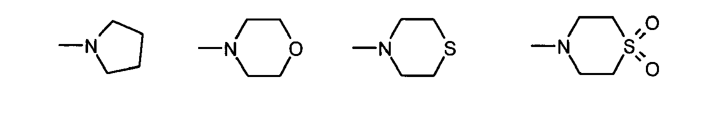 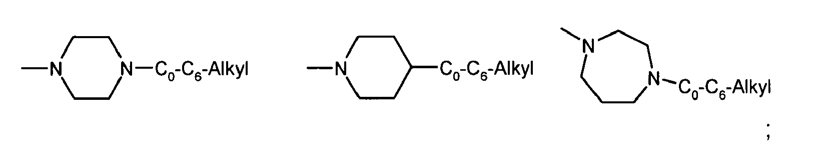 or
independently of one another hydroxy-C₃-C₆-alkenylene, hydroxy-C₃-C₆-alkynylene, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene,
C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl,
C₃-C₇-cycloalkylcarbonyl,
carboxamido [-C(O)NH₂],
C₃-C₇-cycloalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-O-(C₀-C₆)alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the radicals: 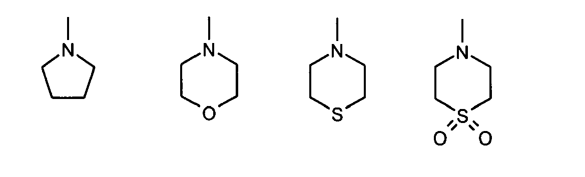 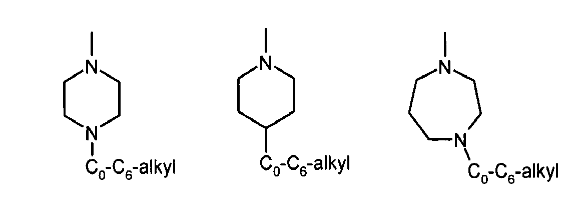 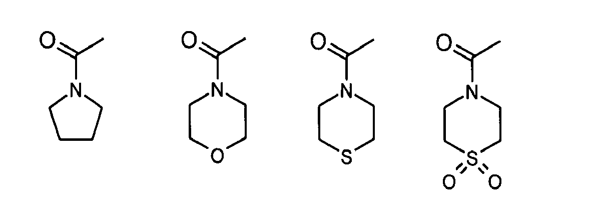 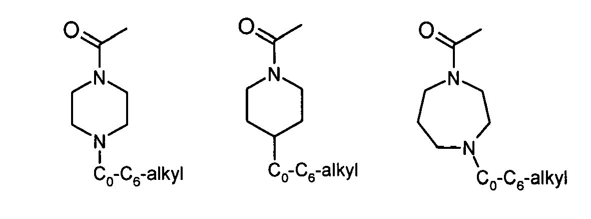 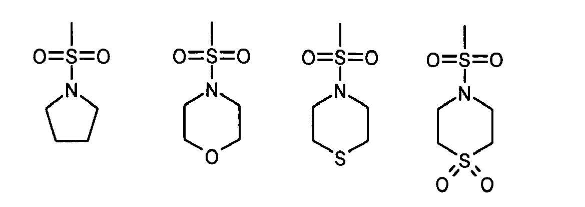 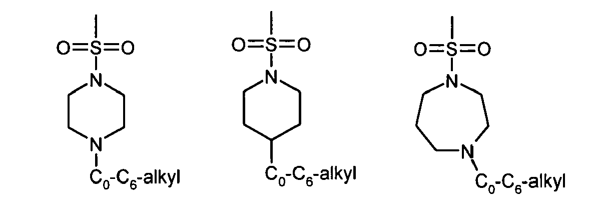 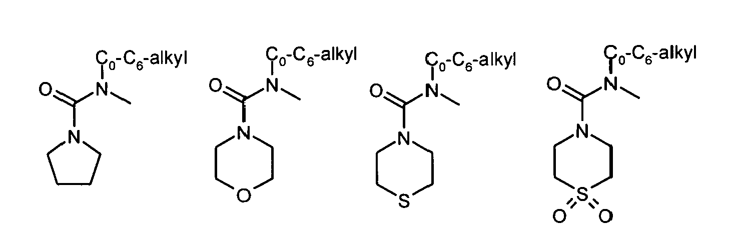 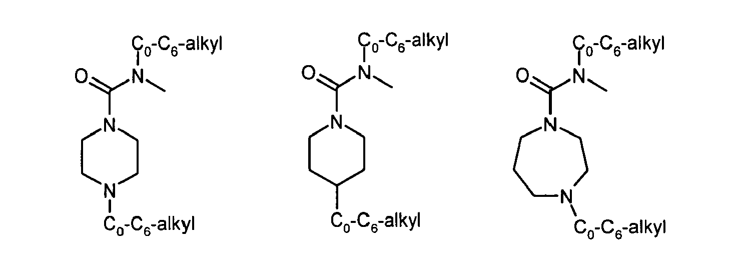 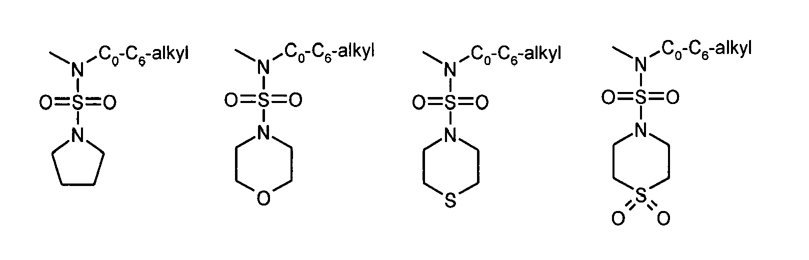 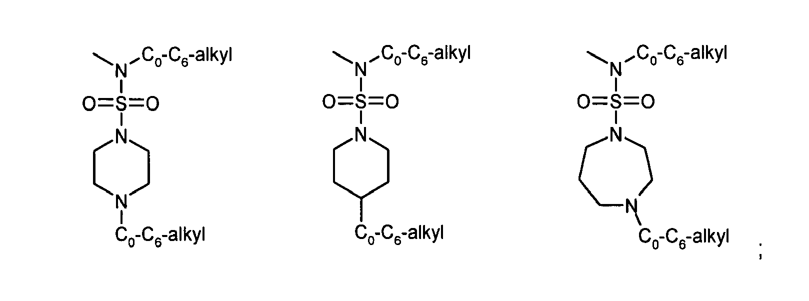
R4 and R5 may together form heterocycloalkyl, cycloalkyl;
and the groups R1, R2, R3, R5, R6, R7, R8, Q, X and W have the same meaning as defined in claim 1.

7. Compounds according to claim 6 having the formula II 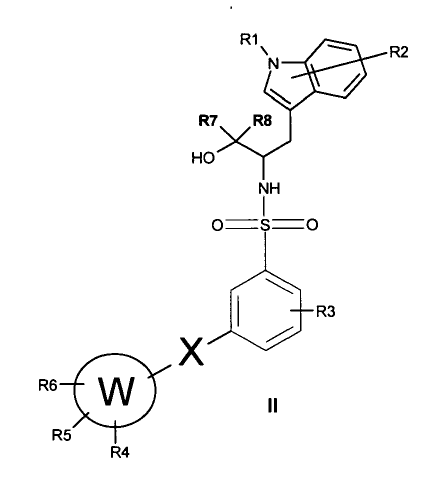 in which
R4 may be C₃-C₇-cycloalkyl-C₁-C₆-alkylene, C₃-C₇-heterocycloalkyl,
where the hydrocarbon chains therein may optionally be substituted one or more times by fluorine, cyano or the radicals: 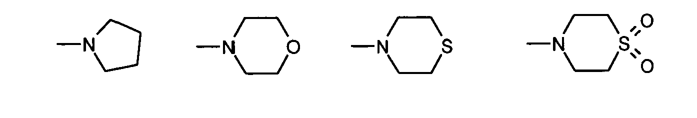 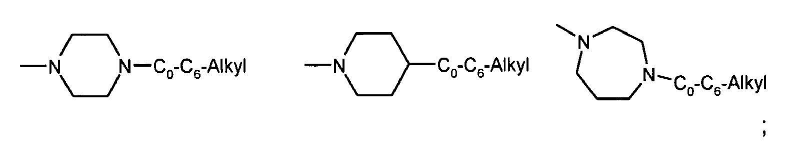 or
independently of one another hydroxy-C₃-C₆-alkenylene, hydroxy-C₃-C₆-alkynylene, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene,
C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl,
C₃-C₇-cycloalkylcarbonyl,
carboxamido [-C(O)NH₂],
C₃-C₇-cycloalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-O-(C₀-C₆)alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the radicals: 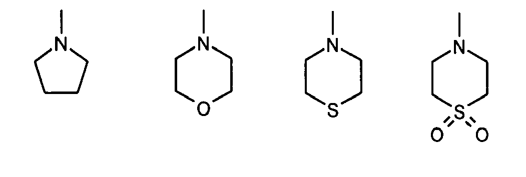 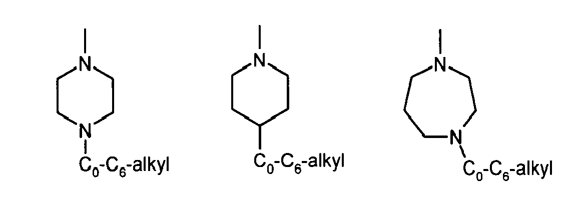 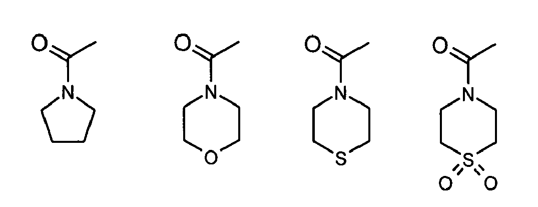 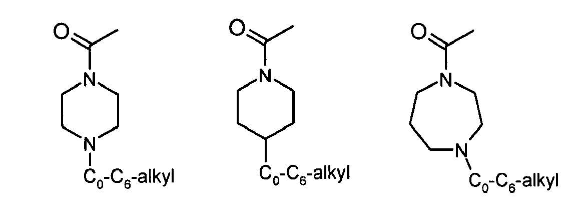 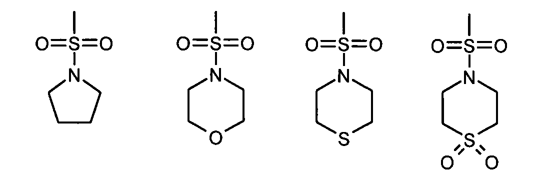 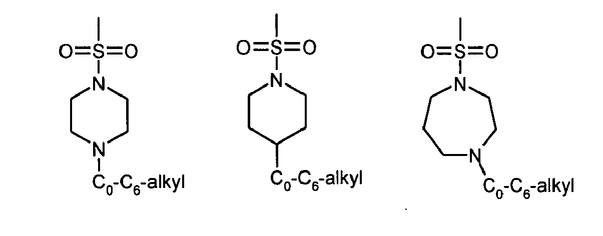 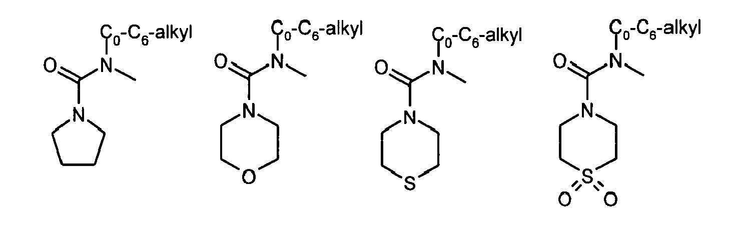 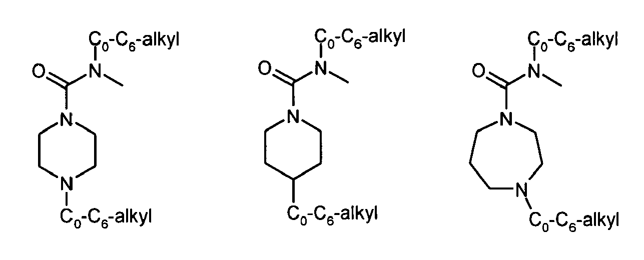 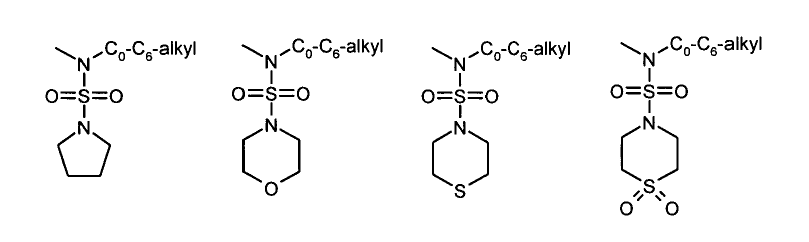 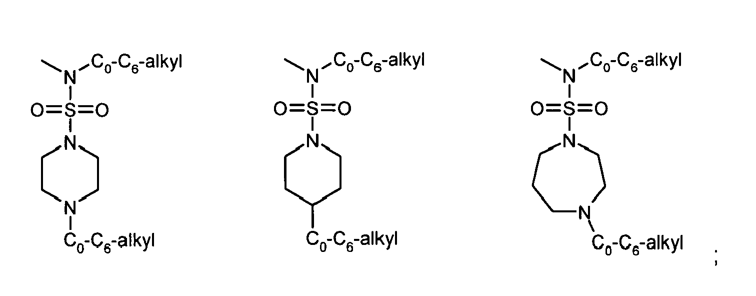
R4 and R5 may together form heterocycloalkyl, cycloalkyl;
and
R1, R2, R3, R5, R6, R7, R8, X and W have the same meaning as defined in claim 1.

8. Compounds according to claim 6, namely
| | |
|---|---|
| **2** | 3-Chloro-2'-[(R)-2-hydroxy-1-(1H-indol-3-ylmethyl)-ethylsulfamoyl]-biphenyl-4-carboxylic acid methylamide; |
| **3** | 3-Chloro-3'-[(R)-2-hydroxy-1-(1H-indol-3-ylmethyl)-ethylsulfamoyl]-4'-methoxy-biphenyl-4-carboxylic acid methylamide; |
| **4** | 3-Chloro-4'-[(R)-2-hydroxy-1-(1H-indol-3-ylmethyl)-ethylsulfamoyl]-biphenyl-4-carboxylic acid methylamide; |
| **5** | 3'-Chloro-4'-(morpholine-4-carbonyl)-5-trifluoromethyl-biphenyl-3-sulfonic acid [(R)-2-hydroxy-1-(1H-indol-3-ylmethyl)-ethyl]-amide; |
| **6** | 3-Chloro-3'-[(R)-2-hydroxy-1-(1H-indol-3-ylmethyl)-ethylsulfamoyl]-5'-trifluoromethyl-biphenyl-4-carboxylic acid methylamide; |
| **7** | 3-Chloro-3'-[(R)-2-hydroxy-1-(1H-indol-3-ylmethyl)-ethylsulfamoyl]-5'-trifluoromethyl-biphenyl-4-carboxylic acid amide; |
| **8** | 4'-Bromo-3-chloro-3'-[(R)-2-hydroxy-1-(1H-indol-3-ylmethyl)-ethylsulfamoyl]-biphenyl-4-carboxylic acid amide; |
| **9** | 3-Chloro-3'-[(R)-2-hydroxy-1-(1H-indol-3-ylmethyl)-ethylsulfamoyl]-4'-iso-propoxy-biphenyl-4-carboxylic acid methylamide; |
| **15** | 3-Chloro-3'-[(R)-2-hydroxy-1-(1H-indol-3-ylmethyl)-2-methyl-propylsulfamoyl]-4'-methoxy-biphenyl-4-carboxylic acid amide; |
| **16** | 3-Chloro-3'-[(R)-2-hydroxy-1-(1H-indol-3-ylmethyl)-2-methyl-propylsulfamoyl]-4'-methoxy-biphenyl-4-carboxylic acid methylamide; |
| **17** | 3'-Chloro-4-methoxy-4'-(morpholine-4-carbonyl)-biphenyl-3-sulfonic acid [(R)-2-hydroxy-1-(1H-indol-3-ylmethyl)-2-methyl-propyl]-amide; |
| **18** | 4-{3-[(R)-2-Hydroxy-1-(1H-indol-3-ylmethyl)-ethylsulfamoyl]-4-methoxy-phenylethynyl}-N-methyl-benzamide; |
| **19** | 3-{3-[(R)-2-Hydroxy-1-(1H-indol-3-ylmethyl)-ethylsulfamoyl]-4-methoxy-phenylethynyl}-N-methyl-benzamide; |
| **20** | 3-{3-[(R)-2-Hydroxy-1-(1H-indol-3-ylmethyl)-ethylsulfamoyl]-4-propoxy-phenylethynyl}-N-methyl-benzamide; |
| **21** | 4-{3-[(R)-2-Hydroxy-1-(1H-indol-3-ylmethyl)-ethylsulfamoyl]-4-propoxy-phenylethynyl}-N-methyl-benzamide; |
| **22** | 4-{3-[(R)-2-Hydroxy-1-(1H-indol-3-ylmethyl)-2-methyl-propylsulfamoyl]-4-methoxy-phenylethynyl}-N-methyl-benzamide; |
| **23** | 3-{3-[(R)-2-Hydroxy-1-(1H-indol-3-ylmethyl)-2-methyl-propylsulfamoyl]-4-methoxy-phenylethynyl}-N-methyl-benzamide; |
| **24** | 2-Chloro-4-{7-[(R)-2-hydroxy-1-(1H-indol-3-ylmethyl)-ethylsulfamoyl]-2,3-dihydro-benzofuran-5-yl}-N-methyl-benzamide; |
| **35** | 2-{7-[(R)-2-Hydroxy-1-(1H-indol-3-ylmethyl)-ethylsulfamoyl]-2,3-dihydro-benzofuran-5-yl})-benzamide; |
| **38** | 4-{7-[(R)-2-Hydroxy-1-(1H-indol-3-ylmethyl)-ethylsulfamoyl]-2,3-dihydro-benzofuran-5-yl}-N-methyl-benzamide; |
| **42** | 3-Fluoro-5-{7-[(R)-2-hydroxy-1-(1H-indol-3-ylmethyl)-ethylsulfamoyl]-2,3-dihydro-benzofuran-5-yl}-N-methyl-benzamide; |
| **47** | 3-{7-[(R)-2-Hydroxy-1-(1H-indol-3-ylmethyl)-ethylsulfamoyl]-2,3-dihydro-benzofuran-5-yl}-benzamide; |
| **50** | N-(3-{7-[(R)-2-Hydroxy-1-(1H-indol-3-ylmethyl)-ethylsulfamoyl]-2,3-dihydro-benzofuran-5-yl}-benzyl)-acetamide; |
| **52** | 5-(4-Cyanomethyl-phenyl)-2,3-dihydro-benzofuran-7-sulfonic acid [(R)-2-hydroxy-1-(1H-indol-3-ylmethyl)-ethyl]-amide; |
| **54** | 5-Benzo[1,3]dioxol-5-yl-2,3-dihydro-benzofuran-7-sulfonic acid [(R)-2-hydroxy-1-(1H-indol-3-ylmethyl)-ethyl]-amide; |
| **58** | 5-(3-Methanesulfonylamino-phenyl)-2,3-dihydro-benzofuran-7-sulfonic acid [(R)-2-hydr 1-(1**H-indol-3-ylmethyl)-ethyl]-amide.** |

9. Compounds of formula III 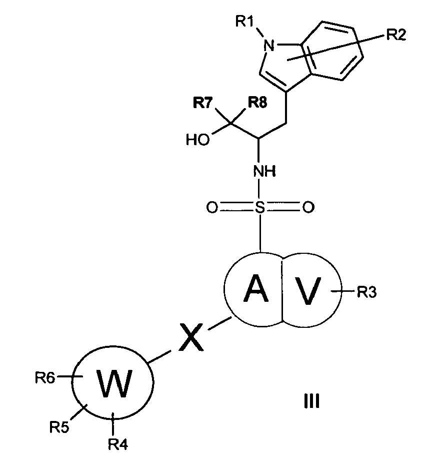 in which
R4 may be hydrogen, hydroxy, halogen, nitro, amino, cyano, phenyl, C₁-C₆-alkyl, C₂-C₆-alkenyl or C₂-C₆-alkynyl, C₃-C₇-cycloalkyl,
where the hydrocarbon chains therein may optionally be substituted one or more times by fluorine, cyano or the radicals: 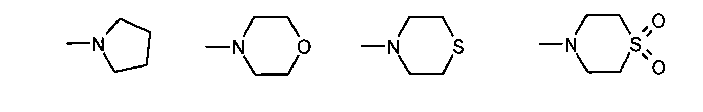 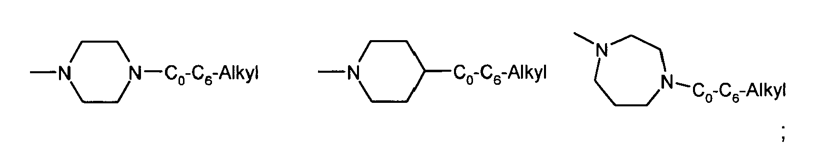 or
independently of one another hydroxy-C₁-C₆-alkylene, C₁-C₆-alkyloxy, C₁-C₆-alkyloxyphenyl-C₁-C₆-alkylene, phenyloxy-C₁-C₆-alkylene, C₁-C₆-alkylamino, di(C₁-C₆-alkyl)amino
C₁-C₆-acyl-(C₀-C₆-alkyl)amido,
C₁-C₆-alkylcarbonyl,
carboxy, C₁-C₆-alkyloxycarbonyl,
C₁-C₃-alkylsulphanyl, C₁-C₆-alkysulphonyl,-N(C₀-C₆-alkyl)-C(O)-N-di(C₀-C₆-alkyl);
and
R1, R2, R3, R5, R6, R7, R8, A, X, V and W have the same meaning as defined in claim 1.

10. Compounds according to claim 9 having the formula IV 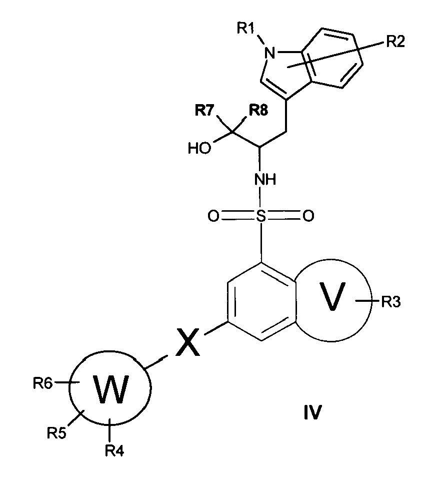 in which
R4 may be hydrogen, hydroxy, halogen, nitro, amino, cyano, phenyl, C₁-C₆-alkyl, C₂-C₆-alkenyl or C₂-C₆-alkynyl, C₃-C₇-cycloalkyl,
where the hydrocarbon chains therein may optionally be substituted one or more times by fluorine, cyano or the radicals: 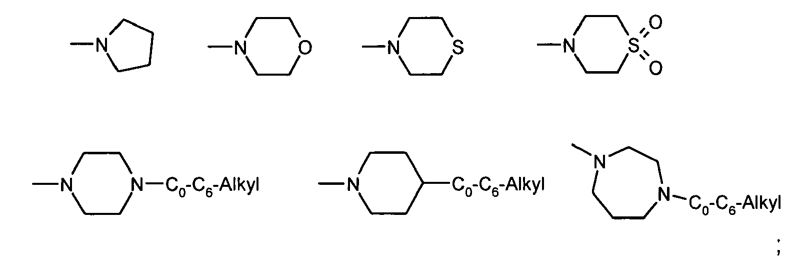 or
independently of one another hydroxy-C₁-C₆-alkylene, C₁-C₆-alkyloxy, C₁-C₆-alkyloxyphenyl-C₁-C₆-alkylene, phenyloxy-C₁-C₆-alkylene,
C₁-C₆-alkylamino, di(C₁-C₆-alkyl)amino
C₁-C₆-acyl-(C₀-C₆-alkyl)amido,
C₁-C₆-alkylcarbonyl,
carboxy, C₁-C₆-alkyloxycarbonyl,
C₁-C₃-alkylsulphanyl, C₁-C₆-alkysulphonyl,-N(C₀-C₆-alkyl)-C(O)-N-di(C₀-C₆-alkyl);
and
R1, R2, R3, R5, R6, R7, R8, V, X and W have the same meaning as defined in formula I.

11. Compounds according to claim 9, namely
| | |
|---|---|
| **25** | 5-(3,4,5-Trimethoxy-phenyl)-2,3-dihydro-benzofuran-7-sulfonic acid [(R)-2-hydroxy-1-(1H-indol-3-ylmethyl)-ethyl]-amide; |
| **26** | 5-(4-Acetyl-phenyl)-2,3-dihydro-benzofuran-7-sulfonic acid [(R)-2-hydroxy-1-(1H-indol-3-ylmethyl)-ethyl]-amide; |
| **27** | 5-(4-Methylsulfanyl-phenyl)-2,3-dihydro-benzofuran-7-sulfonic acid [(R)-2-hydroxy-1-(1H-indol-3-ylmethyl)-ethyl]-amide; |
| **28** | 5-(3-Amino-phenyl)-2,3-dihydro-benzofuran-7-sulfonic acid [(R)-2-hydroxy-1-(1H-indol-3-ylmethyl)-ethyl]-amide; |
| **29** | 5-(3-Trifluoromethyl-phenyl)-2,3-dihydro-benzofuran-7-sulfonic acid [(R)-2-hydroxy-1-(1H-indol-3-ylmethyl)-ethyl]-amide; |
| **30** | 5-(4-Hydroxy-phenyl)-2,3-dihydro-benzofuran-7-sulfonic acid [(R)-2-hydroxy-1-(1H-indol-3-ylmethyl)-ethyl]-amide; |
| **31** | 5-(4-Fluoro-phenyl)-2,3-dihydro-benzofuran-7-sulfonic acid [(R)-2-hydroxy-1-(1H-indol-3-ylmethyl)-ethyl]-amide; |
| **32** | 5-(4-Cyano-phenyl)-2,3-dihydro-benzofuran-7-sulfonic acid [(R)-2-hydroxy-1-(1H-indol-3-ylmethyl)-ethyl]-amide; |
| **33** | 5-Naphthalen-1-yl-2,3-dihydro-benzofuran-7-sulfonic acid [(R)-2-hydroxy-1-(1H-indol-3-ylmethyl)-ethyl]-amide; |
| **34** | 5-(4-Chloro-phenyl)-2,3-dihydro-benzofuran-7-sulfonic acid [(R)-2-hydroxy-1-(1H-indol-3-ylmethyl)-ethyl]-amide; |
| **36** | 5-(6-Methoxy-pyridin-3-yl)-2,3-dihydro-benzofuran-7-sulfonic acid [(R)-2-hydroxy-1-(1H-indol-3-ylmethyl)-ethyl]-amide; |
| **37** | 5-(2-Fluoro-phenyl)-2,3-dihydro-benzofuran-7-sulfonic acid [(R)-2-hydroxy-1-(1H-indol-3-ylmethyl)-ethyl]-amide; |
| **39** | 5-Quinolin-6-yl-2,3-dihydro-benzofuran-7-sulfonic acid [(R)-2-hydroxy-1-(1H-indol-3-ylmethyl)-ethyl]-amide; |
| **40** | 5-((E)-Styryl)-2,3-dihydro-benzofuran-7-sulfonic acid [(R)-2-hydroxy-1-(1H-indol-3-ylmethyl)-ethyl]-amide; |
| **41** | 5-(3-Hydroxymethyl-phenyl)-2,3-dihydro-benzofuran-7-sulfonic acid [(R)-2-hydroxy-1-(1H-indol-3-ylmethyl)-ethyl]-amide; |
| **43** | 5-(3-Fluoro-5-methoxy-phenyl)-2,3-dihydro-benzofuran-7-sulfonic acid [(R)-2-hydroxy-1-(1H-indol-3-ylmethyl)-ethyl]-amide; |
| **44** | N-(4-{7-[(R)-2-Hydroxy-1-(1H-indol-3-ylmethyl)-ethylsulfamoyl]-2,3-dihydro-benzofuran-5-yl}-phenyl)-acetamide; |
| **45** | 5-(3,5-Dimethyl-phenyl)-2,3-dihydro-benzofuran-7-sulfonic acid [(R)-2-hydroxy-1-(1H-indol-3-ylmethyl)-ethyl]-amide; |
| **46** | 5-Quinolin-3-yl-2,3-dihydro-benzofuran-7-sulfonic acid [(R)-2-hydroxy-1-(1H-indol-3-ylmethyl)-ethyl]-amide; |
| **48** | 5-(2-Fluoro-pyridin-3-yl)-2,3-dihydro-benzofuran-7-sulfonic acid [(R)-2-hydroxy-1-(1H-indol-3-ylmethyl)-ethyl]-amide; |
| **49** | 5-(5-Cyano-thiophen-2-yl)-2,3-dihydro-benzofuran-7-sulfonic acid [(R)-2-hydroxy-1-(1H-indol-3-ylmethyl)-ethyl]-amide; |
| **51** | 5-(2-Methoxy-pyrimidin-5-yl)-2,3-dihydro-benzofuran-7-sulfonic acid [(R)-2-hydroxy-1-(1H-indol-3-ylmethyl)-ethyl]-amide; |
| **53** | 5-(3-Cyano-phenyl)-2,3-dihydro-benzofuran-7-sulfonic acid [(R)-2-hydroxy-1-(1H-indol-3-ylmethyl)-ethyl]-amide; |
| **55** | N-(3-{7-[(R)-2-Hydroxy-1-(1H-indol-3-ylmethyl)-ethylsulfamoyl]-2,3-dihydro-benzofuran-5-yl}-phenyl)-acetamide; |
| **56** | 5-Biphenyl-2-yl-2,3-dihydro-benzofuran-7-sulfonic acid [(R)-2-hydroxy-1-(1H-indol-3-ylmethyl)-ethyl]-amide; |
| **57** | 5-o-Tolyl-2,3-dihydro-benzofuran-7-sulfonic acid [(R)-2-hydroxy-1-(1H-indol-3-ylmethyl)-ethyl]-amide; |
| **59** | 5-(4-Trifluoromethyl-phenyl)-2,3-dihydro-benzofuran-7-sulfonic acid [(R)-2-hydroxy-1-(1H-indol-3-ylmethyl)-ethyl]-amide; |
| **60** | 5-Benzo[b]thiophen-3-yl-2,3-dihydro-benzofuran-7-sulfonic acid [(R)-2-hydroxy-1-(1H-indol-3-ylmethyl)-ethyl]-amide; |
| **61** | 5-Biphenyl-3-yl-2,3-dihydro-benzofuran-7-sulfonic acid [(R)-2-hydroxy-1-(1H-indol-3-ylmethyl)-ethyl]-amide; |
| **62** | 5-(3-Acetyl-phenyl)-2,3-dihydro-benzofuran-7-sulfonic acid [(R)-2-hydroxy-1-(1H-indol-3-ylmethyl)-ethyl]-amide; |
| **63** | 5-(3-Fluoro-phenyl)-2,3-dihydro-benzofuran-7-sulfonic acid [(R)-2-hydroxy-1-(1H-indol-3-ylmethyl)-ethyl]-amide; |
| **64** | 2',3'-Dihydro-[2,5']bibenzofuranyl-7'-sulfonic acid [(R)-2-hydroxy-1-(1H-indol-3-ylmethyl)-ethyl]-amide; |
| **65** | 5-Benzo[b]thiophen-2-yl-2,3-dihydro-benzofuran-7-sulfonic acid [(R)-2-hydroxy-1-(1H-indol-3-ylmethyl)-ethyl]-amide; |
| **66** | 5-(3-Chloro-phenyl)-2,3-dihydro-benzofuran-7-sulfonic acid [(R)-2-hydroxy-1-(1H-indol-3-ylmethyl)-ethyl]-amide; |
| **67** | 5-p-Tolyl-2,3-dihydro-benzofuran-7-sulfonic acid [(R)-2-hydroxy-1-(1H-indol-3-ylmethyl)-ethyl]-amide; |
| **68** | 5-Naphthalen-2-yl-2,3-dihydro-benzofuran-7-sulfonic acid [(R)-2-hydroxy-1-(1H-indol-3-ylmethyl)-ethyl]-amide; |
| **69** | 5-(3-Methoxy-phenyl)-2,3-dihydro-benzofuran-7-sulfonic acid [(R)-2-hydroxy-1-(1H-indol-3-ylmethyl)-ethyl]-amide; |
| **70** | 5-(4-Methoxy-phenyl)-2,3-dihydro-benzofuran-7-sulfonic acid [(R)-2-hydroxy-1-(1H-indol-3-ylmethyl)-ethyl]-amide; |
| **71** | 5-Thiophen-3-yl-2,3-dihydro-benzofuran-7-sulfonic acid [(R)-2-hydroxy-1-(1H-indol-3-ylmethyl)-ethyl]-amide. |

12. Process for preparing compounds of the formula I of claim 1, wherein a tryptophanol derivative of the formula V 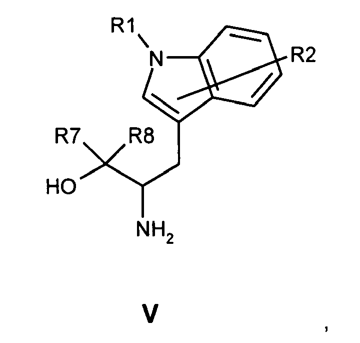 in which the radicals R1, R2, R7 and R8 have the same meaning as defined in claim 1,
is coupled in a sulphonamide-forming reaction,
where appropriate in the presence of a base,
with a sulfonyl chloride of the formula VI 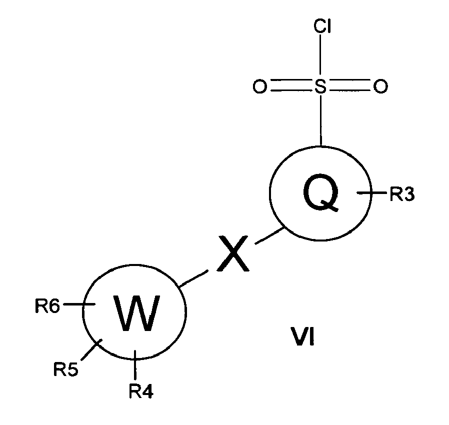 in which R3, R4, R5, R6, Q, X, V and W have the same meaning as defined in claim 1.

13. Process according to claim 12 for preparing compounds of the formula II of claim 7, wherein a tryptophanol derivative of the formula V is coupled with a sulfonyl chloride of the formula VII 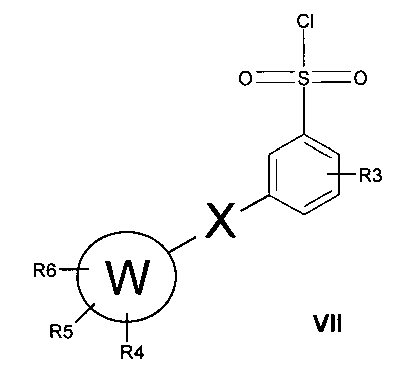 in which R3, R4, R5, R6, X and W have the same meaning as defined in claim 8.

14. Process according to claim 12 for preparing compounds of the formula III of claim 9, wherein a tryptophanol derivative of the formula V is coupled with a sulfonyl chloride of the formula VIII 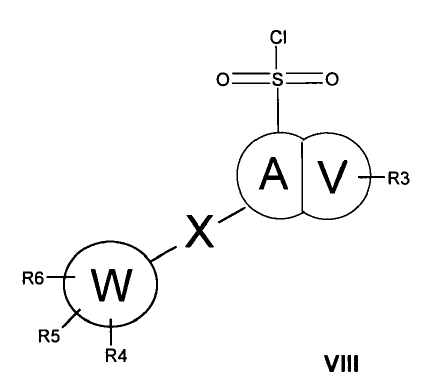 in which R3, R4, R5, R6, A, V, X and W have the same meaning as defined in claim 9.

15. Process according to claim 12 for preparing compounds of the formula IV of claim 10, wherein a tryptophanol derivative of the formula V is coupled with a sulfonyl chloride of the formula IX 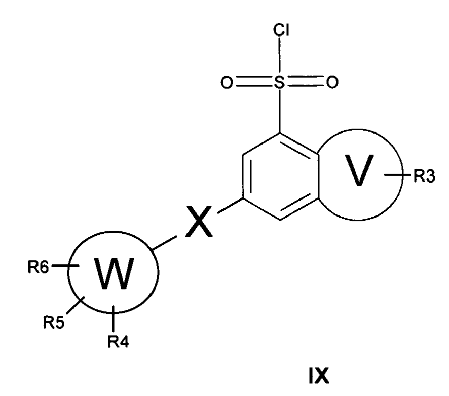 in which R3, R4, R5, R6, V, X and W have the same meaning as defined in claim 10.

16. Process for preparing compounds of the formula I of claim 1, wherein the building block of the formula X 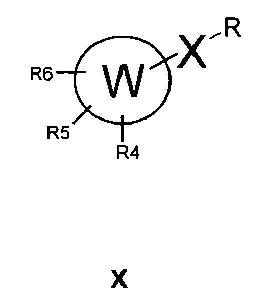 in which R4, R5, R6, W and X have the same meaning as defined in claim 1 and
R is a -B(OH)₂, -C≡C-H, -Zn-Hal or -Sn(alkyl)₃) group,
is coupled in a metal catalyzed cross-coupling reaction
with an aryl halide of the formula XI 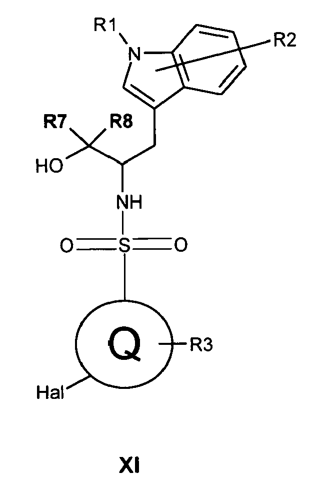 in which R1, R2, R3, R7, R8 and Q have the same meaning as defined in claim 1,
and
Hal stands for a chlorine, bromine or iodine.

17. Process according to claim 16 for preparing compounds of the formula II of claim 7, wherein the building block of the formula X is coupled with an aryl halide of the formula XII 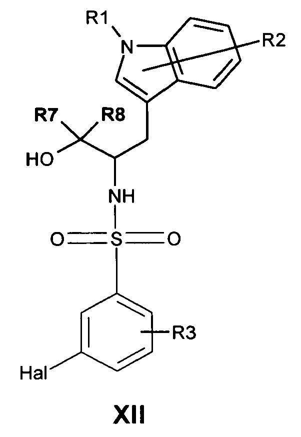 in which R1, R2, R3, R7, R8, V have the same meaning as defined in claim 7, and
Hal stands for a chlorine, bromine or iodine.

18. Process according to claim 16 for preparing compounds of the formula III of claim 9, wherein the building block of the formula X is coupled with an aryl halide of the formula XIII 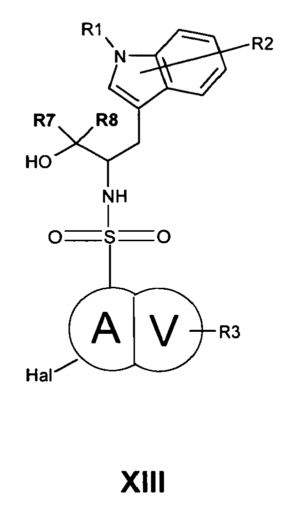 in which R1, R2, R3, R7, R8, A and V have the same meaning as defined in claim 9, and
Hal stands for a chlorine, bromine or iodine.

19. Process according to claim 10 for preparing compounds of the formula IV of claim 10, wherein the building block of the formula X is coupled with an aryl halide of the formula XIV 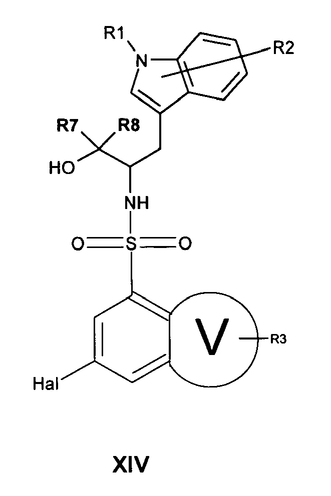 in which R1, R2, R3, R7, R8 and V have the same meaning as defined in claim 10, and
Hal stands for a chlorine, bromine or iodine.

20. Aryl halides as intermediates in a process according to any of claims 16 to 19, namely
5-Bromo-N-[(R)-2-hydroxy-1-(1H-indol-3-ylmethyl)-ethyl]-2-methoxy-benzenesulfonamide;
2-Bromo-N-[(R)-2-hydroxy-1-(1H-indol-3-ylmethyl)-ethyl]-benzenesulfonamide;
4-Bromo-N-[(R)-2-hydroxy-1-(1H-indol-3-ylmethyl)-ethyl]-benzenesulfonamide;
3-Bromo-N-[(R)-2-hydroxy-1-(1H-indol-3-ylmethyl)-ethyl]-5-trifluoromethylbenzenesulfonamide;
2,5-Dibromo-N-[(R)-2-hydroxy-1-(1H-indol-3-ylmethyl)-ethyl]-benzenesulfonamide;
5-Bromo-N-[(R)-2-hydroxy-1-(1H-indol-3-ylmethyl)-ethyl]-2-isopropoxy
benzenesulfonamide;
5-Bromo-N-[(R)-2-hydroxy-1-(1H-indol-3-ylmethyl)-2-methyl-propyl]-2-methoxy-benzenesulfonamide;
5-Bromo-N-[(R)-2-hydroxy-1-(1H-indol-3-ylmethyl)-ethyl]-2-propoxy-benzenesulfonamide;
5-Bromo-2,3-dihydro-benzofuran-7-sulfonic acid [(R)-2-hydroxy-1-(1H-indol-3-ylmethyl)-ethyl]-amide.

21. Pharmaceutical compositions comprising at least one of the compounds according to any of claims 4 to 11 and pharmaceutically suitable excipients and/or carriers.

## Amended claims

### Amended claims in accordance with Rule 137(2) EPC.

**24.** 2-Chloro-4-{7-[(R)-2-hydroxy-1-(1H-indol-3-ylmethyl)-ethylsulfamoyl]-2,3-dihydro-benzofuran-5-yl}-N-methyl-benzamide

**35.** 2-{7-[(R)-2-Hydroxy-1-(1H-indol-3-ylmethyl)-ethylsulfamoyl]-2,3-dihydrobenzofuran-5-yl}-benzamide;

**38.** 4-{7-[(R)-2-Hydroxy-1-(1H-indol-3-ylmethyl)-ethylsulfamoyl]-2,3-dihydrobenzofuran-5-yl}-N-methyl-benzamide;

**42.** 3-Fluoro-5-{7-[(R)-2-hydroxy-1-(1H-indol-3-ylmethyl)-ethylsulfamoyl]-2,3-dihydro-benzofuran-5-yl}-N-methyl-benzamide;

**47.** 3-{7-[(R)-2-Hydroxy-1-(1H-indol-3-ylmethyl)-ethylsulfamoyl]-2,3-dihydrobenzofuran-5-yl}-benzamide;

**50.** N-(3-{7-[(R)-2-Hydroxy-1-(1H-indol-3-ylmethyl)-ethylsulfamoyl]-2,3-dihydrobenzofuran-5-yl}-benzyl)-acetamide;

**52.** 5-(4-Cyanomethyl-phenyl)-2,3-dihydro-benzofuran-7-sulfonic acid [(R)-2-hydroxy-1-(1H-indol-3-ylmethyl)-ethyl]-amide;

**54.** 5-Benzo[1,3]dioxol-5-yl-2,3-dihydro-benzofuran-7-sulfonic acid [(R)-2-hydroxy-1-(1H-indol-3-ylmethyl)-ethyl]-amide;

**58.** 5-(3-Methanesulfonylamino-phenyl)-2,3-dihydro-benzofuran-7-sulfonic acid [(R)-2-hydroxy-1-(1H-indol-3-ylmethyl)-ethyl]-amide.
